# EUROPEAN PATENT SPECIFICATION

(11) **EP 2 793 912 B1**
(45) Date of publication and mention of the grant of the patent: **18.03.2020**
(21) Application number: 12860504.5
(22) Date of filing: 21.12.2012
(51) Int. Cl.: A61K 9/14, A61F 13/00, A61L 33/00, A61L 27/48, A61K 35/50, A61L 27/36, A61P 3/00, A61P 5/00, A61P 15/08

(54) **ORGANOIDS COMPRISING DECELLULARIZED AND REPOPULATED PLACENTAL VASCULAR SCAFFOLD**
ORGANELLEN MIT DEZELLULARISIERTEM UND WIEDERBESIEDELTEM PLAZENTALEM VASKULÄREM GERÜST
ORGANOÏDES COMPRENANT UN ÉCHAFAUDAGE VASCULAIRE PLACENTAIRE REPEUPLÉ ET DÉCELLULARISÉ

(30) Priority: 23.12.2011 US 201161579942 P; 30.01.2012 US 201261592350 P; 04.09.2012 US 201261696527 P
(43) Date of publication of application: 29.10.2014
(73) Proprietor: Celularity, Inc., Warren, NJ 07059 (US)
(72) Inventor: BHATIA, Mohit, B., Manalapan, NJ 07726 (US); HARIRI, Robert, J., Bernardsville, NJ 07924 (US); HOFGARTNER, Wolfgang, Florham Park, NJ 07932 (US); WANG, Jia-lun, Cherry Hill, NJ 08003 (US); YI, Qian, Livingston, NJ 07039 (US)
(74) Representative: Mewburn Ellis LLP
(86) International application number: PCT/US2012/071192
(87) International publication number: WO 2013/096741

(56) References cited:
- US-A1- 2006 282 173
- US-A1- 2010 272 782
- US-A1- 2011 002 996
- FLYNN ET AL: "Adipose tissue engineering with naturally derived scaffolds and adipose-derived stem cells", BIOMATERIALS, ELSEVIER SCIENCE PUBLISHERS BV., BARKING, GB, vol. 28, no. 26, 14 July 2007 (2007-07-14) , pages 3834-3842, XP022153660, ISSN: 0142-9612, DOI: 10.1016/J.BIOMATERIALS.2007.05.002
- FLYNN ET AL: "Proliferation and differentiation of adipose-derived stem cells on naturally derived scaffolds", BIOMATERIALS, ELSEVIER SCIENCE PUBLISHERS BV., BARKING, GB, vol. 29, no. 12, 1 February 2008 (2008-02-01), pages 1862-1871, XP022499084, ISSN: 0142-9612, DOI: 10.1016/J.BIOMATERIALS.2007.12.028
- FLYNN LAUREN ET AL: "Decellularized placental matrices for adipose tissue engineering.", JOURNAL OF BIOMEDICAL MATERIALS RESEARCH. PART A NOV 2006, vol. 79, no. 2, November 2006 (2006-11), pages 359-369, XP002745466, ISSN: 1549-3296
- STEPHEN F. BADYLAK ET AL: "Whole-Organ Tissue Engineering: Decellularization and Recellularization of Three-Dimensional Matrix Scaffolds", ANNUAL REVIEW OF BIOMEDICAL ENGINEERING, vol. 13, no. 1, 15 August 2011 (2011-08-15), pages 27-53, XP55127197, ISSN: 1523-9829, DOI: 10.1146/annurev-bioeng-071910-124743
- WANG MIN ET AL: "Hepatogenesis of Adipose-Derived Stem Cells on Poly-Lactide-co-Glycolide Scaffolds: In Vitro and In Vivo Studies", TISSUE ENGINEERING PART C-METHODS, vol. 16, no. 5, October 2010 (2010-10), pages 1041-1050, XP002745467,
- TAYLOR ET AL.: 'Factors secreted by Schwann cells stimulate the regeneration of neonatal retinal ganglion cells' J. ANAT. vol. 204, no. 1, January 2004, pages 25 - 31, XP055165706

## Description

This application claims priority to U.S. provisional patent application No. 61/579,942, filed December 23, 2011, U.S. provisional patent application No. 61/592,350, filed January 30, 2012, and U.S. provisional patent application No. 61/696,527, filed September 4, 2012, the disclosure of each of which is herein incorporated by reference in its entirety.

### 1. FIELD

Provided herein are organoids comprising decellularized placental vascular scaffold comprising, or consisting of, a decellularized placental vascular scaffold, and methods of making and using the same.

### 2. BACKGROUND

There exists a great medical need for the replacement of the physiological functionality diseased, damaged or surgically removed tissues. Provided herein are organoids comprising decellularized placental vascular scaffold, and methods of making and using the same, which fulfill this need.

### 3. SUMMARY

The invention is defined in the claims. Provided herein are organoids comprising one or more types of cells, and decellularized placental vascular scaffold, wherein said organoids perform at least one function of an organ, or a tissue from an organ, wherein said at least one function of an organ or tissue from an organ is production of a protein, growth factor, cytokine, interleukin, or small molecule characteristic of at least one cell type from said organ or tissue; and wherein said decellularized placental vascular scaffold comprises substantially intact placental vasculature matrix; that is, the structure of the vasculature of the placenta from which the matrix is obtained is substantially preserved during decellularization and subsequent production of the organoids and wherein said decellularized placental vascular scaffold comprises substantially intact placental vasculature matrix;wherein said cells have been genetically engineered to produce a protein or polypeptide not naturally produced by the cell, or have been genetically engineered to produce a protein or polypeptide in an amount greater than that naturally produced by the cell.

In various embodiments, said organoids comprise about, no more than, or at least, 10¹², 10¹¹, 10¹⁰ cells, 10⁹ cells, 10⁸ cells, 10⁷ cells, 10⁶ cells, 10⁵ cells, 10⁴ cells, 10³ cells, or 10² cells.

In a specific embodiment of any of the embodiments herein, said organoids additionally comprise a synthetic matrix. In a more specific embodiment, said synthetic matrix stabilizes the three-dimensional structure of said organoids. In certain specific embodiments, said synthetic matrix comprises a polymer or a thermoplastic. In certain specific embodiments, said synthetic matrix is a polymer or a thermoplastic. In more specific embodiments, said thermoplastic is polycaprolactone (PCL), polylactic acid, polybutylene terephthalate, polyethylene terephthalate, polyethylene, polyester, polyvinyl acetate, or polyvinyl chloride. In a specific embodiment, the thermoplastic is the synthetic polymer PCL. In certain other specific embodiments, said polymer is polyvinylidine chloride, poly(o-carboxyphenoxy)-p-xylene) (poly(o-CPX)), poly(lactide-anhydride) (PLAA), n-isopropyl acrylamide, acrylamide, pent erythritol diacrylate, polymethyl acrylate, carboxymethylcellulose, or poly(lactic-co-glycolic acid) (PLGA). In certain other specific embodiments, said polymer is polyacrylamide.

In certain specific embodiments, said one or more types of cells in said organoids comprise natural killer (NK) cells, *e.g.,* CD56⁺ CD16⁻ placental intermediate natural killer (PiNK) cells. In certain other specific embodiments, said organoids comprise dendritic cells.

In certain specific embodiments, said organoids comprise thymocytes. In certain other embodiments, said organoids comprise thymocytes, lymphoid cells, epithelial reticular cells, and thymic stromal cells.

In certain other specific embodiments, said organoids comprise thyroid follicular cells. In certain other embodiments, said organoids comprise cells that express thyroglobulin. In certain other specific embodiments, said organoids additionally comprise thyroid epithelial cells and parafollicular cells.

In certain specific embodiments, said organoids comprise stem cells or progenitor cells. In specific embodiments, said stem cells or progenitor cells are embryonic stem cells, embryonic germ cells, induced pluripotent stem cells, mesenchymal stem cells, bone marrow-derived mesenchymal stem cells, bone marrow-derived mesenchymal stromal cells, CD34⁻, CD10⁺, CD105⁺, and CD200⁺ placental tissue plastic-adherent placental stem cells (PDAC®), umbilical cord stem cells, amniotic fluid stem cells, amnion derived adherent cells (AMDACs), osteogenic placental adherent cells (OPACs), adipose stem cells, limbal stem cells, dental pulp stem cells, myoblasts, endothelial progenitor cells, neuronal stem cells, exfoliated teeth derived stem cells, hair follicle stem cells, dermal stem cells, parthenogenically derived stem cells, reprogrammed stem cells, amnion derived adherent cells, or side population stem cells. In certain other specific embodiments, said organoids comprise hematopoietic stem cells or hematopoietic progenitor cells. In certain other specific embodiments, said organoids comprise tissue culture plastic-adherent CD34⁻, CD10⁺, CD105⁺, and CD200⁺ placental stem cells. In a more specific embodiment, said placental stem cells are additionally one or more of CD45⁻, CD80⁻, CD86⁻, or CD90⁺. In a more specific embodiment, said placental stem cells are additionally CD45⁻, CD80⁻, CD86⁻, and CD90⁺. In another more specific embodiment, said placental stem cells, when said organoids are implanted into a recipient, suppresses an immune response in said recipient, e.g., locally within said recipient.

In certain other specific embodiments, any of the organoids described herein comprise differentiated cells. In more specific embodiments, said differentiated cells comprise one or more of:
endothelial cells, epithelial cells, dermal cells, endodermal cells, mesodermal cells, fibroblasts, osteocytes, chondrocytes, natural killer cells, dendritic cells, hepatic cells, pancreatic cells, or stromal cells;
salivary gland mucous cells, salivary gland serous cells, von Ebner's gland cells, mammary gland cells, lacrimal gland cells, ceruminous gland cells, eccrine sweat gland dark cells, eccrine sweat gland clear cells, apocrine sweat gland cells, gland of Moll cells, sebaceous gland cells. bowman's gland cells, Brunner's gland cells, seminal vesicle cells, prostate gland cells, bulbourethral gland cells, Bartholin's gland cells, gland of Littre cells, uterus endometrium cells, isolated goblet cells, stomach lining mucous cells, gastric gland zymogenic cells, gastric gland oxyntic cells, pancreatic acinar cells, paneth cells, type II pneumocytes, clara cells,
somatotropes, lactotropes, thyrotropes, gonadotropes, corticotropes, intermediate pituitary cells, magnocellular neurosecretory cells, gut cells, respiratory tract cells, thyroid epithelial cells, parafollicular cells, parathyroid gland cells, parathyroid chief cell, oxyphil cell, adrenal gland cells, chromaffin cells, Leydig cells, theca interna cells, corpus luteum cells, granulosa lutein cells, theca lutein cells, juxtaglomerular cell, macula densa cells, peripolar cells, mesangial cell,
blood vessel and lymphatic vascular endothelial fenestrated cells, blood vessel and lymphatic vascular endothelial continuous cells, blood vessel and lymphatic vascular endothelial splenic cells, synovial cells, serosal cell (lining peritoneal, pleural, and pericardial cavities), squamous cells, columnar cells, dark cells, vestibular membrane cell (lining endolymphatic space of ear), stria vascularis basal cells, stria vascularis marginal cell (lining endolymphatic space of ear), cells of Claudius, cells of Boettcher, choroid plexus cells, pia-arachnoid squamous cells, pigmented ciliary epithelium cells, nonpigmented ciliary epithelium cells, corneal endothelial cells, peg cells,
respiratory tract ciliated cells, oviduct ciliated cell, uterine endometrial ciliated cells, rete testis ciliated cells, ductulus efferens ciliated cells, ciliated ependymal cells,
epidermal keratinocytes, epidermal basal cells, keratinocyte of fingernails and toenails, nail bed basal cells, medullary hair shaft cells, cortical hair shaft cells, cuticular hair shaft cells, cuticular hair root sheath cells, hair root sheath cells of Huxley's layer, hair root sheath cells of Henle's layer, external hair root sheath cells, hair matrix cells,
surface epithelial cells of stratified squamous epithelium, basal cell of epithelia, urinary epithelium cells,
auditory inner hair cells of organ of Corti, auditory outer hair cells of organ of Corti, basal cells of olfactory epithelium, cold-sensitive primary sensory neurons, heat-sensitive primary sensory neurons, Merkel cells of epidermis, olfactory receptor neurons, pain-sensitive primary sensory neurons, photoreceptor rod cells, photoreceptor blue-sensitive cone cells, photoreceptor green-sensitive cone cells, photoreceptor red-sensitive cone cells, proprioceptive primary sensory neurons, touch-sensitive primary sensory neurons, type I carotid body cells, type II carotid body cell (blood pH sensor), type I hair cell of vestibular apparatus of ear (acceleration and gravity), type II hair cells of vestibular apparatus of ear, type I taste bud cells,
cholinergic neural cells, adrenergic neural cells, peptidergic neural cells,
inner pillar cells of organ of Corti, outer pillar cells of organ of Corti, inner phalangeal cells of organ of Corti, outer phalangeal cells of organ of Corti, border cells of organ of Corti, Hensen cells of organ of Corti, vestibular apparatus supporting cells, taste bud supporting cells, olfactory epithelium supporting cells, Schwann cells, satellite cells, enteric glial cells,
astrocytes, neurons, oligodendrocytes, spindle neurons,
anterior lens epithelial cells, crystallin-containing lens fiber cells,
hepatocytes, adipocytes, white fat cells, brown fat cells, liver lipocytes,
kidney glomerulus parietal cells, kidney glomerulus podocytes, kidney proximal tubule brush border cells, loop of Henle thin segment cells, kidney distal tubule cells, kidney collecting duct cells, type I pneumocytes, pancreatic duct cells, nonstriated duct cells, duct cells, intestinal brush border cells, exocrine gland striated duct cells, gall bladder epithelial cells, ductulus efferens nonciliated cells, epididymal principal cells, epididymal basal cells,
ameloblast epithelial cells, planum semilunatum epithelial cells, organ of Corti interdental epithelial cells, loose connective tissue fibroblasts, corneal keratocytes, tendon fibroblasts, bone marrow reticular tissue fibroblasts, nonepithelial fibroblasts, pericytes, nucleus pulposus cells, cementoblast/cementocytes, odontoblasts, odontocytes, hyaline cartilage chondrocytes, fibrocartilage chondrocytes, elastic cartilage chondrocytes, osteoblasts, osteocytes, osteoclasts, osteoprogenitor cells, hyalocytes, stellate cells (ear), hepatic stellate cells (Ito cells), pancreatic stelle cells,
red skeletal muscle cells, white skeletal muscle cells, intermediate skeletal muscle cells, nuclear bag cells of muscle spindle, nuclear chain cells of muscle spindle, satellite cells, ordinary heart muscle cells, nodal heart muscle cells, Purkinje fiber cells, smooth muscle cells, myoepithelial cells of iris, myoepithelial cell of exocrine glands,
reticulocytes, megakaryocytes, monocytes, connective tissue macrophages. epidermal Langerhans cells, dendritic cells, microglial cells, neutrophils, eosinophils, basophils, mast cell, helper T cells, suppressor T cells, cytotoxic T cell, natural Killer T cells, B cells, natural killer cells,
melanocytes, retinal pigmented epithelial cells,
oogonia/oocytes, spermatids, spermatocytes, spermatogonium cells, spermatozoa, ovarian follicle cells, Sertoli cells, thymus epithelial cell, and/or interstitial kidney cells.

In certain other specific embodiments, said cells are primary culture cells. In another specific embodiment, cells are cells that have been cultured *in vitro.* In certain other specific embodiments, said cells have been genetically engineered to produce a protein or polypeptide not naturally produced by the cells, or have been genetically engineered to produce a protein or polypeptide in an amount greater than that naturally produced by the cells. In specific embodiments, said protein or polypeptide is a cytokine or a peptide comprising an active part thereof. In more specific embodiments, said cytokine is one or more of adrenomedullin (AM), angiopoietin (Ang), bone morphogenetic protein (BMP), brain-derived neurotrophic factor (BDNF), epidermal growth factor (EGF), erythropoietin (Epo), fibroblast growth factor (FGF), glial cell line-derived neurotrophic factor (GNDF), granulocyte colony stimulating factor (G-CSF), granulocyte-macrophage colony stimulating factor (GM-CSF), growth differentiation factor (GDF-9), hepatocyte growth factor (HGF), hepatoma derived growth factor (HDGF), insulin-like growth factor (IGF), migration-stimulating factor, myostatin (GDF-8), myelomonocytic growth factor (MGF), nerve growth factor (NGF), placental growth factor (PlGF), platelet-derived growth factor (PDGF), thrombopoietin (Tpo), transforming growth factor alpha (TGF-α), TGF-β, tumor necrosis factor alpha (TNF-α), vascular endothelial growth factor (VEGF), or a Wnt protein. In any of the above embodiments, an individual said organoid, e.g., an organoid comprising 1 x 10⁸ cells, produces at least 1.0 to 10 µM said cytokine in *in vitro* culture in growth medium over 24 hours.

In other more specific embodiments, said protein or polypeptide is a soluble receptor for AM, Ang, BMP, BDNF, EGF, Epo, FGF, GNDF, G-CSF, GM-CSF, GDF-9, HGF, HDGF, IGF, migration-stimulating factor, GDF-8, MGF, NGF, PlGF, PDGF, Tpo, TGF-α, TGF-β, TNF-α, VEGF, or a Wnt protein. In other specific embodiments, an individual said organoid, e.g., an organoid comprising 1 x 10⁸ cells, produces at least 1.0 to 10 µM of said soluble receptor in *in vitro* culture in growth medium over 24 hours.

In other specific embodiments, said protein or polypeptide is an interleukin or an active portion thereof. In various more specific embodiments, said interleukin is interleukin-1 alpha (IL-1α), IL-1β, IL-1F1, IL-1F2, IL-1F3, IL-1F4, IL-1F5, IL-1F6, IL-1F7, IL-1F8, IL-1F9, IL-2, IL-3, IL-4, IL-5, IL-6, IL-7, IL-8, IL-9, IL-10, IL-11, IL-12 35 kDa alpha subunit, IL-12 40 kDa beta subunit, both IL-12 alpha and beta subunits, IL-13, IL-14, IL-15, IL-16, IL-17A, IL-17B, IL-17C, IL-17D, IL-17E, IL-17F isoform 1, IL-17F isoform 2, IL-18, IL-19, IL-20, IL-21, IL-22, IL-23 p19 subunit, IL-23 p40 subunit, IL-23 p19 subunit and IL-23 p40 subunit together, IL-24, IL-25, IL-26, IL-27B, IL-27-p28, IL-27B and IL-27-p28 together, IL-28A, IL-28B, IL-29, IL-30, IL-31, IL-32, IL-33, IL-34, IL-35, IL-36α, IL-36β, IL-36γ. In other more specific embodiments, an individual said organoid, e.g., an organoid comprising 1 x 10⁸ cells, produces at least 1.0 to 10 µM of said interleukin or active portion thereof in *in vitro* culture in growth medium over 24 hours.

In certain more specific embodiments, said protein or polypeptide is a soluble receptor for IL-1α, IL-1β, IL-1F1, IL-1F2, IL-1F3, IL-1F4, IL-1F5, IL-1F6, IL-1F7, IL-1F8, IL-1F9, IL-2, IL-3, IL-4, IL-5, IL-6, IL-7, IL-8, IL-9, IL-10, IL-11, IL-12 35 kDa alpha subunit, IL-12 40 kDa beta subunit, IL-13, IL-14, IL-15, IL-16, IL-17A, IL-17B, IL-17C, IL-17D, IL-17E, IL-17F isoform 1, IL-17F isoform 2, IL-18, IL-19, IL-20, IL-21, IL-22, IL-23 p19 subunit, IL-23 p40 subunit, IL-24, IL-25, IL-26, IL-27B, IL-27-p28, IL-28A, IL-28B, IL-29, IL-30, IL-31, IL-32, IL-33, IL-34, IL-35, IL-36α, IL-36β, IL-36γ. In a more specific embodiment, an individual said organoid, e.g., an organoid comprising 1 x 10⁸ cells, produces at least 1.0 to 10 µM of said soluble receptor in *in vitro* culture in growth medium over 24 hours.

In another more specific embodiment, said protein is an interferon (IFN). In specific embodiments, said interferon is IFN-α, IFN-β, IFN-γ, IFN-λ1, IFN-λ2, IFN-λ3, IFN-K, IFN-ε, IFN-κ, IFN-τ, IFN-δ, IFN-ζ, IFN-ω, or IFN-v. In other specific embodiments, an individual said organoid, e.g., an organoid comprising 1 x 10⁸ cells, produces at least 1.0 to 10 µM of said interferon in *in vitro* culture in growth medium over 24 hours.

In other more specific embodiments, said protein or polypeptide is a soluble receptor for IFN-α, IFN-β, IFN-γ, IFN-λ1, IFN-λ2, IFN-λ3, IFN-K, IFN-ε, IFN-κ, IFN-τ, IFN-δ, IFN-ζ, IFN-ω, or IFN-v. In certain specific embodiments, an individual said organoid, e.g., an organoid comprising 1 x 10⁸ cells, produces at least 1.0 to 10 µM of said soluble receptor in *in vitro* culture in growth medium over 24 hours.

In another specific embodiment, said protein is insulin or proinsulin. In a specific embodiment, an individual said organoid, e.g., an organoid comprising 1 x 10⁸ cells, produces at least 1.0 to 10 µM of said insulin in *in vitro* culture in growth medium over 24 hours. In another specific embodiment, said protein is a receptor for insulin. In certain more specific embodiments, said cells producing insulin or proinsulin have additionally been genetically engineered to produce one or more of prohormone convertase 1, prohormone convertase 2, or carboxypeptidase E.

In another specific embodiment, said protein is leptin (LEP). In another specific embodiment, an individual said organoid, e.g., an organoid comprising 1 x 10* cells, produces at least 1.0 to 10 µM of said leptin in *in vitro* culture in growth medium over 24 hours.

In another specific embodiment, said protein is erythropoietin. In another specific embodiment, an individual said organoid, e.g., an organoid comprising 1 x 10* cells, produces at least 1.0 to 10 µM of said erythropoietin in *in vitro* culture in growth medium over 24 hours. In another specific embodiment, said protein is thrombopoietin. In another specific embodiment, the organoid, e.g., comprises 1 x 10⁸ cells, and, e.g., produces at least 1.0 to 10 µM of said thrombopoietin in *in vitro* culture in growth medium over 24 hours.

In another specific embodiment, said protein is tyrosine 3-monooxygenase. In certain specific embodiments, an individual said organoid comprising cells engineered to express tyrosine 3-monooxygenase, e.g., an organoid comprising 1 x 10⁸ such cells, produces at least 1.0 to 10 µM of L-DOPA in *in vitro* culture in growth medium over 24 hours. In a more specific embodiment, said cells expressing said tyrosine 3-monoosygenase have been further engineered to express aromatic L-amino acid decarboxylase. In a more specific embodiment, an individual said organoid, e.g., an organoid comprising 1 x 10⁸ cells, produces at least 1.0 to 10 µM of dopamine in *in vitro* culture in growth medium over 24 hours.

In certain other specific embodiments, said protein is a hormone or prohormone. In various specific embodiments, said hormone is antimullerian hormone (AMH), adiponectin (Acrp30), adrenocorticotropic hormone (ACTH), angiotensin (AGT), angiotensinogen (AGT), antidiuretic hormone (ADH), vasopressin, atrial-natriuretic peptide (ANP), calcitonin (CT), cholecystokinin (CCK), corticotrophin-releasing hormone (CRH), erythropoietin (Epo), follicle-stimulating hormone (FSH), testosterone, estrogen, gastrin (GRP), ghrelin, glucagon (GCG), gonadotropin-releasing hormone (GnRH), growth hormone (GH), growth hormone releasing hormone (GHRH), human chorionic gonadotropin (hCG), human placental lactogen (HPL), inhibin, leutinizing hormone (LH), melanocyte stimulating hormone (MSH), orexin, oxytocin (OXT), parathyroid hormone (PTH), prolactin (PRL), relaxin (RLN), secretin (SCT), somatostatin (SRIF), thrombopoietin (Tpo), thyroid-stimulating hormone (Tsh), and/or thyrotropin-releasing hormone (TRH).

In another specific embodiment, protein is cytochrome P450 side chain cleavage enzyme (P450SCC).

In another specific embodiment, said protein is a protein missing or malfunctioning in an individual who has a genetic disorder or disease. In certain specific embodiments, said genetic disease is familial hypercholesterolemia and said protein is low density lipoprotein receptor (LDLR); said genetic disease is polycystic kidney disease, and said protein is polycystin-1 (PKD1), PKD-2 or PKD3; or said genetic disease is phenylketonuria and said protein is phenylalanine hydroxylase.

In a specific embodiment of any of the organoids disclosed herein, said organoids comprise an immune suppressive compound or an anti-inflammatory compound. In specific embodiments, said immune-suppressive or anti-inflammatory compound is a non-steroidal anti-inflammatory drug (NSAID), acetaminophen, naproxen, ibuprofen, acetylsalicylic acid, a steroid, an anti-T cell receptor antibody, an anti-IL-2 receptor antibody, basiliximab, daclizumab (ZENAPAX)®), anti T cell receptor antibodies (e.g., Muromonab-CD3), azathioprine, a corticosteroid, cyclosporine, tacrolimus, mycophenolate mofetil, sirolimus, calcineurin inhibitors, and the like. In a specific embodiment, the immumosuppressive agent is a neutralizing antibody to macrophage inflammatory protein (MIP)-1α or MIP-1β.

In certain embodiments of any of the organoids disclosed herein, said organoids maintain said at least one physiological function for 1, 2, 3, 4, 5, 6, or 7 days, or for 1, 2, 3, 4, 5, 6, 7, 8, 9, 10 or more weeks after administration to an individual.

In certain specific embodiments of any of the organoids presented herein, said organoids perform at least one function of a liver, kidney, pancreas, thyroid or lung.

The organoids can comprise pituitary-specific cells, and/or cells that perform pituitary-specific functions. In certain embodiments, any of the organoids presented herein comprises pituitary gland acidophil cells. In certain other embodiments, any of the organoids presented herein comprises pituitary basophil cells. In certain other embodiments, any of the organoids presented herein comprises both pituitary gland acidophil cells and basophil cells. In another embodiment, any of the organoids presented herein comprises pituitary somatotropes. In another embodiment, any of the organoids presented herein comprises pituitary mammotrophs. In another embodiment, any of the organoids presented herein comprises pituitary corticotrophs. In another embodiment, any of the organoids presented herein comprises pituitary thyrotrophs. In another embodiment, any of the organoids presented herein comprises pituitary gonadotrophs. In another embodiment, any of the organoids presented herein comprises said organoids comprise two or more of pituitary somatotrophs, pituitary mammotrophs, pituitary corticotrophs, pituitary thyrotrophs, and/or pituitary gonadotrophs. In another embodiment of any of the organoids presented herein, said organoids produce a measurable amount of growth hormone (GH) in *in vitro* culture. In another embodiment of any of the organoids presented herein, said organoids produce a measurable amount of somatotrophic hormone (STH) in *in vitro* culture. In another embodiment of any of the organoids presented herein, said organoids produce a measurable amount of prolactin (PRL) in *in vitro* culture. In another embodiment of any of the organoids presented herein, said organoids produce a measurable amount of adrenocorticotropic hormone (ACTH) in *in vitro* culture. In another embodiment of any of the organoids presented herein, said organoids produce a measurable amount of melanocyte-stimulating hormone (MSH) in *in vitro* culture. In another embodiment of any of the organoids presented herein, said organoids produce a measurable amount of thyroid-stimulating hormone (TSH) in *in vitro* culture. In another embodiment of any of the organoids presented herein, said organoids produce a measurable amount of follicle-stimulating hormone (FSH) in *in vitro* culture. In another embodiment of any of the organoids presented herein, said organoids produce a measurable amount of leutinizing hormone (LH) in *in vitro* culture. In another embodiment of any of the organoids presented herein, said organoids comprise cells that produce one or more of GH, STH, PRL, ACTH, MSH, TSH, FSH, and/or LH. In a specific embodiment, said cells have been genetically engineered to produce one or more of GH, STH, PRL, ACTH, MSH, TSH, FSH, and/or LH.

In another embodiment of any of the organoids presented herein, said organoids comprise hypothalamic neurons and/or pituicytes. In another embodiment of any of the organoids presented herein, said organoids produce a measurable amount of antidiuretic hormone (ADH) in *in vitro* culture. In another embodiment of any of the organoids presented herein, said organoids produce a measurable amount of oxytocin in *in vitro* culture. In another embodiment of any of the organoids presented herein, said organoids comprise cells that produce one or both of ADH and/or oxytocin. In a specific embodiment, said organoids comprise cells that have been genetically engineered to produce one or both of ADH and/or oxytocin.

In specific embodiments, any of the organoids provided herein comprise endothelial vessel-forming cells.

The organoids can comprise thyroid gland-specific cells, and/or cells that perform thyroid gland-specific functions. In certain embodiments, any of the organoids provided herein comprise thyroid epithelial cells. In certain embodiments, any of the organoids provided herein comprise thyroid parafollicular cells. In certain embodiments, any of the organoids provided herein comprise thyroglobulin-producing cells. In certain embodiments, any of the organoids provided herein comprise two or more of thyroid epithelial cells, thyroid parafollicular cells, and thyroglobulin-producing cells. In specific embodiments, any of the organoids provided herein comprise endothelial vessel-forming cells. In other specific embodiments, said organoids comprise a plurality of vessels, *e.g.,* blood vessels and/or lymphatic vessels. In certain embodiments of any of the organoids presented herein, said organoids produce a measurable amount of thyroxine (T4) in *in vitro* culture. In certain other embodiments of any of the organoids presented herein, said organoids produce a measurable amount of triiodothyronine (T3) in *in vitro* culture. In certain other embodiments of any of the organoids presented herein, said organoids produce a measurable amount of calcitonin. In certain other embodiments of any of the organoids presented herein, said organoids comprise cells that produce one or more of T3, T4 and/or calcitonin. In more specific embodiments, said organoids comprise cells genetically engineered to produce one or more of T3, T4 and/or calcitonin.

The organoids can comprise parathyroid gland-specific cells, or cells that perform parathyroid-specific functions. In certain embodiments of any of the organoids presented herein, said organoids comprise parathyroid chief cells. In other embodiments of any of the organoids presented herein, said organoids comprise parathyroid oxyphil cells. In other embodiments of any of the organoids presented herein, said organoids comprise both parathyroid chef cells and parathyroid oxyphil cells. In certain embodiments, any of the organoids provided herein comprise endothelial vessel-forming cells. In other specific embodiments, said organoids comprise a plurality of vessels, *e.g.,* blood vessels and/or lymphatic vessels. In certain embodiments of any of the organoids presented herein, said organoids produce a measurable amount of parathyroid hormone (PTH) in *in vitro* culture. In other embodiments of any of the organoids presented herein, said organoids comprise cells that produce PTH. In more specific embodiments, said organoids comprise cells that have been genetically engineered to produce said PTH.

The organoids can comprise adrenal gland-specific cells, and/or cells that perform adrenal gland-specific functions. In certain embodiments of any of the organoids presented herein, said organoids comprise adrenal gland zona glomerulosa cells. In other embodiments of any of the organoids presented herein, said organoids comprise adrenal gland fasciculate cells. In other embodiments of any of the organoids presented herein, said organoids comprise adrenal gland zona reticulata cells. In other embodiments of any of the organoids presented herein, said organoids comprise adrenal gland chromaffin cells. In certain embodiments, any of the organoids provided herein comprise endothelial vessel-forming cells. In other specific embodiments, said organoids comprise a plurality of vessels, *e.g.,* blood vessels and/or lymphatic vessels. In certain embodiments of any of the organoids presented herein, said organoids produce a measurable amount of aldosterone in *in vitro* culture. In other embodiments of any of the organoids presented herein, said organoids produce a measurable amount of 18 hydroxy 11 deoxycorticosterone in *in vitro* culture. In other embodiments of any of the organoids presented herein, said organoids produce a measurable amount of fludrocortisone in *in vitro* culture. In other embodiments of any of the organoids presented herein, said organoids produce a measurable amount of cortisol. In other embodiments of any of the organoids presented herein, said organoids produce a measurable amount of a non-cortisol glucocorticoid. In other embodiments of any of the organoids presented herein, said organoids produce a measurable amount of epinephrine. In other embodiments of any of the organoids presented herein, said organoids produce a measurable amount of adrenosterone. In other embodiments of any of the organoids presented herein, said organoids produce a measurable amount of dehydroepiandreosterone. In other embodiments of any of the organoids presented herein, said organoids comprise cells that produce one or more of aldosterone, 18 hydroxy 11 deoxycorticosterone, cortisol, fludrocortisones, a non-cortisol glucocorticoid, epinephrine, adrenosterone, and/or dehydroepiandrosterone. In other embodiments of any of the organoids presented herein, said organoids produce two or more of aldosterone, 18 hydroxy 11 deoxycorticosterone, cortisol, fludrocortisones, a non-cortisol glucocorticoid, epinephrine, adrenosterone, and/or dehydroepiandrosterone. In more specific embodiments, said organoids comprise cells that have been genetically engineered to produce one or more of aldosterone, 18 hydroxy 11 deoxycorticosterone, cortisol, fludrocortisones, a non-cortisol glucocorticoid, epinephrine, adrenosterone, and/or dehydroepiandrosterone.

The organoids provided herein can comprise liver-specific cells, or cells that perform one or more liver-specific functions. In certain embodiments of any of the organoids provided herein, said organoids comprise hepatocytes. In various embodiments of any of the organoids provided herein, said organoids produce a measurable amount of one or more of coagulation factor I (fibrinogen); coagulation factor II (prothrombin); coagulation factor V (factor five); coagulation factor VII (proconvertin); coagulation factor IX (Christmas factor); coagulation factor X (Stuart-Prower factor; prothrombinase); coagulation factor XI (plasma thromboplastin antecedent); protein C (autoprothrombin IIA; blood coagulation factor XIV), protein S and/or antithrombin. In various other embodiments of any of the organoids provided herein, said organoids produce detectable amounts of glucose from an amino acid, lactate, glycerol or glycogen. In other embodiments, said organoids produce detectable amounts of insulin-like growth factor (IGF-1) or thrombopoietin. In other embodiments, said organoids produce bile. In certain embodiments of any of the organoids provided herein, said organoids comprise cells that produce one or more of coagulation factor I (fibrinogen); coagulation factor II (prothrombin); coagulation factor V (factor five); coagulation factor VII (proconvertin); coagulation factor IX (Christmas factor); coagulation factor X (Stuart-Prower factor; prothrombinase); coagulation factor XI (plasma thromboplastin antecedent); protein C (autoprothrombin IIA; blood coagulation factor XIV), protein S, antithrombin, IGF-1 or thrombopoietin. In certain embodiments of any of the organoids provided herein, said organoids comprise hepatic vessel endothelial cells. In a specific embodiment, said hepatic vessel endothelial cells are disposed within said organoids so as to define one or more vessels. In a more specific embodiment, said hepatocytes are disposed along and substantially parallel to said vessels.

The organoids provided herein can also comprise pancreatic cells, or can comprise cells that perform at least one pancreatic cell-specific function. In certain embodiments, said pancreatic cells are pancreatic alpha cells. In certain embodiments of any of the organoids provided herein, said organoids comprise pancreatic beta cells. In other embodiments of any of the organoids provided herein, said organoids comprise pancreatic delta cells. In other embodiments of any of the organoids provided herein, said organoids comprise pancreatic PP cells. In other embodiments of any of the organoids provided herein, said organoids comprise pancreatic epsilon cells. In other embodiments of any of the organoids provided herein, said organoids comprise two or more of pancreatic alpha cells, pancreatic beta cells, pancreatic delta cells, pancreatic PP cells, and/or pancreatic epsilon cells. In other embodiments of any of the organoids provided herein, said organoids produce a detectable amount of glucagon. In other embodiments of any of the organoids provided herein, said organoids produce a detectable amount of insulin. In other embodiments of any of the organoids provided herein, said organoids produce a detectable amount of amylin. In a more specific embodiment, said organoids produce a detectable amount of insulin and a detectable amount of amylin. In a more specific embodiment, said insulin and said amylin in a ratio of about 50:1 to about 200:1. In other embodiments of any of the organoids provided herein, said organoids produce a detectable amount of somatostatin. In other embodiments of any of the organoids provided herein, said organoids produce a detectable amount of grehlin. In other embodiments of any of the organoids provided herein, said organoids produce a detectable amount of pancreatic polypeptide. In other embodiments of any of the organoids provided herein, said organoids comprise cells that produce a detectable amount of one or more of insulin, glucagon, amylin, somatostatin, pancreatic polypeptide, and/or grehlin.

In another aspect, provided herein are methods of using the organoids provided herein in methods of treating individuals, e.g., individuals suffering a deficiency in one or more biomolecules or physiological functions of an organ or tissue. In one embodiment, for example, provided herein is a method of treating an individual in need of human growth hormone (hGH) comprising administering to said individual an organoid that produces hGH, or an organoid comprising cells that produce hGH, e.g., a therapeutically effective amount of hGH. In certain other embodiments, provided herein is a method of treating an individual in need of somatotrophic hormone (STH) comprising administering to said individual an organoid that produces, or an organoid that comprises cells that produce, STH, e.g., a therapeutically effective amount of STH.

In another embodiment, provided herein is a method of treating an individual in need of prolactin (PRL) comprising administering to said individual organoid that produces, or an organoid that comprises, cells that produce, PRL, e.g., a therapeutically effective amount of PRL. In specific embodiment, said individual has one or more of metabolic syndrome, arteriogenic erectile dysfunction, premature ejaculation, oligozoospermia, asthenospermia, hypofunction of seminal vesicles, or hypoandrogenism.

In another embodiment, provided herein is a method of treating an individual in need of adrenocorticotropic hormone (ACTH), comprising administering to said individual an organoid that produces, or an organoid that comprises cells that produce, ACTH, e.g., a therapeutically effective amount of ACTH. In a specific embodiment, said individual has Addison's disease.

In another embodiment, provided herein is a method of treating an individual in need of melanocyte-stimulating hormone (MSH), comprising administering to said individual an organoid that produces, or an organoid that comprises cells that produce, MSH, e.g., a therapeutically effective amount of MSH. In a specific embodiment, said individual has Alzheimer's disease.

In another embodiment, provided herein is a method of treating an individual in need of thyroid-stimulating hormone (TSH), comprising administering to said individual an organoid that produces, or an organoid that comprises cells that produce, TSH, e.g., a therapeutically effective amount of TSH. In a specific embodiment, said individual has or manifests cretinism.

In another embodiment, provided herein is a method of treating an individual in need of follicle-stimulating hormone (FSH), comprising administering to said individual an organoid that produces, or an organoid that comprises cells that produce, FSH, e.g., a therapeutically effective amount of FSH. In a specific embodiment, said individual has or manifests infertility or azoospermia.

In another embodiment, provided herein is method of treating an individual in need of leutenizing hormone (LH) comprising administering to said individual an organoid that produces, or an organoid that comprises cells that produce, LH, e.g., a therapeutically effective amount of LH. In a specific embodiment, said individual has or manifests low testosterone, low sperm count or infertility.

Further provided herein is a method of treating an individual in need of antidiuretic hormone (ADH), comprising administering to said individual an organoid that produces, or an organoid that comprises cells that produce, ADH, e.g., a therapeutically effective amount of ADH.. In a specific embodiment, said individual has hypothalamic diabetes insipidus.

In another embodiment, provided herein is a method of treating an individual in need of oxytocin, comprising administering to said individual an organoid that produces, or an organoid that comprises cells that produce, oxytocin, e.g., a therapeutically effective amount of oxytocin.

In another embodiment, provided herein is a method of treating an individual in need of thyroxine (T4), comprising administering to said individual an organoid that produces, or an organoid that comprises cells that produce, T4, e.g., a therapeutically effective amount of T4. In a specific embodiment, said individual has or manifests mental retardation, dwarfism, weakness, lethargy, cold intolerance, or moon face.

In another embodiment, provided herein is a method of treating an individual in need of triiodothyronine (T3), comprising administering to said individual an organoid that produces, or an organoid that comprises cells that produce, T3, e.g., a therapeutically effective amount of T3. In a specific embodiment, said individual has heart disease. In a more specific embodiment, said individual, prior to administration of said organoid, has a serum concentration of T3 that is less than 3.1 pmol/L.

In another embodiment, provided herein is a method of treating an individual in need of calcitonin, comprising administering to said individual an organoid that produces, or an organoid that comprises cells that produce, calcitonin, e.g., a therapeutically effective amount of calcitonin. In a specific embodiment, said individual has osteoporosis or chronic autoimmune hypothyroidism.

Further provided herein is a method of treating an individual in need of parathyroid hormone (PTH), comprising administering to said individual an organoid that produces, or an organoid that comprises cells that produce, PTH, e.g., a therapeutically effective amount of PTH.

In another embodiment, provided herein is a method of treating an individual in need of aldosterone, comprising administering to said individual produces, or an organoid that comprises cells that produce, aldosterone, e.g., a therapeutically effective amount of aldosterone. In a specific embodiment, said individual has idiopathic hypoaldosteronism, hypereninemic hypoaldosteronism, or hyporeninemic hypoaldosteronism. In another specific embodiment, said individual has chronic renal insufficiency.

In another embodiment, provided herein is a method of treating an individual in need of 18 hydroxy 11 deoxycorticosterone comprising administering to said individual an organoid that produces, or an organoid that comprises cells that produce, 18 hydroxy 11 deoxycorticosterone, e.g., a therapeutically effective amount of 18 hydroxy 11 deoxycorticosterone.

Further provided herein is a method of treating an individual in need of fludrocortisone comprising administering to said individual an organoid that produces, or an organoid that comprises cells that produce, fludrocortisone a therapeutically effective amount of fludrocortisone.

In another embodiment, provided herein is a method of treating an individual in need of cortisol, the method comprising administering to said individual an organoid that produces, or an organoid that comprises cells that produce, cortisol, e.g., a therapeutically effective amount of cortisol. In a specific embodiment, said individual has acute adrenal deficiency, Addison's disease, or hypoglycemia.

In another embodiment, provided herein is a method of treating an individual in need of a non-cortisol glucocorticoid, the method comprising administering to said individual an organoid that produces, or an organoid that comprises cells that produce, non-cortisol glucocorticoid, e.g., a therapeutically effective amount of said non-cortisol glucocorticoid.

Further provided herein is a method of treating an individual in need of epinephrine, the method comprising administering to said individual an organoid that produces, or an organoid that comprises cells that produce, epinephrine, e.g., a therapeutically effective amount of epinephrine.

In another embodiment, provided herein is a method of treating an individual in need of adrenosterone, comprising administering to said individual an organoid that produces, or an organoid that comprises cells that produce, adrenosterone, e.g., a therapeutically effective amount of adrenosterone.

In another embodiment, provided herein is a method of treating an individual in need of dehydroepiandrosterone comprising administering to said individual a plurality of, *e.g.,* a therapeutically effective amount of, organoids producing, or comprising cells that produce, dehydroepiandrosterone.

In another embodiment, provided herein is a method of treating an individual in need of a compound, comprising administering an organoid that produces, or an organoid that comprises cells that produce, said compound, wherein said compound is coagulation factor I (fibrinogen); coagulation factor II (prothrombin); coagulation factor V (factor five); coagulation factor VII (proconvertin); coagulation factor IX (Christmas factor); coagulation factor X (Stuart-Prower factor; prothrombinase); coagulation factor XI (plasma thromboplastin antecedent); protein C (autoprothrombin IIA; blood coagulation factor XIV), protein S and/or antithrombin, e.g., a therapeutically effective amount of said compound.

In another embodiment, provided herein is a method of treating an individual in need of IGF-1, comprising administering to said individual an organoid that produces, or an organoid that comprises cells that produce, IGF-1, e.g., a therapeutically effective amount of IGF-1.

In another embodiment, provided herein is a method of treating an individual in need of thrombopoietin, comprising administering to said individual an organoid that produces, or an organoid that comprises cells that produce, thrombopoietin, e.g., a therapeutically effective amount of thrombopoietin.

In another embodiment, provided herein is a method of treating an individual in need of glucagon, comprising administering to said individual an organoid that produces, or an organoid that comprises cells that produce, glucagon, e.g., a therapeutically effective amount of glucagon.

In another embodiment, provided herein is a method of treating an individual in need of insulin, comprising administering to said individual an organoid that produces, or an organoid that comprises cells that produce, insulin, e.g., a therapeutically effective amount of insulin. In a specific embodiment, said individual has diabetes mellitus.

In another embodiment, provided herein is a method of treating an individual in need of amylin, comprising administering to said individual an organoid that produces, or an organoid that comprises cells that produce, amylin, e.g., a therapeutically effective amount of amylin.

In another embodiment, provided herein is a method of treating an individual in need of grehlin, comprising administering to said individual an organoid that produces, or an organoid that comprises cells that produce, grehlin, e.g., a therapeutically effective amount of grehlin.

Further provided herein is a method of treating an individual in need of pancreatic polypeptide, comprising administering to said individual an organoid that produces, or an organoid that comprises cells that produce, pancreatic polypeptide, e.g., a therapeutically effective amount of pancreatic polypeptide.

"Organoid," as used herein, means a combination of at least one type of cell and placental vascular scaffold or portion thereof, wherein the combination performs at least one physiological function of a tissue, gland or organ. In certain embodiments, the placental vascular scaffold of the organoids described herein comprises decellularized human placental vascular scaffold (DHPVS). In certain embodiments, the organoids described herein comprise an entire DHPVS, that is, an entire human placenta comprising placental vasculature that has been decellularized in accordance with the methods described herein. In certain embodiments, the organoids described herein comprise a portion of a placenta, e.g., a portion of a DHPVS. In a specific embodiment, the organoids described herein comprise a portion of a placenta, e.g., a portion of a DHPVS, wherein said portion comprises one or more regions of the placenta that comprise vasculature, e.g., one or more cotyledons, which are separations of the decidua basalis of the placenta that comprise distinct vascular domains. In another specific embodiment, the organoids described herein comprise a portion of a placenta, e.g., a portion of a DHPVS, wherein said portion comprises a portion of the placenta that has been removed from the remainder of the placenta and decellularized according to the methods described herein, either prior or subsequent to such removal from the remainder of the placenta. For example, the portion is of a desired size and shape, e.g., a cube, that has been removed from (e.g., excised out of or stamped out of) the placenta (e.g., the DHPVS).

In certain embodiments, the methods of generating organoids described herein comprise bioprinting of one or more cell types onto or into decellularized placental vascular scaffold. Bioprinting," as used herein, generally refers to the deposition of material, such as living cells, and, optionally, other components (e.g., extracellular matrix; synthetic matrices) onto a surface using standard or modified printing technology, e.g., ink jet printing technology. Basic methods of depositing cells onto surfaces, and of bioprinting cells, including cells in combination with hydrogels, are described in Warren et al. US 6,986,739, Boland et al. US 7,051,654, Yoo et al. US 2009/0208466 and Xu et al. US 2009/0208577, the disclosures of each of which are incorporated by reference herein their entirety. Additionally, bioprinters useful for production of the organoids provided herein are commercially available, e.g., the 3D-Bioplotter™ from Envisiontec GmbH (Gladbeck, Germany); and the NovoGen MMX Bioprinter™ from Organovo (San Diego, CA).

### 3.1 BRIEF DESCRIPTION OF DRAWINGS

Fig. 1 depicts the growth of 293/GFP cells grown on decellularized human placental vascular scaffold (DHPVS; "PI plus cells") as compared to the growth of such cells in control medium ("cells").
Fig. 2 depicts the growth of placental stem cells on DHPVS ("with matrix") as compared to the growth of such cells in control medium ("no matrix").
Fig. 3 depicts the growth over time of hepatocyte cells in DHPVS.
Fig. 4 depicts the results of an albumin secretion assay. Secretion of albumin by hepatocyte cells cultured in DHPVS ("T+C") was compared to levels of albumin in medium alone ("Med"), DHPVS alone ("Tissue"), and hepatocyte cells grown in culture ("Cell").
Fig. 5 depicts scaffolds comprising polycaprolactone (PCL) that were bioprinted at various angles and in such a way that scaffolds of various pore sizes were generated.
Fig. 6 depicts multiple views of bioprinted scaffolds onto which extracellular matrix (ECM) has been applied to both sides of the scaffold and subsequently dehydrated.
Fig. 7 depicts the results of a cell proliferation assay. Placental stem cells cultured on a hybrid scaffold comprising bioprinted PCL and dehydrated ECM proliferate over an 8-day culture period.
Fig. 8 depicts the results of a cell viability assay. Placental stem cells cultured on a hybrid scaffold comprising bioprinted PCL and dehydrated ECM proliferated and remained viable over an 8-day culture period.
Fig. 9 depicts an intact three-dimensional hybrid scaffold comprising PCL, ECM, and placental stem cells, each of which were bioprinted as layers (layers of PCL and layers of ECM/cells).
Fig. 10 demonstrates that placental stem cells distribute throughout three-dimensional bioprinted scaffolds over a 7-day culture period.
Fig. 11 depicts the results of a cell viability assay. Placental stem cells bioprinted with ECM and PCL to form a three-dimensional hybrid scaffold proliferate and remain viable over a 7-day culture period.
Fig. 12 demonstrates that stem cells bioprinted with ECM and PCL to form a three-dimensional hybrid scaffold spread throughout the ECM in the hybrid scaffolds over a 7-day culture period.
Fig. 13 depicts the results of a cell proliferation assay. Placental stem cells cultured in a three-dimensional hybrid scaffold that was generated by bioprinting PCL, ECM, and placental stem cells proliferate over a 7-day culture period.
Fig. 14 depicts viability of TT cells and HUVEC following co-culture in the presence or absence of decellularized placental vascular scaffold and following culture alone in the presence or absence of decellularized placental vascular scaffold.
Fig. 15 depicts calcitonin production by TT cells following co-culture with HUVEC in the presence or absence of decellularized placental vascular scaffold and following culture alone in the presence or absence of decellularized placental vascular scaffold. Calcitonin production by HUVEC cultured alone in the presence or absence of decellularized placental vascular scaffold also is presented.
Fig. 16 depicts viability of TT cells and PDAC® following co-culture in the presence or absence of decellularized placental vascular scaffold and following culture alone in the presence or absence of decellularized placental vascular scaffold.
Fig. 17 depicts a time course of calcitonin production by TT cells following co-culture with PDAC® in the presence or absence of decellularized placental vascular scaffold and following culture alone in the presence or absence of decellularized placental vascular scaffold. A time course of calcitonin production by PDAC® cultured alone in the presence or absence of decellularized placental vascular scaffold also is presented.
Fig. 18 depicts a time course of HGF production by PDAC® following co-culture with TT cells in the presence or absence of decellularized placental vascular scaffold and following culture alone in the presence or absence of decellularized placental vascular scaffold. A time course of HGF production by TT cells cultured alone in the presence or absence of decellularized placental vascular scaffold also is presented.
Fig. 19 depicts the distribution pattern of HCT116 cells following infusion into the vasculature of a decellularized placental vascular scaffold.
Fig. 20 depicts adiponectin production by PDAC® cultured in the presence or absence of adipocyte differentiation medium and either alone or on decellularized placental vascular scaffold.
Fig. 21 depicts metabolism of HepaRG cultured on decellularized placental vascular scaffold. (A) levels of glucose and lactate in culture medium following time course of culture of PDAC® alone or on decellularized placental vascular scaffold. (B-D) ΔL/ΔG value for PDAC® cultured alone or on decellularized placental vascular scaffold at 3^{rd} week, 2^{nd} week, and 4^{th} week of culture, respectively.

### 4. DETAILED DESCRIPTION

Provided herein is an organoid as defined in the claims comprising one or more types of cells, and decellularized placental vascular scaffold, wherein said organoid performs at least one function of an organ, or a tissue from an organ, wherein said at least one function of an organ or tissue from an organ is production of a protein, growth factor, cytokine, interleukin, or small molecule characteristic of at least one cell type from said organ or tissue; and wherein said decellularized placental vascular scaffold comprises substantially intact placental vasculature matrix; that is, the structure of the vasculature of the placenta from which the matrix is obtained is substantially preserved during decellularization and subsequent production of the organoids and wherein said decellularized placental vascular scaffold comprises substantially intact placental vasculature matrix;wherein said cells have been genetically engineered to produce a protein or polypeptide not naturally produced by the cell, or have been genetically engineered to produce a protein or polypeptide in an amount greater than that naturally produced by the cell. In certain embodiments, once the organoid is completed, blood or other nutrient solution is passed through said placental vasculature.

### 4.1. Methods of Obtaining Placenta

Generally, a human placenta is recovered shortly after its expulsion after normal birth, or after a Caesarian section. In a preferred embodiment, the placenta is recovered from a patient after informed consent and after a complete medical history of the patient is taken and is associated with the placenta. Preferably, the medical history continues after delivery. Such a medical history can be used to coordinate subsequent use of the placenta or the stem cells harvested therefrom. For example, human placental stem cells can be used, in light of the medical history, for personalized medicine for the infant associated with the placenta, or for parents, siblings or other relatives of the infant.

The umbilical cord blood and placental blood are removed, and can be used for other purposes or discarded. In certain embodiments, after delivery, the cord blood in the placenta is recovered. The placenta can be subjected to a conventional cord blood recovery process. Typically a needle or cannula is used, with the aid of gravity, to exsanguinate the placenta (see, e.g., Anderson, U.S. Pat. No. 5,372,581; Hessel et al., U.S. Pat. No. 5,415,665). The needle or cannula is usually placed in the umbilical vein and the placenta can be gently massaged to aid in draining cord blood from the placenta. Such cord blood recovery may be performed commercially, e.g., by LifeBank USA, Cedar Knolls, N.J. Preferably, the placenta is gravity drained without further manipulation so as to minimize tissue disruption during cord blood recovery.

Typically, a placenta is transported from the delivery or birthing room to another location, e.g., a laboratory, for recovery of cord blood and collection of stem cells by, e.g., perfusion or tissue dissociation. The placenta is preferably transported in a sterile, thermally insulated transport device (maintaining the temperature of the placenta between about 20°C to about 28°C), for example, by placing the placenta, with clamped proximal umbilical cord, in a sterile zip-lock plastic bag, which is then placed in an insulated container. In another embodiment, the placenta is transported in a cord blood collection kit substantially as described in pending U.S. Pat. No. 7,147,626. Preferably, the placenta is delivered to the laboratory four to twenty-four hours following delivery. In certain embodiments, the proximal umbilical cord is clamped, preferably within 4-5 cm (centimeter) of the insertion into the placental disc prior to cord blood recovery. In other embodiments, the proximal umbilical cord is clamped after cord blood recovery but prior to further processing of the placenta.

The placenta can be stored under sterile conditions and at either room temperature or at a temperature of 5°C to 25°C. The placenta may be stored for a period of for a period of four to twenty-four hours, up to forty-eight hours, or longer than forty eight hours, prior to perfusing the placenta to remove any residual cord blood. In one embodiment, the placenta is harvested from between about zero hours to about two hours post-expulsion. The placenta is preferably stored in an anticoagulant solution at a temperature of 5°C to 25°C. Suitable anticoagulant solutions are well known in the art, e.g., a solution of heparin or warfarin sodium. In a preferred embodiment, the anticoagulant solution comprises a solution of heparin (e.g., 1% w/w in 1:1000 solution). The exsanguinated placenta is preferably stored for no more than 36 hours before placental stem cells are collected.

The placenta may also be perfused, e.g., to collect placental stem cells and/or placental perfusate cells, e.g., as described in U.S. Patent No. 7,468,276.

In certain embodiments, an organoid described herein comprises only a portion of a decellularized placenta obtained in accordance with the above-described methods. For example, the placenta may be manipulated to obtain the desired portion, e.g., to obtain a desired placental circulatory unit (e.g., a cotyledon) before the portion of the placenta is further processed (e.g., processed as described herein, e.g., decellularized). In certain embodiments, when only a portion of a placenta is used in the generation of the organoids described herein, the entire placenta is processed as desired (e.g., decellularized as described below), followed by isolation of the specific portion of the placenta to be used (e.g., by cutting or stamping out the desired portion of the placenta from the whole processed placenta).

### 4.2. Methods of Decellularizing Placenta

Once the placenta is prepared as above, and optionally perfused, it is decellularized in such a manner as to preserve the native structure of the placental vasculature, e.g., leave the placental vasculature substantially intact. As used herein, "substantially intact" means that the placental vasculature remaining after decellularization retains all, or most, of the gross structure of the placental vasculature prior to decellularization. In certain embodiments, the placental vasculature is capable of being re-seeded, e.g., with vascular endothelial cells or other cells, so as to recreate the placental vasculature.

Placental tissue may be sterilized, e.g., by incubation in a sterile buffered nutrient solution containing antimicrobial agents, for example an antibacterial, an antifungal, and/or a sterilant compatible with the transplant tissue. The sterilized placental tissue may then be cryopreserved for further processing at a later time or may immediately be further processed according to the next steps of this process including a later cryopreservation of the tissue matrix or other tissue products of the process.

Several means of reducing the viability of native cells in tissues and organs are known, including physical, chemical, and biochemical methods. See, e.g. U.S. Pat. No. 5,192,312 (Orton) which is incorporated herein by reference. Such methods may be employed in accordance with the process described herein. However, the decellularization technique employed preferably does not result in gross disruption of the anatomy of the placental tissue or substantially alter the biomechanical properties of its structural elements, and preferably leaves the placental vasculature substantially intact. In certain embodiments, the treatment of the placental tissue to produce a decellularized tissue matrix does not leave a cytotoxic environment that mitigates against subsequent repopulation of the matrix with cells that are allogeneic or autologous to the recipient. As used herein, cells and tissues that are "allogeneic" to the recipient are those that originate with or are derived from a donor of the same species as a recipient of the placental vascular scaffold, and "autologous" cells or tissues are those that originate with or are derived from a recipient of the placental vascular scaffold.

Physical forces, for example the formation of intracellular ice, can be used to decellularize transplant tissues. As such, in certain embodiment, the placenta is first cryopreserved as part of decellularization. For example, vapor phase freezing (slow rate of temperature decline) of placental tissue can be performed. Optionally, the placental tissue is cryopreserved in the presence of one or more cryoprotectants. Colloid-forming materials may be added during freeze-thaw cycles to alter ice formation patterns in the tissue. For example, polyvinylpyrrolidone (10% w/v) and dialyzed hydroxyethyl starch (10% w/v) may be added to standard cryopreservation solutions (DMEM, 10% DMSO, 10% fetal bovine serum) to reduce extracellular ice formation while permitting formation of intracellular ice.

In certain embodiments, various enzymatic or other chemical treatments to eliminate viable native cells from implant tissues or organs may be used. For instance, extended exposure of cells to proteases such as trypsin result in cell death.

In certain other embodiments, the placental tissue is decellularized using detergents or combinations thereof, for example, a nonionic detergent, e.g., Triton X-100, and an anionic detergent, e.g., sodium dodecyl sulfate, may disrupt cell membranes and aid in the removal of cellular debris from tissue. Preferably, residual detergent in the decellularized tissue matrix is removed, e.g., by washing with a buffer solution, so as to avoid interference with the later repopulating of the tissue matrix with viable cells.

The decellularization of placental tissue is preferably accomplished by the administration of a solution effective to lyse native placental cells. Preferably, the solution is an aqueous hypotonic or low ionic strength solution formulated to effectively lyse the cells. In certain embodiments, the aqueous hypotonic solution is, e.g. deionized water or an aqueous hypotonic buffer. In specific embodiments, the aqueous hypotonic buffer contains one or more additives that provide sub-optimal conditions for the activity of one or more proteases, for example collagenase, which may be released as a result of cellular lysis. Additives such as metal ion chelators, for example 1,10-phenanthroline and ethylenediaminetetraacetic acid (EDTA), create an environment unfavorable to many proteolytic enzymes. In other embodiments, the hypotonic lysis solution is formulated to eliminate or limit the amount of divalent cations, e.g., calcium and/or zinc ions, available in solution, which would, in turn, reduce the activity of proteases dependent on such ions.

Preferably, the hypotonic lysis solution is prepared selecting conditions of pH, reduced availability of calcium and zinc ions, presence of metal ion chelators and the use of proteolytic inhibitors specific for collagenase such that the solution will optimally lyse the native cells while protecting the underlying tissue matrix from proteolytic degradation. In certain embodiments, a hypotonic lysis solution may include a buffered solution of water, pH 5.5 to 8, preferably pH 7 to 8, free or substantially free from calcium and zinc ions, and/or including a metal ion chelator such as EDTA. Additionally, control of the temperature and time parameters during the treatment of the tissue matrix with the hypotonic lysis solution may also be employed to limit the activity of proteases.

In some embodiments, decellularization of placental tissue includes treatment of the tissue with one or more nucleases, e.g., effective to inhibit cellular metabolism, protein production and cell division without degrading the underlying collagen matrix. Nucleases that can be used for digestion of native cell DNA and RNA include either or both of exonucleases or endonucleases. Suitable nucleases for decellularization are commercially available. For example, exonucleases that effectively inhibit cellular activity include DNAase I (SIGMA Chemical Company, St. Louis, Mo.) and RNAase A (SIGMA Chemical Company, St. Louis, Mo.) and endonucleases that effectively inhibit cellular activity include EcoRI (SIGMA Chemical Company, St. Louis, Mo.) and Hind III (SIGMA Chemical Company, St. Louis, Mo.).

Selected nucleases may be contained in a physiological buffer solution which contains ions that are optimal for the activity of the nuclease, e.g., magnesium salts or calcium salts. It is also preferred that the ionic concentration of the buffered solution, the treatment temperature and the length of treatment are selected to assure the desired level of effective nuclease activity. The buffer is preferably hypotonic to promote access of the nucleases to cell interiors. In certain embodiments, the one or more nucleases comprise DNAase I and RNAase A. Preferably, the nuclease degradation solution contains about 0.1 microgram/mL to about 50 microgram/mL, or about 10 microgram/mL, of the nuclease DNAase I, and about 0.1 microgram/mL to about 10 microgram/mL, preferably about 1.0 microgram/mL, of RNAase A. The placental tissue may be decellularized by application of the foregoing enzymes at a temperature of about 20°C to 38°C, preferably at about 37°C, e.g., for about 30 minutes to 6 hours.

In other embodiments, the decellularization solution comprises one or more phospholipases, e.g. phospholipase A and/or phospholipase C, e.g., in a buffered solution. Preferably, the phospholipase as used should not have a detrimental effect on the tissue matrix protein. The pH of the vehicle, as well as the composition of the vehicle, will also be adjusted with respect to the pH activity profile of the enzyme chosen for use. Moreover, the temperature applied during application of the enzyme to the tissue is, in various embodiments, adjusted in order to optimize enzymatic activity.

Following decellularization, the tissue matrix in certain embodiments is washed in a wash solution to assure removal of cell debris which may include cellular protein, cellular lipids, and cellular nucleic acid, as well as any extracellular debris. Removal of this cellular and extracellular debris reduces the likelihood of the transplant tissue matrix eliciting an adverse immune response from the recipient upon implant. For example, the tissue may be washed one or more times with a wash solution, wherein the wash solution is, e.g., PBS or Hanks' Balanced Salt Solution (HBSS). The composition of the balanced salt solution wash, and the conditions under which it is applied to the transplant tissue matrix may be selected to diminish or eliminate the activity of proteases or nucleases utilized during the decellularization process. In specific embodiments, the wash solution does not contain magnesium or calcium, e.g. magnesium salts or calcium salts, and the washing process proceeds at a temperature of between about 2°C and 42°C, e.g., 4°C most preferable. The transplant tissue matrix may be washed, e.g., incubated in the balanced salt wash solution for up to 1, 2, 3, 4, 5, 6, 7, 8, 9, 10, 11 or 12 days, e.g., with changes in wash solution every -13 days. Optionally, an antibacterial, an antifungal or a sterilant or a combination thereof, may be included in the wash solution to protect the transplant tissue matrix from contamination with environmental pathogens. Washing may be performed by soaking the placental tissue with or without mild agitation.

The tissue matrix, once decellularized, can be preserved by cryopreservation. Techniques of cryopreservation of tissue are well known in the art. See, e.g., Brockbank, K. G. M., "Basic Principles of Viable Tissue Preservation," In: Transplantation Techniques and Use of Cryopreserved Allograft Cardiac Valves and Vascular Tissue, D. R. Clarke (ed.), Adams Publishing Group, Ltd., Boston, pp 9-23 (discussing cryopreservation of tissues and organs).

The tissue matrix, whether or not having been cryopreserved, in certain embodiments is treated to enhance the adhesion and inward migration of the allogeneic or autologous cells, in vitro, which will be used to repopulate the transplant tissue.

In certain embodiments, attachment of autologous or allogeneic cells to decellularized placental vascular scaffold may be increased, e.g., by contacting the placental vascular scaffold with serum (human or fetal bovine, maximal binding with 1% serum) and/or purified fibronectin, e.g., in culture medium in which the decellularized placental vascular scaffold is placed, e.g., in preparation for repopulation with allogeneic or autologous cells. Each of the two homologous subunits of fibronectin has two cell recognition regions, including one comprising the Arg-Gly-Asp (RGD) sequence. A second site, binding glycosaminoglycans, acts synergistically and appears to stabilize the fibronectin-cell interactions mediated by the RGD sequence.

As such, in a specific embodiment, the decellularized placental vascular scaffold is contacted with both fibronectin and a glycosaminoglycan, e.g., heparin, for a period effective for binding of the fibronectin to surfaces of the placental vascular scaffold to be repopulated with allogeneic or autologous cells. The fibronectin, and optionally glycosaminoglycan, can be included within a physiologically-acceptable buffer or culture medium, e.g., sodium phosphate/glycerin/bovine serum albumin and Dulbecco's Modified Eagle's Medium (DMEM) (e.g., GIBCO). The buffer or culture medium is preferably maintained at a physiologically acceptable pH, e.g., about 6.8 to 7.6. Fibronectin may be obtained from human blood, processed to limit contamination with virus, or may be obtained from commercial sources. The concentration of fibronectin and/or glycoprotein may range from about 1 microgram/mL to about 100 microgram/mL, e.g., about 10 microgram/mL. The preferred weight ratio of fibronectin to heparin is about 100:1 to about 1:100, or about 10:1 to about 1:10, e.g., 10:1 fibronectin:glycosaminoglycan, e.g. heparin.

The decellularized placental vascular scaffold may be contacted with, e.g., treated with, one or more compositions that act, e.g., to enhance cell chemotaxis, increasing the rate of directional movement along a concentration gradient of the substance in solution. With respect to fibroblast cells, fibroblast growth factor, platelet-derived growth factor, transforming growth factor-beta (TGF-β), fibrillar collagens, collagen fragments, and fibronectin are chemotactic.

In a specific, preferred embodiment, the placenta is decellularized as follows. Placental tissue, e.g., a whole placenta or lobule (cotyledon) of a placenta, from which blood has been removed is first frozen at -20°C to -180°C, e.g., about -80°C, e.g., for about 24 hours. The tissue is then thawed at about 4°C overnight. The thawed tissue is then digested with 0.1% trypsin at room temperature for 2 hours to 24 hours to produce digested placental tissue at 25°C to about 37°C. In this digestion, and in subsequent steps, solution is passed through the placental vasculature (perfusion decellularization). The digested tissue is then treated sequentially with 1%, 2% and 3% Triton-XlOO for 24 hours each at room temperature or about 25°C. The Triton-X100 treatments are then followed by treatment of the tissue with 0.1% SDS-PBS for 24 h at room temperature or at about 25°C, after which the cellular material is substantially removed. The tissue is then extensively washed with 1-10 changes of phosphate buffered saline (PBS), followed by treatment with DNase I (150 U/mL) for 1 hour at room temperature, each step at room temperature or about 25°C. Finally, the remaining decellularized placental vascular scaffold is again extensively washed at room temperature or about 25°C with PBS + 1% antibiotics (penicillin+streptomycin), optionally dried, and preserved at 4°C.

Following decellularization, the resulting placental vascular scaffold may be combined with one or more synthetic matrices, e.g., synthetic polymers. In a specific embodiment, the synthetic matrix stabilizes the three-dimensional structure of the placental vascular scaffold, e.g., to facilitate production of the organoid. In another specific embodiment, said synthetic matrix comprises a polymer or a thermoplastic. In a more specific embodiment, said synthetic matrix is a polymer or a thermoplastic. In more specific embodiments, said thermoplastic is polycaprolactone, polylactic acid, polybutylene terephthalate, polyethylene terephthalate, polyethylene, polyester, polyvinyl acetate, or polyvinyl chloride. In other more specific embodiments, said polymer is polyvinylidine chloride, poly(o-carboxyphenoxy)-p-xylene) (poly(o-CPX)), poly(lactide-anhydride) (PLAA), n-isopropyl acrylamide, acrylamide, pent erythritol diacrylate, polymethyl acrylate, carboxymethylcellulose, or poly(lactic-co-glycolic acid) (PLGA). In another more specific embodiment, said polymer is polyacrylamide.

In any of the above embodiments, the placental vascular scaffold may be decellularized by passage of any of the decellularizing and/or wash components described above through the placental vasculature, e.g., through the placental arteries and/or placental vein. Methods of perfusing through the placental vasculature are described, e.g., in U.S. Patent No. 8,057,78.

### 4.3. Methods of Loading Cells Onto the Matrix

Cells may be loaded onto the decellularized placental vascular scaffold by any physiologically-acceptable method. In certain embodiments, the cells are suspended in, e.g., a liquid culture medium, salt solution or buffer solution, and the cell-containing liquid is perfused into the placental vascular scaffold through one or more of the vascular matrices. The placental vascular scaffold may also be cultured in such a cell-containing liquid culture medium, salt solution or buffer solution for a time sufficient for a plurality of the cells to attach to said placental vascular scaffold. Cells may also be loaded onto the placental vascular matrix by seeding on the surface of the scaffold, or by injecting cells into the vessels using, e.g., a needle or an infusion pump. In certain embodiments, cells are loaded onto the decellularized placental vascular scaffold by bioprinting.

In certain embodiments after cells are loaded onto a decellularized placental vascular scaffold, the cells and scaffold are cultured for a desired period of time. In a specific embodiment, the cells and scaffold are cultured in a roller bioreactor.

### 4.4. Cells to Be Used

Depending on the physiological function(s) the organoids are designed to augment, or replace, the organoids provided herein can comprise one or more relevant cell types.

In certain embodiments of any of the organoids provided herein, for example, the one or more types of cells comprise cells of the immune system, *e.g.,* T cells, B cells, dendritic cells, and/or natural killer (NK) cells. In a specific embodiment, said NK cells comprise, or are, CD56⁺ CD16⁻ placental intermediate natural killer (PiNK) cells, *e.g.,* the placental NK cells described in US 2009/0252710.

In certain other embodiments of any of the organoids provided herein, the one or more types of cells are, or comprise, isolated stem cells or progenitor cells. In specific embodiments, said isolated stem cells or progenitor cells are isolated embryonic stem cells, embryonic germ cells, induced pluripotent stem cells, mesenchymal stem cells, bone marrow-derived mesenchymal stem cells, bone marrow-derived mesenchymal stromal cells, tissue plastic-adherent placental stem cells (PDAC®), umbilical cord stem cells, amniotic fluid stem cells, amnion derived adherent cells (AMDACs), osteogenic placental adherent cells (OPACs), adipose stem cells, limbal stem cells, dental pulp stem cells, myoblasts, endothelial progenitor cells, neuronal stem cells, exfoliated teeth derived stem cells, hair follicle stem cells, dermal stem cells, parthenogenically derived stem cells, reprogrammed stem cells, amnion derived adherent cells, or side population stem cells. In other specific embodiments, the one or more types of cells comprised within the organoids are, or comprise, isolated hematopoietic stem cells or hematopoietic progenitor cells. In other specific embodiments, the one or more types of cells comprised within the organoids are tissue culture plastic-adherent CD34⁻, CD10⁺, CD105⁺, and CD200⁺ placental stem cells, *e.g.,* the placental stem cells described in US 7,468,276 and US 8,057,788, the disclosures of which are hereby incorporated by reference in their entireties. In a specific embodiment, said placental stem cells are additionally one or more of CD45⁻, CD80⁻, CD86⁻, or CD90⁺. In a more specific embodiment, said placental stem cells are additionally CD45⁻, CD80⁻, CD86⁻, and CD90⁺.

Such placental stem cells are immunomodulatory. *See, e.g.,* US 7,682,803 and US 2008/0226595, the disclosures of which are hereby incorporated by reference in their entireties. In another specific embodiment, therefore, said placental stem cells, or said organoids comprising said placental stem cells, when said organoids are implanted into a recipient, suppress an immune response in said recipient. In another specific embodiment, any of said isolated stem cells recited above, or said organoids comprising said isolated stem cells, wherein said isolated stem cells are immunomodulatory, suppress an immune response in a recipient when said organoids are implanted into said recipient. In a specific embodiment, said organoids, or the immunomodulatory stem cells comprised therein, suppress an immune response locally within said recipient, e.g., at or adjacent to a site of administration or implantation. In another specific embodiment, said organoids, or the immunomodulatory stem cells comprised therein, suppress an immune response globally within said recipient.

In various other specific embodiments, the organoids comprise one or more cell types, wherein said one or more cell types are, or comprise, differentiated cells, *e.g.,* one or more of endothelial cells, epithelial cells, dermal cells, endodermal cells, mesodermal cells, fibroblasts, osteocytes, chondrocytes, natural killer cells, dendritic cells, hepatic cells, pancreatic cells, or stromal cells. In various more specific embodiments, said differentiated cells are, or comprise salivary gland mucous cells, salivary gland serous cells, von Ebner's gland cells, mammary gland cells, lacrimal gland cells, ceruminous gland cells, eccrine sweat gland dark cells, eccrine sweat gland clear cells, apocrine sweat gland cells, gland of Moll cells, sebaceous gland cells. bowman's gland cells, Brunner's gland cells, seminal vesicle cells, prostate gland cells, bulbourethral gland cells, Bartholin's gland cells, gland of Littre cells, uterus endometrium cells, isolated goblet cells, stomach lining mucous cells, gastric gland zymogenic cells, gastric gland oxyntic cells, pancreatic acinar cells, paneth cells, type II pneumocytes, clara cells, somatotropes, lactotropes, thyrotropes, gonadotropes, corticotropes, intermediate pituitary cells, magnocellular neurosecretory cells, gut cells, respiratory tract cells, thyroid epithelial cells, parafollicular cells, parathyroid gland cells, parathyroid chief cell, oxyphil cell, adrenal gland cells, chromaffin cells, Leydig cells, theca interna cells, corpus luteum cells, granulosa lutein cells, theca lutein cells, juxtaglomerular cell, macula densa cells, peripolar cells, mesangial cell, blood vessel and lymphatic vascular endothelial fenestrated cells, blood vessel and lymphatic vascular endothelial continuous cells, blood vessel and lymphatic vascular endothelial splenic cells, synovial cells, serosal cell (lining peritoneal, pleural, and pericardial cavities), squamous cells, columnar cells, dark cells, vestibular membrane cell (lining endolymphatic space of ear), stria vascularis basal cells, stria vascularis marginal cell (lining endolymphatic space of ear), cells of Claudius, cells of Boettcher, choroid plexus cells, pia-arachnoid squamous cells, pigmented ciliary epithelium cells, nonpigmented ciliary epithelium cells, corneal endothelial cells, peg cells, respiratory tract ciliated cells, oviduct ciliated cell, uterine endometrial ciliated cells, rete testis ciliated cells, ductulus efferens ciliated cells, ciliated ependymal cells, epidermal keratinocytes, epidermal basal cells, keratinocyte of fingernails and toenails, nail bed basal cells, medullary hair shaft cells, cortical hair shaft cells, cuticular hair shaft cells, cuticular hair root sheath cells, hair root sheath cells of Huxley's layer, hair root sheath cells of Henle's layer, external hair root sheath cells, hair matrix cells, surface epithelial cells of stratified squamous epithelium, basal cell of epithelia, urinary epithelium cells, auditory inner hair cells of organ of Corti, auditory outer hair cells of organ of Corti, basal cells of olfactory epithelium, cold-sensitive primary sensory neurons, heat-sensitive primary sensory neurons, Merkel cells of epidermis, olfactory receptor neurons, pain-sensitive primary sensory neurons, photoreceptor rod cells, photoreceptor blue-sensitive cone cells, photoreceptor green-sensitive cone cells, photoreceptor red-sensitive cone cells, proprioceptive primary sensory neurons, touch-sensitive primary sensory neurons, type I carotid body cells, type II carotid body cell (blood pH sensor), type I hair cell of vestibular apparatus of ear (acceleration and gravity), type II hair cells of vestibular apparatus of ear, type I taste bud cells, cholinergic neural cells, adrenergic neural cells, peptidergic neural cells, inner pillar cells of organ of Corti, outer pillar cells of organ of Corti, inner phalangeal cells of organ of Corti, outer phalangeal cells of organ of Corti, border cells of organ of Corti, Hensen cells of organ of Corti, vestibular apparatus supporting cells, taste bud supporting cells, olfactory epithelium supporting cells, Schwann cells, satellite cells, enteric glial cells, astrocytes, neurons, oligodendrocytes, spindle neurons, anterior lens epithelial cells, crystallin-containing lens fiber cells, hepatocytes, adipocytes, white fat cells, brown fat cells, liver lipocytes, kidney glomerulus parietal cells, kidney glomerulus podocytes, kidney proximal tubule brush border cells, loop of Henle thin segment cells, kidney distal tubule cells, kidney collecting duct cells, type I pneumocytes, pancreatic duct cells, nonstriated duct cells, duct cells, intestinal brush border cells, exocrine gland striated duct cells, gall bladder epithelial cells, ductulus efferens nonciliated cells, epididymal principal cells, epididymal basal cells, ameloblast epithelial cells, planum semilunatum epithelial cells, organ of Corti interdental epithelial cells, loose connective tissue fibroblasts, corneal keratocytes, tendon fibroblasts, bone marrow reticular tissue fibroblasts, nonepithelial fibroblasts, pericytes, nucleus pulposus cells, cementoblast/cementocytes, odontoblasts, odontocytes, hyaline cartilage chondrocytes, fibrocartilage chondrocytes, elastic cartilage chondrocytes, osteoblasts, osteocytes, osteoclasts, osteoprogenitor cells, hyalocytes, stellate cells (ear), hepatic stellate cells (Ito cells), pancreatic stelle cells, red skeletal muscle cells, white skeletal muscle cells, intermediate skeletal muscle cells, nuclear bag cells of muscle spindle, nuclear chain cells of muscle spindle, satellite cells, ordinary heart muscle cells, nodal heart muscle cells, Purkinje fiber cells, smooth muscle cells, myoepithelial cells of iris, myoepithelial cell of exocrine glands, reticulocytes, megakaryocytes, monocytes, connective tissue macrophages. epidermal Langerhans cells, dendritic cells, microglial cells, neutrophils, eosinophils, basophils, mast cell, helper T cells, suppressor T cells, cytotoxic T cell, natural Killer T cells, B cells, natural killer cells, melanocytes, retinal pigmented epithelial cells, oogonia/oocytes, spermatids, spermatocytes, spermatogonium cells, spermatozoa, ovarian follicle cells, Sertoli cells, thymus epithelial cell, and/or interstitial kidney cells.

In specific embodiments of any of the organoids comprising any of the cell types listed herein, the at least one type of cells are primary culture cells, cells that have been directly obtained from a tissue or organ without culturing, cells that have been cultured *in vitro,* or cells of a cell line, e.g., partially, conditionally, or fully immortalized cells.

Cells useful in the production of the organoids provided herein may be isolated from the relevant tissue or organs, e.g., from particular glands, using one or more art-known proteases, e.g., collagenase, dispase, trypsin, LIBERASE, or the like. Organ, e.g., gland tissue may be physically dispersed prior to, during, or after treatment of the tissue with a protease, e.g., by dicing, macerating, filtering, or the like. Cells may be cultured using standard, art-known cell culture techniques prior to production of the organoids, e.g., in order to produce homogeneous or substantially homogeneous cell populations, to select for particular cell types, or the like.

Isolation, culture, and identification of pituitary gland cells may be performed according to procedures known in the art, e.g., using lipocortin 1 (LC1) as a marker according to the procedures disclosed in Christian et al., "Characterization and localization of lipocortin 1-binding sites on rat anterior pituitary cells by fluorescence-activated cell analysis/sorting and electron microscopy," Endocrinology 138(12):5341-5351 (1997); see also Kim et al., "Isolation, culture and cell-type identification of adult human pituitary cells," Acta Neuropathol. 68(3):205-208 (1985); Baranowska et al., "Direct effect of cortistatin on GH release from cultured pituitary cells in the rat," Neuro Endocrinol Lett. 27(1-2):153-156 (2006).

Isolation, culture, and identification of thyroid gland cells may be performed according to procedures known in the art. See, e.g., Pavlov et al., "Isolation of cells for cytological and cytogenetic studies of the thyroid epithelium," Morfologiia 130(6):81-83 (2006); Fayet et al., "Isolation of a normal human thyroid cell line: hormonal requirement for thyroglobulin regulation," Thyroid 12(7):539-546 (2002).

Isolation, culture, and identification of adrenal gland cells may be performed according to procedures known in the art. See, e.g., Creutz, "Isolation of chromaffin granules," Curr Protoc Cell Biol. Chapter 3:Unit 3.39.1-10 (Sept. 2010); Caroccia et al., "Isolation of human adrenocortical aldosterone-producing cells by a novel immunomagnetic beads method," Endocrinology 151(3):1375-80 (2010); Fawcett et al., "Isolation and properties in culture of human adrenal capillary endothelial cells," Biochem Biophys Res Commun. 174(2):903-8 (1991); Notter et al., "Rodent and primate adrenal medullary cells in vitro: phenotypic plasticity in response to coculture with C6 glioma cells or NGF," Exp Brain Res. 76(1):38-46 (1989).

### 4.5. Physiological Functions Replicated By The Organoids

A primary function of the organoids provided herein is that the organoids, by the cells comprised within them, perform one or more physiological functions, e.g., one or more physiological functions in an individual that needs to be augmented or replaced. More specifically, the organoids and/or the cells comprised within them replicate or augment one or more physiological functions of an organ or a tissue in an individual who is a recipient of said organoids. In certain embodiments, as above, the organoids comprise isolated primary or cultured cells that perform the one or more physiological functions. In other embodiments, the organoids comprise cells have been genetically engineered to perform the physiological function. In a specific embodiment, said genetically engineered cells produce a protein or polypeptide not naturally produced by the corresponding un-engineered cells, or have been genetically engineered to produce a protein or polypeptide in an amount greater than that naturally produced by the corresponding un-engineered cells, wherein said cellular composition comprises differentiated cells.

In embodiments in which the physiological function is production of a protein or polypeptide, in specific embodiments, said protein or polypeptide is a cytokine or a peptide comprising an active part thereof. In more specific embodiments, said cytokine is adrenomedullin (AM), angiopoietin (Ang), bone morphogenetic protein (BMP), brain-derived neurotrophic factor (BDNF), epidermal growth factor (EGF), erythropoietin (Epo), fibroblast growth factor (FGF), glial cell line-derived neurotrophic factor (GNDF), granulocyte colony stimulating factor (G-CSF), granulocyte-macrophage colony stimulating factor (GM-CSF), growth differentiation factor (GDF-9), hepatocyte growth factor (HGF), hepatoma derived growth factor (HDGF), insulin-like growth factor (IGF), migration-stimulating factor, myostatin (GDF-8), myelomonocytic growth factor (MGF), nerve growth factor (NGF), placental growth factor (PlGF), platelet-derived growth factor (PDGF), thrombopoietin (Tpo), transforming growth factor alpha (TGF-α), TGF-β, tumor necrosis factor alpha (TNF-α), vascular endothelial growth factor (VEGF), or a Wnt protein. In a more specific embodiment of said organoids, an individual said organoid, e.g., an organoid comprising 1 x 10⁸ cells, produces at least 1.0 to 10 µM said cytokine in *in vitro* culture in growth medium over 24 hours.

In other specific embodiments, said protein or polypeptide is a soluble receptor for AM, Ang, BMP, BDNF, EGF, Epo, FGF, GNDF, G-CSF, GM-CSF, GDF-9, HGF, HDGF, IGF, migration-stimulating factor, GDF-8, MGF, NGF, PlGF, PDGF, Tpo, TGF-α, TGF-β, TNF-α, VEGF, or a Wnt protein. In a more specific embodiment of said organoids, an individual organoid, e.g., an organoid comprising 1 x 10* cells, produces at least 1.0 to 10 µM of said soluble receptor in *in vitro* culture in growth medium over 24 hours.

In other specific embodiments, said protein or polypeptide is an interleukin, e.g., interleukin-1 alpha (IL-1α), IL-1β, IL-1F1, IL-1F2, IL-1F3, IL-1F4, IL-1F5, IL-1F6, IL-1F7, IL-1F8, IL-1F9, IL-2, IL-3, IL-4, IL-5, IL-6, IL-7, IL-8, IL-9, IL-10, IL-11, IL-12 35 kDa alpha subunit, IL-12 40 kDa beta subunit, both IL-12 alpha and beta subunits, IL-13, IL-14, IL-15, IL-16, IL-17A, IL-17B, IL-17C, IL-17D, IL-17E, IL-17F isoform 1, IL-17F isoform 2, IL-18, IL-19, IL-20, IL-21, IL-22, IL-23 p19 subunit, IL-23 p40 subunit, IL-23 p19 subunit and IL-23 p40 subunit together, IL-24, IL-25, IL-26, IL-27B, IL-27-p28, IL-27B and IL-27-p28 together, IL-28A, IL-28B, IL-29, IL-30, IL-31, IL-32, IL-33, IL-34, IL-35, IL-36α, IL-36β, IL-36γ. In a more specific embodiment of said organoids, an individual said organoid, e.g., an organoid comprising 1 x 10⁸ cells, produces at least 1.0 to 10 µM of said interleukin in *in vitro* culture in growth medium over 24 hours.

In other specific embodiments, said protein or polypeptide is a soluble receptor for IL-1α, IL-1β, IL-1F1, IL-1F2, IL-1F3, IL-1F4, IL-1F5, IL-1F6, IL-1F7, IL-1F8, IL-1F9, IL-2, IL-3, IL-4, IL-5, IL-6, IL-7, IL-8, IL-9, IL-10, IL-11, IL-12 35 kDa alpha subunit, IL-12 40 kDa beta subunit, IL-13, IL-14, IL-15, IL-16, IL-17A, IL-17B, IL-17C, IL-17D, IL-17E, IL-17F isoform 1, IL-17F isoform 2, IL-18, IL-19, IL-20, IL-21, IL-22, IL-23 p19 subunit, IL-23 p40 subunit, IL-24, IL-25, IL-26, IL-27B, IL-27-p28, IL-28A, IL-28B, IL-29, IL-30, IL-31, IL-32, IL-33, IL-34, IL-35, IL-36α, IL-36β, IL-36γ. In a more specific embodiment of said organoids, an individual organoid, e.g., an organoid comprising 1 x 10⁸ cells, produces at least 1.0 to 10 µM of said soluble receptor in *in vitro* culture in growth medium over 24 hours.

In other specific embodiments, said protein or polypeptide is an interferon (IFN), *e.g.,* IFN-α, IFN-β, IFN-γ, IFN-λ1, IFN-λ2, IFN-λ3, IFN-K, IFN-ε, IFN-κ, IFN-τ, IFN-δ, IFN-ζ, IFN-co, or IFN-v. In a more specific embodiment of said organoids, an individual said organoid, e.g., an organoid comprising 1 x 10⁸ cells, produces at least 1.0 to 10 µM of said interferon in *in vitro* culture in growth medium over 24 hours.

In other specific embodiments, said protein or polypeptide is a soluble receptor for IFN-α, IFN-β, IFN-γ, IFN-λ1, IFN-λ2, IFN-λ3, IFN-K, IFN-ε, IFN-κ, IFN-τ, IFN-δ, IFN-ζ, IFN-ω, or IFN-v. In a more specific embodiment of said organoids, an individual organoid, e.g., an organoid comprising 1 x 10⁸ cells, produces at least 1.0 to 10 µM of said soluble receptor in *in vitro* culture in growth medium over 24 hours.

In other specific embodiments, said protein or polypeptide is insulin or proinsulin. In a more specific embodiment of said organoids, an individual said organoid, e.g., an organoid comprising 1 x 10⁸ cells, produces at least 1.0 to 10 µM of said insulin in *in vitro* culture in growth medium over 24 hours. In other specific embodiments, said protein is a receptor for insulin. In a more specific embodiment, said cells have additionally been genetically engineered to produce one or more of prohormone convertase 1, prohormone convertase 2, or carboxypeptidase E.

In another specific embodiment, said protein or polypeptide is leptin (LEP). In a more specific embodiment of said organoids, an individual said organoid, e.g., an organoid comprising 1 x 10⁸ cells, produces at least 1.0 to 10 µM of said leptin in *in vitro* culture in growth medium over 24 hours.

In other specific embodiments, said protein is erythropoietin (Epo). In a more specific embodiment of said organoids, an individual said organoid, e.g., an organoid comprising 1 x 10⁸ cells, produces at least 1.0 to 10 µM of said Epo in *in vitro* culture in growth medium over 24 hours.

In another specific embodiment, said protein is thrombopoietin (Tpo). In a more specific embodiment of said organoids, an individual said organoid, e.g., an organoid comprising 1 x 10⁸ cells, produces at least 1.0 to 10 µM of said Tpo in *in vitro* culture in growth medium over 24 hours.

The organoids may in certain embodiments comprise cells engineered to produce dopamine, or a precursor to dopamine. In a specific embodiment of any of the organoids provided herein, for example, said protein is tyrosine 3-monooxygenase. In a more specific embodiment of said organoids, an individual organoid, e.g., an organoid comprising 1 x 10⁸ cells, produces at least 1.0 to 10 µM of L-DOPA in *in vitro* culture in growth medium over 24 hours. In a more specific embodiment, said cells in said organoids are further engineered to express aromatic L-amino acid decarboxylase. In a more specific embodiment of said organoids, an individual said organoid, e.g., an organoid comprising 1 x 10⁸ cells, produces at least 1.0 to 10 µM of dopamine in *in vitro* culture in growth medium over 24 hours.

In another specific embodiment of said organoids, said protein or polypeptide is a hormone or prohormone. In more specific embodiments, said hormone is antimullerian hormone (AMH), adiponectin (Acrp30), adrenocorticotropic hormone (ACTH), angiotensin (AGT), angiotensinogen (AGT), antidiuretic hormone (ADH), vasopressin, atrial-natriuretic peptide (ANP), calcitonin (CT), cholecystokinin (CCK), corticotrophin-releasing hormone (CRH), erythropoietin (Epo), follicle-stimulating hormone (FSH), testosterone, estrogen, gastrin (GRP), ghrelin, glucagon (GCG), gonadotropin-releasing hormone (GnRH), growth hormone (GH), growth hormone releasing hormone (GHRH), human chorionic gonadotropin (hCG), human placental lactogen (HPL), inhibin, leutinizing hormone (LH), melanocyte stimulating hormone (MSH), orexin, oxytocin (OXT), parathyroid hormone (PTH), prolactin (PRL), relaxin (RLN), secretin (SCT), somatostatin (SRIF), thrombopoietin (Tpo), thyroid-stimulating hormone (Tsh), and/or thyrotropin-releasing hormone (TRH).

In another specific embodiment, said protein or polypeptide is cytochrome P450 side chain cleavage enzyme (P450SCC).

In other specific embodiments, said protein is a protein missing or malfunctioning in an individual who has a genetic disorder or disease. In specific embodiments, said genetic disease is familial hypercholesterolemia and said protein is low density lipoprotein receptor (LDLR); said genetic disease is polycystic kidney disease, and said protein is polycystin-1 (PKD1), PKD-2 or PKD3; or said genetic disease is phenylketonuria and said protein is phenylalanine hydroxylase.

In embodiments, in which the organoids comprise immunomodulatory cells, as described elsewhere herein, the organoids can further comprise one or more immunomodulatory compounds, e.g., compound is a non-steroidal anti-inflammatory drug (NSAID), acetaminophen, naproxen, ibuprofen, acetylsalicylic acid, a steroid, an anti-T cell receptor antibody, an anti-IL-2 receptor antibody, basiliximab, daclizumab (ZENAPAX)®), anti T cell receptor antibodies (e.g., Muromonab-CD3), azathioprine, a corticosteroid, cyclosporine, tacrolimus, mycophenolate mofetil, sirolimus, calcineurin inhibitors, and the like. In a specific embodiment, the immumosuppressive agent is a neutralizing antibody to macrophage inflammatory protein (MIP)-1α or MIP-1β.

### 4.6. Specific Examples of Organoids

Specific embodiments of gland-specific organoids are provided below in each of Sections 4.6.1 to 4.6.6, below.

### 4.6.1. Pituitary Gland

The pituitary gland comprises a body of cells, acidophils and chromophils in the anterior pituitary and neurosecretory cells in the posterior pituitary, surrounded by an anastomosing network of blood vessels. In certain embodiments, therefore, provided herein are organoids that perform at least one physiological function of a pituitary gland, e.g., provided herein are pituitary organoids. In specific embodiments, said at least one physiological function of a pituitary gland is production of, or said pituitary organoids produce, detectable amounts of one or more pituitary-specific hormones, *e.g.,* one or more of human growth hormone (hGH), prolactin (PRL), adrenocorticotropic hormone (ACTH) (also referred to as corticotrophin), melanocyte-stimulating hormone (MSH), thyroid-stimulating hormone (TSH) (also referred to as thyrotrophin), follicle-stimulating hormone (FSH), leutenizing hormone (LH), antidiuretic hormone (ADH), and/or oxytocin. In certain embodiments, said organoids comprise (e.g., additionally comprises), cells that have been genetically engineered to produce detectable amounts of one or more pituitary-specific hormones, *e.g.,* one or more of human growth hormone (hGH), prolactin (PRL), adrenocorticotropic hormone (ACTH) (also referred to as corticotrophin), melanocyte-stimulating hormone (MSH), thyroid-stimulating hormone (TSH) (also referred to as thyrotrophin), follicle-stimulating hormone (FSH), leutenizing hormone (LH), antidiuretic hormone (ADH), and/or oxytocin.

Production of said one or more pituitary-specific hormones by said organoids may be assayed, *e.g.,* by commercially-available kits and assays. For example, hGH production may be assayed *in vitro* using the Human GH ELISA kit (AbFrontier Co., Ltd.; Seoul, KR); ACTH production may be assayed *in vitro* using the ACTH (1-39) EIA Kit (Bachem, Torrance, CA); MSH production may be assayed *in vitro* by the Human / Mouse / Rat MSH EIA Kit (Raybiotech, Inc.; Norcross GA); TSH production may be assayed *in vitro* using the Human TSH ELISA Kit (Calbiotech, Inc., Spring Valley, CA); FSH production can be assayed *in vitro* using the Human FSH ELISA Kit (Anogen, Mississauga, Ontario, Canada); LH production can be assayed *in vitro* using the ELISA Kit for Leutenizing Hormone (Uscn Life Science, Wuhan, China); ADH production may be assessed *in vitro* using the CLIA Kit for Antidiuretic Hormone (ADH) (Uscn Life Science, Wuhan, China); prolactin production by said organoids can be assessed *in vitro* using the Prolactin ELISA (Immuno-Biological Laboratories America), and oxytocin production may be assessed *in vitro* using the Oxytocin OT ELISA Kit (MyBiosource, San Diego, CA). In each of the foregoing assays, in certain embodiments, culture medium in which the organoids are cultured is assayed for production of the particular hormone by said organoids.

In specific embodiments, said pituitary organoids comprise one or more of pituitary somatotrophs, pituitary mammotrophs, pituitary corticotrophs, pituitary thyrotrophs, pituitary gonadotrophs, and/or pituitary neurosecretory cells. In certain other specific embodiments, the pituitary organoids can comprise (*e.g.,* can also comprise), cells that have been genetically engineered to produce one or more pituitary-specific hormones. In certain specific embodiments, the organoids further comprise vascular endothelial cells, wherein said vascular endothelial cells are arranged within said organoids, e.g., along one or more vessels in said placental vascular scaffold. In other more specific embodiments, said organoids are constructed so that said one or more of pituitary somatotrophs, pituitary mammotrophs, pituitary corticotrophs, pituitary thyrotrophs, pituitary gonadotrophs, and/or pituitary neurosecretory cells are positioned adjacent to one or more of said vessels.

In certain other embodiments, said one or more of pituitary somatotrophs, pituitary mammotrophs, pituitary corticotrophs, pituitary thyrotrophs, pituitary gonadotrophs, and/or pituitary neurosecretory cells are positioned at or adjacent to the exterior surface of said organoids, such that cells can take up nutrients from the exterior of the organoids by diffusion, and said one or more pituitary-specific hormones can diffuse from said organoids into the surrounding environment, *e.g.,* into culture medium or into an individual into which said organoids are implanted.

### 4.6.2. Thyroid Gland

The thyroid comprises thyroid follicular cells, which secrete colloid; thyroid epithelial cells, which produce T3 and T4; and thyroid parafollicular cells, which produce calcitonin. In certain embodiments, therefore, provided herein are organoids that perform at least one physiological function of a thyroid gland, *e.g.,* provided herein are thyroid organoids. In specific embodiments, said at least one physiological function of a thyroid is, or said thyroid organoids produce, detectable amounts of one or more thyroid-specific hormones, e.g., one or more of triiodothyronine (T3), thyroxine (T4) and/or calcitonin. Production of said one or more thyroid-specific hormones by said organoids may be assayed, *e.g.,* by commercially-available kits and assays. For example, T3 production may be assayed *in vitro* using the Total T3 ELISA Kit (MyBiosource, San Diego, CA); T4 production may be assayed *in vitro* using the Total T4 ELISA Kit (MyBiosource, San Diego, CA); and calcitonin production may be assayed *in vitro* using the Calcitonin ELISA Kit (MyBiosource, San Diego, CA). In certain embodiments, said organoids comprise (*e.g.,* additionally comprise), cells that have been genetically engineered to produce detectable amounts of one or more thyroid-specific hormones, *e.g.,* one or more of T3, T4 and/or calcitonin. In each of the foregoing assays, in certain embodiments, culture medium in which the organoids are cultured is assayed for production of the particular hormone by said organoids.

In specific embodiments, said thyroid organoids comprise one or more of thyroid follicular cells, thyroid epithelial cells, and/or thyroid parafollicular cells. In certain specific embodiments, the thyroid organoids further comprise vascular endothelial cells, wherein said vascular endothelial cells are, e.g., disposed within one or more vessels in said placental vascular scaffold.

### 4.6.3. Parathyroid Gland

The parathyroid gland primarily comprises two types of cells: parathyroid chief cells, responsible for the production of parathyroid hormone, and parathyroid oxyphil cells. In certain embodiments, therefore, provided herein are organoids that performs at least one physiological function of a parathyroid gland, *e.g.,* provided herein are parathyroid organoids. In specific embodiments, said at least one physiological function of a parathyroid gland is production of, or said parathyroid organoids produce, detectable amounts of parathyroid hormone (PTH). Production of PTH can be assessed *in vitro, e.g.* by testing culture medium in which said organoids are cultured, for the presence of PTH using the Intact-PTH ELISA Kit (Immuno-Biological Laboratories, Minneapolis, MN). In certain embodiments, the parathyroid organoids comprise parathyroid chief cells. In more specific embodiments, the parathyroid organoids comprise both parathyroid chief cells and parathyroid oxyphil cells. In certain embodiments, said organoids comprise (*e.g.,* additionally comprises), cells that have been genetically engineered to produce detectable amounts of PTH. In each of the foregoing assays, in certain embodiments, culture medium in which the organoids are cultured is assayed for production of the particular hormone or protein by said organoids.

In certain specific embodiments, the parathyroid organoids further comprise vascular endothelial cells, wherein said vascular endothelial cells are disposed within one or more vessels in said placental vascular scaffold. In other more specific embodiments, said organoids are constructed so that said parathyroid chief cells and/or said parathyroid oxyphil cells are positioned adjacent to one or more of said vessels.

### 4.6.4. Adrenal Gland

The adrenal gland comprises adrenal chromaffin cells, which are primarily responsible for production of epinephrine; adrenal zona glomerulosa cells, which produce mineralocorticoids (primarily aldosterone); adrenal zona fasciculata cells, which produce glucocorticoids (*e.g.,* 11-deoxycorticosterone, corticosterone, and/or cortisol); and adrenal zona reticularis cells, which produce androgens (*e.g.,* dehydroepiandrosterone (DHEA) and/or androstenedione). In certain embodiments, therefore, provided herein are organoids that perform at least one physiological function of an adrenal gland, *e.g.,* provided herein are adrenal organoids. In specific embodiments, said at least one physiological function of an adrenal gland is production of, or said adrenal organoids produce, detectable amounts of one or more adrenal-specific hormones, *e.g.,* one or more of aldosterone, fludrocortisone, dehydroepiandrosterone, 18 hydroxy 11 deoxycorticosterone, corticosterone, cortisol, DHEA and/or androstenedione. In certain embodiments, said organoids comprise (*e.g.,* additionally comprise), cells that have been genetically engineered to produce detectable amounts of one or more of, *e.g.,* aldosterone, 11-deoxycorticosterone, corticosterone, cortisol, fludrocortisone, DHEA and/or androstenedione.

Production of said one or more adrenal gland-specific hormones may be assayed, *e.g.,* by published and/or commercially-available kits and assays. For example, production of fludrocortisone by said adrenal organoids can be assessed using a liquid chromatography assay; *see* Ast et al., J. Pharm. Sci. 68(4):421-423 (1979). Production of aldosterone by the adrenal organoids can be assayed using the Human Aldosterone ELISA Kit (BioVendor Laboratory Medicine, Inc., Candler, NC). Production of cortisol by the adrenal organoids can be assayed by the Cortisol ELISA Kit (Enzo Life Sciences, Inc., Farmingdale, NY). Production of 18 hydroxy 11 deoxycorticosterone by said adrenal organoids can be assayed using a radioimmune assay; see Chandler et al., Steroids 27(2):235-246 (1976). Production of epinephrine by said adrenal organoids may be assayed by the Epinephrine RIA (Alpco Diagnostics, Salem, NH). Androstenedione production by said adrenal organoids can be assayed by mass spectrometry; see Booker et al., Drug Testing and Analysis 1(11-12):587-595 (2009). DHEA production by the adrenal organoids may be assayed by the DHEA ELISA kit (Abnova Corporation, Taipei City, Taiwan). In each of the foregoing assays, in certain embodiments, culture medium in which the organoids are cultured is assayed for production of the particular hormone or protein by said organoids.

In certain specific embodiments, the adrenal organoids comprise adrenal chromaffin cells, adrenal zona fasciculata cells, adrenal zona glomerulosa cells, and/or adrenal zona reticularis cells. In a specific embodiment, said adrenal organoids comprise two or more of adrenal zona fasciculata cells, adrenal zona glomerulosa cells, and/or adrenal zona reticularis cells. In certain specific embodiments, said adrenal chromaffin cells, adrenal zona fasciculata cells, adrenal zona glomerulosa cells, and/or adrenal zona reticularis cells are arranged randomly, or are regularly ordered, within said adrenal organoids. In certain other specific embodiments, said adrenal chromaffin cells are grouped together within said organoids, said adrenal zona fasciculata cells are grouped together within said organoids, said adrenal zona glomerulosa cells are grouped together within said organoids, and/or adrenal zona reticularis cells are grouped together within said adrenal organoids. In another specific embodiment, said adrenal organoids comprises zona glomerulosa cells and zona fasciculata cells, wherein said zona glomerulosa cells and zona fasciculata cells are separate from each other in said adrenal organoids. In another specific embodiment, said adrenal organoids comprise zona glomerulosa cells and zona reticularis cells, wherein said zona glomerulosa cells and zona reticularis cells are separate from each other in said adrenal organoids. In another said adrenal organoids comprise zona reticularis cells and zona fasciculata cells, wherein said zona reticularis cells and zona fasciculata cells are separate from each other in said adrenal organoids. In another specific embodiment, the adrenal organoids comprise zona glomerulosa cells, zona fasciculata cells, and zona reticularis cells, wherein each of said zona glomerulosa cells, zona fasciculata cells, and zona reticularis cells are each separate from the other cell types in said adrenal organoids.

In certain specific embodiments, the adrenal organoids further comprise vascular endothelial cells, wherein said vascular endothelial cells are disposed along one or more vessels in said placental vascular scaffold.

### 4.6.5. Pancreas

The pancreas comprises pancreatic alpha cells, pancreatic beta cells, pancreatic delta cells, pancreatic PP cells, and pancreatic epsilon cells. In certain embodiments, therefore, provided herein are organoids that perform at least one physiological function of a pancreas, *e.g.,* provided herein are pancreatic organoids. In specific embodiments, said at least one physiological function of a pancreas is production of, or said pancreatic organoids produce, detectable amounts of a pancreas-specific hormone or protein, *e.g.,* amylin (also known as islet amyloid polypeptide, or IAPP), insulin, somatostatin, grehlin, pancreatic polypeptide, and/or glucagon, *e.g., in vitro.* In a more specific embodiment, said organoids produce insulin and amylin, *in vitro,* in a ratio of about 10:1, 60:1, 70:1, 80:1, 90:1, 100:1, 110:1, 120:1, 130:1, 140:1, 150:1, 160:1, 170:1, 180:1, 190:1 or 200:1. In certain embodiments, said organoids comprise (*e.g.,* additionally comprise), cells that have been genetically engineered to produce detectable amounts of one or more of amylin, insulin, glucagon, somatostatin, grehlin, an/or pancreatic polypeptide.

Production of said one or more pancreas-specific hormones by said pancreatic organoids can be assayed using commercially-available assays or kits. For example, production of insulin by said pancreatic organoids *in vitro* may be assayed by any commonplace insulin test kits; production of glucagon by said pancreatic organoids *in vitro* may be assayed by the ELISA Kit for Glucagon (Uscn Life Science, Inc., Wuhan, China); production of somatostatin by the pancreatic organoids *in vitro* may be assayed by the Human Somatostatin (SST) ELISA (Kamiya Biomedical Company, Seattle, WA); production of grehlin by the pancreatic organoids *in vitro* may be assayed by the Grehlin (Human, Mouse, Rat) ELISA Kit (Abnova, Taipei City, Taiwan); production of pancreatic polypeptide by the pancreatic organoids *in vitro* may be assayed by the Human Pancreatic Polypeptide (PP) ELISA Kit (EMD Millipore, Billerica, ME); and production of amylin by said pancreatic organoids may be assayed by the IAPP (Human) ELISA Kit (Abnova, Taipei City, Taiwan). In each of the foregoing assays, in certain embodiments, culture medium in which the organoids are cultured is assayed for production of the particular hormone or protein by said organoids.

In certain specific embodiments, the adrenal organoids further comprise vascular endothelial cells, wherein said vascular endothelial cells are, e.g., disposed along one or more vessels in said placental vascular scaffold.

### 4.6.6. Liver

The liver comprises primarily parenchymal hepatocytes, which make up 70%-80% of the liver's mass, along with vascular endothelial cells and Kupffer cells. In certain embodiments, therefore, provided herein are organoids that perform at least one physiological function of a liver, e.g., provided herein are liver organoids.

In certain specific embodiments, said organoids produce a measurable amount of one or more of coagulation factor I (fibrinogen); coagulation factor II (prothrombin); coagulation factor V (factor five); coagulation factor VII (proconvertin); coagulation factor IX (Christmas factor); coagulation factor X (Stuart-Prower factor; prothrombinase); coagulation factor XI (plasma thromboplastin antecedent); protein C (autoprothrombin IIA; blood coagulation factor XIV), protein S and/or antithrombin. In various other embodiments of any of the organoids provided herein, said organoids produce detectable amounts of glucose from an amino acid, lactate, glycerol or glycogen. In other embodiments, said organoids produce detectable amounts of insulin-like growth factor (IGF-1) or thrombopoietin. In other embodiments, said organoids produce bile. In certain embodiments of any of the organoids provided herein, said organoids comprise cells that produce one or more of coagulation factor I (fibrinogen); coagulation factor II (prothrombin); coagulation factor V (factor five); coagulation factor VII (proconvertin); coagulation factor IX (Christmas factor); coagulation factor X (Stuart-Prower factor; prothrombinase); coagulation factor XI (plasma thromboplastin antecedent); protein C (autoprothrombin IIA; blood coagulation factor XIV), protein S, antithrombin, IGF-1 or thrombopoietin. In certain embodiments of any of the organoids provided herein, said organoids comprise hepatic vessel endothelial cells, e.g., disposed along vessels in said placental vascular scaffold. In a more specific embodiment, said hepatocytes are disposed along and substantially parallel to said vessels.

Production of said one or more pancreas-specific hormones by said liver organoids can be assayed using published commercially-available assays or kits. For example, production of fibrinogen by said liver organoids can be assayed by the Human Fibrinogen ELISA Kit (AbFrontier Co., Ltd., Seoul, KR); production of prothrombin by said liver organoids may be assayed by the Prothrombin (Human) ELISA kit (Abnova, Taipei City, Taiwan); production of factor five by said liver-specific organoids may be assayed by the Zymutest Factor V ELISA (Aniara, Mason, OH); production of proconvertin by said liver organoids can be assayed by the Factor VII (Proconvertin) Activity assay (Gentaur Molecular Products, Whetstone, London, UK); production of coagulation factor XI by said liver organoids can be assayed by the Total Human Coagulation Factor XI Antigen Assay (Molecular Innovations, Novi, MI); production of prothrombinase by said liver organoids can be assayed by the ELISA Kit for Coagulation Factor X (Uscn Life Science, Wuhan, China); production of coagulation factor XI by said liver organoids may be assayed by the Factor XI Human ELISA Kit (ab 108834) (Abcam, Cambridge, MA); production of protein C by said liver organoids may be assayed by the Chromogenic Assay Kit for Plasma Protein C (American Diagnostica, Pfungstadt, Germany); production of protein S by said liver organoids may be assayed by the Human Free Protein S DLISA Kit (American Diagnostica, Pfungstadt, Germany); production of antithrombin by said liver organoids may be assayed by the ACTICHROME® Antithrombin III Chromogenic Activity Kit (American Diagnostica, Pfungstadt, Germany); production of IGF-1 by said liver organoids may be assayed by the Human IGF-1 ELISA Kit (AbFrontier, Co., Ltd., Seoul, KR); and production of thrombopoietin by said liver organoids may be assessed using the Human TPO / Thrombopoietin ELISA Kit (Cell Sciences, Canton, MA). In each of the foregoing assays, in certain embodiments, culture medium in which the organoids are cultured is assayed for production of the particular hormone or protein by said organoids.

### 4.7. METHODS OF USING ORGANOIDS

The organoids provided herein can be used in methods of treating an individual having a particular disease or disorder treatable by replacement of, or augmentation of, a physiological function, *e.g.,* production of a biomolecule, e.g., protein or polypeptide, hormone, cytokine, interleukin, interferon, receptors for any of the foregoing, or the like, e.g., by administration of organoids that produce such biomolecule, e.g., and which, when administered, replaces or augments the naturally-occurring biomolecule in the individual. Any of the organoids provided elsewhere herein can be used for therapeutic purposes, as judged by one of ordinary skill in the art to be appropriate.

In other embodiments, the biomolecule produced by the organoid can be isolated, e.g., from culture medium or buffer in which the organoid is cultured or maintained. In other embodiments, the organoid, or a plurality of organoids, each producing at least one biomolecule, are contained within a container external to a person in need of said at least one biomolecule, wherein the biomolecule is made available to said individual, e.g., by a physical connection between the individual and the container, e.g., by tubing conducting the culture medium or buffer in which the organoid is maintained to the individual.

Pituitary organoids, as described above, wherein the organoids produce one or more pituitary hormones in an individual to whom they are administered, may be therapeutic where the individual is experiencing a disorder due to lack, or reduced production, of a pituitary hormone. Such disorders may, in various embodiments, relate to abnormally reduced growth, disorders of blood pressure, breast milk production, sex organ function, thyroid gland function, water regulation, and/or temperature regulation.

In one embodiment, provided herein is method of treating an individual in need of human growth hormone (hGH) comprising administering to said individual an organoid that produces hGH, or an organoid comprising cells that produce hGH, or hGH produced by such organoids, e.g., a therapeutically effective amount of hGH, e.g., the organoid described in section 4.6.1, above. Production of hGH in said individual can be assessed, e.g., using the Human GH ELISA kit (AbFrontier Co., Ltd.; Seoul, KR) with a sample of the individual's serum post-administration.

In another embodiment, provided herein is method of treating an individual in need of prolactin (PRL) comprising administering to said individual an organoid that produces PRL, or an organoid comprising cells that produce PRL, or PRL produced by such organoids, e.g., a therapeutically effective amount of PRL, e.g., the organoid described in Section 4.6.1, above. Production of PRL in said individual can be assessed, e.g., using the Prolactin ELISA (Immuno-Biological Laboratories America) with a sample of the individual's serum post-administration. In specific embodiments, said individual has one or more of metabolic syndrome, arteriogenic erectile dysfunction, premature ejaculation, oligozoospermia, asthenospermia, hypofunction of seminal vesicles, or hypoandrogenism.

In another embodiment, provided herein is a method of treating an individual in need of adrenocorticotropic hormone (ACTH) comprising administering to said individual an organoid that produces ACTH, or an organoid comprising cells that produce ACTH, or ACTH produced by such organoids, e.g., a therapeutically effective amount of ACTH, e.g., the organoid described in Section 4.6.1, above. Production of ACTH in said individual can be assessed, e.g., using the ACTH (1-39) EIA Kit (Bachem, Torrance, CA) with a sample of the individual's serum post-administration. In a specific embodiment, said individual has Addison's disease.

In another embodiment, provided herein is a method of treating an individual in need of melanocyte-stimulating hormone (MSH), comprising administering to said individual an organoid that produces MSH, or an organoid comprising cells that produce MSH, or MSH produced by such organoids, e.g., a therapeutically effective amount of MSH, e.g., the organoid described in section 4.6.1, above. Production of MSH in said individual can be assessed, e.g., using the Human / Mouse / Rat MSH EIA Kit (Raybiotech, Inc.; Norcross GA) with a sample of the individual's serum post-administration. In a specific embodiment, said individual has Alzheimer's disease.

In another embodiment, provided herein is a method of treating an individual in need of thyroid-stimulating hormone (TSH), comprising administering to said individual an organoid that produces TSH, or an organoid comprising cells that produce TSH, or TSH produced by such organoids, e.g., a therapeutically effective amount of TSH, e.g., the organoid described in Section 4.6.1, above. Production of TSH in said individual can be assessed, e.g., using the Human TSH ELISA Kit (Calbiotech, Inc., Spring Valley, CA) with a sample of the individual's serum post-administration. In a specific embodiment, said individual has or manifests cretinism.

In another embodiment, provided herein is a method of treating an individual in need of follicle-stimulating hormone (FSH) comprising administering to said individual an organoid that produces FSH, or an organoid comprising cells that produce FSH, or FSH produced by such organoids, e.g., a therapeutically effective amount of FSH, e.g., the organoid described in Section 4.6.1, above. Production of FSH in said individual can be assessed, e.g., using the Human FSH ELISA Kit (Anogen, Mississauga, Ontario, Canada) with a sample of the individual's serum post-administration. In a specific embodiment, said individual has or manifests infertility or azoospermia.

In another embodiment, provided herein is a method of treating an individual in need of leutenizing hormone (LH) comprising administering to said individual an organoid that produces LH, or an organoid comprising cells that produce LH, or LH produced by such organoids, e.g., a therapeutically effective amount of LH, e.g., the organoid described in Section 4.6.1, above. Production of LH in said individual can be assessed, e.g., using the ELISA Kit for Leutenizing Hormone (Uscn Life Science, Wuhan, China) with a sample of the individual's serum post-administration. In a specific embodiment, said individual has or manifests low testosterone, low sperm count or infertility.

In another embodiment, provided herein is a method of treating an individual in need of antidiuretic hormone (ADH) comprising administering to said individual an organoid that produces ADH, or an organoid comprising cells that produce ADH, or ADH produced by such organoids, e.g., a therapeutically effective amount of ADH, e.g., the organoids described in Section 4.6.1, above. Production of ADH in said individual can be assessed using the CLIA Kit for Antidiuretic Hormone (ADH) (Uscn Life Science, Wuhan, China) with a sample of the individual's serum post-administration. In a specific embodiment, said individual has hypothalamic diabetes insipidus.

In another embodiment, provided herein is a method of treating an individual in need of oxytocin, comprising administering to said individual an organoid that produces oxytocin, or an organoid comprising cells that produce oxytocin, or oxytocin produced by such organoids, e.g., a therapeutically effective amount of oxytocin, e.g., the organoid described in Section 4.6.1, above. Production of oxytocin in said individual can be assessed, e.g., using the Oxytocin OT ELISA Kit (MyBiosource, San Diego, CA) with a sample of the individual's serum post-administration.

Thyroid organoids, as described above, wherein the organoids produce one or more thyroid hormones in an individual to whom they are administered, may be therapeutic where the individual is experiencing a disorder due to lack, or reduced production, of a thyroid hormone. Such disorders may, in various embodiments, relate to reduced metabolism, hypothyroidism, Graves disease, Hashimoto's thyroiditis, and the like.

In another embodiment, provided herein is a method of treating an individual in need of thyroxine (T4) comprising administering to said individual an organoid that produces T4, or an organoid comprising cells that produce T4, or T4 produced by such organoids, e.g., a therapeutically effective amount of T4, e.g., the organoid described in Section 4.6.2, above. T4 production in said individual may be assessed, e.g., using the Total T4 ELISA Kit (MyBiosource, San Diego, CA) with a sample of the individual's serum post-administration. In specific embodiments, said individual has or manifests mental retardation, dwarfism, weakness, lethargy, cold intolerance, or moon face associated with T4 deficiency.

In another embodiment, provided herein is a method of treating an individual in need of triiodothyronine (T3) comprising administering to said individual an organoid that produces T3, or an organoid comprising cells that produce T3, or T3 produced by such organoids, e.g., a therapeutically effective amount of T3, e.g., the organoid described in Section 4.6.2, above. Production of T3 in said individual can be assessed, e.g., using the Total T3 ELISA Kit (MyBiosource, San Diego, CA) with a sample of the individual's serum post-administration. In a specific embodiment, said individual has heart disease. In a more specific embodiment, said individual has a serum concentration of T3 that is less than 3.1 pmol/L.

In another embodiment, provided herein is a method of treating an individual in need of calcitonin comprising administering to said individual an organoid that produces calcitonin, or an organoid comprising cells that produce calcitonin, or calcitonin produced by such organoids, e.g., a therapeutically effective amount of calcitonin, e.g., the organoid described in Section 4.6.2, above. Production of calcitonin in said individual may be assessed, e.g., using the Calcitonin ELISA Kit (MyBiosource, San Diego, CA) with a sample of the individual's serum post-administration. In specific embodiments, said individual has osteoporosis or chronic autoimmune hypothyroidism.

In another embodiment, provided herein is a method of treating an individual in need of parathyroid hormone (PTH) comprising administering to said individual an organoid that produces PTH, or an organoid comprising cells that produce PTH, or PTH produced by such organoids, e.g., a therapeutically effective amount of PTH, e.g., the organoid described in Section 4.6.3, above. Production of PTH in said individual may be assessed, e.g., using the Intact-PTH ELISA Kit (Immuno-Biological Laboratories, Minneapolis, MN) with a sample of the individual's serum post-administration.

Adrenal organoids, as described above, wherein the organoids produce one or more adrenal gland hormones in an individual to whom they are administered, may be therapeutic where the individual is experiencing a disorder due to lack, or reduced production, of an adrenal hormone. Such disorders may, in various embodiments, relate to metabolic activity, fat or carbohydrate utilization, inflammation, Cushing syndrome, and/or dysregulation of salt and water balance.

In another embodiment, provided herein is a method of treating an individual in need of aldosterone comprising administering to said individual an organoid that produces aldosterone, or an organoid comprising cells that produce aldosterone, or aldosterone produced by such organoids, e.g., a therapeutically effective amount of aldosterone, e.g., the organoids described in Section 4.6.4, above. Production of aldosterone in said individual may be assessed, e.g., using the Human Aldosterone ELISA Kit (BioVendor Laboratory Medicine, Inc., Candler, NC) with a sample of the individual's serum post-administration. In specific embodiments, said individual has idiopathic hypoaldosteronism, hypereninemic hypoaldosteronism, or hyporeninemic hypoaldosteronism. In another embodiment, said individual has chronic renal insufficiency.

In another embodiment, provided herein is a method of treating an individual in need of 18 hydroxy 11 deoxycorticosterone comprising administering to said individual an organoid that produces 18 hydroxy 11 deoxycorticosterone, or an organoid comprising cells that produce 18 hydroxy 11 deoxycorticosterone, or 18 hydroxy 11 deoxycorticosterone produced by such organoids, e.g., a therapeutically effective amount of 18 hydroxy 11 deoxycorticosterone, e.g., the organoid described in Section 4.4.4, above. Production of 18 hydroxy 11 deoxycorticosterone in said individual may be assessed, e.g., using a radioimmune assay, see Chandler et al., Steroids 27(2):235-246 (1976) with a sample of the individual's serum post-administration.

In another embodiment, provided herein is a method of treating an individual in need of fludrocortisone comprising administering to said individual an organoid that produces fludrocortisone, or an organoid comprising cells that produce fludrocortisone, or fludrocortisone produced by such organoids, e.g., a therapeutically effective amount of fludrocortisone, e.g., the organoids described in Section 4.6.4, above. Production of fludrocortisone in said individual may be assessed, e.g., using a liquid chromatography assay, *see* Ast et al., J. Pharm. Sci. 68(4):421-423 (1979), with a sample of the individual's serum post-administration.

In another embodiment, provided herein is a method of treating an individual in need of cortisol comprising administering to said individual an organoid that produces cortisol, or an organoid comprising cells that produce cortisol, or cortisol produced by such organoids, e.g., a therapeutically effective amount of cortisol, e.g., the organoid described in Section 4.6.4, above. Production of cortisol in said individual may be assessed, e.g., using the Cortisol ELISA Kit (Enzo Life Sciences, Inc., Farmingdale, NY) with a sample if the individual's serum. In specific embodiments, said individual has acute adrenal deficiency, Addison's disease, or hypoglycemia.

In another embodiment, provided herein is a method of treating an individual in need of epinephrine, comprising administering to said individual an organoid that produces epinephrine, or an organoid comprising cells that produce epinephrine, or epinephrine produced by such organoids, e.g., a therapeutically effective amount of epinephrine, e.g., the organoid described in Section 4.6.4, above. Production of epinephrine in said individual can be assessed, e.g., using the Epinephrine RIA (Alpco Diagnostics, Salem, NH) with a sample of the individual's serum post-administration.

In another embodiment, provided herein is a method of treating an individual in need of androstenedione comprising administering to said individual an organoid that produces androstenedione, or an organoid comprising cells that produce androstenedione, or androstenedione produced by such organoids, e.g., a therapeutically effective amount of androstenedione, e.g., the organoid described in Section 4.6.4, above. Production of androstenedione in the individual can be assessed, e.g., using mass spectrometry, see Booker et al., Drug Testing and Analysis 1(11-12):587-595 (2009), with a sample of the individual's serum post-administration.

In another embodiment, provided herein is a method of treating an individual in need of dehydroepiandrosterone (DHEA) comprising administering to said individual an organoid that produces DHEA, or an organoid comprising cells that produce DHEA, or DHEA produced by such organoids, e.g., a therapeutically effective amount of DHEA, e.g., the organoid described in Section 4.6.4, above. Production of DHEA in said individual may be assessed, e.g., using the DHEA ELISA kit (Abnova Corporation, Taipei City, Taiwan) with a sample of the individual's serum post-administration.

Further provided herein is a method of treating an individual in need of a compound, comprising administering an organoid that produces said compound, or an organoid comprising cells that produce said compound, or said compound produced by such organoids, e.g., a therapeutically effective amount of said compound, e.g., the organoid described in Section 4.6.6 above, wherein said compound is coagulation factor I (fibrinogen); coagulation factor II (prothrombin); coagulation factor V (factor five); coagulation factor VII (proconvertin); coagulation factor IX (Christmas factor); coagulation factor X (Stuart-Prower factor; prothrombinase); coagulation factor XI (plasma thromboplastin antecedent); protein C (autoprothrombin IIA; blood coagulation factor XIV), protein S and/or antithrombin. The presence of these compounds in said individual may be assessed using art-known assays with a sample of the individual's serum post-administration

In another embodiment, provided herein is a method of treating an individual in need of IGF-1, comprising administering to said individual an organoid that produces IGF-1, or an organoid comprising cells that produce IGF-1, or IGF-1 produced by such organoids, e.g., a therapeutically effective amount of IGF-1, e.g., the organoid described in Section 4.6.6, above. Production of IGF-1 in said individual may be assessed, e.g., using the Human IGF-1 ELISA Kit (AbFrontier, Co., Ltd., Seoul, KR) with a sample of serum from said individual.

In another embodiment, provided herein is a method of treating an individual in need of thrombopoietin (Tpo), comprising administering to said individual an organoid that produces Tpo, or an organoid comprising cells that produce Tpo, or Tpo produced by such organoids, e.g., a therapeutically effective amount of Tpo, e.g., the organoid described in Section 4.6.6, above. Production of Tpo in said individual may be assessed, e.g., using the Human TPO / Thrombopoietin ELISA Kit (Cell Sciences, Canton, MA) with a sample of serum from said individual.

In another embodiment, provided herein is a method of treating an individual in need of glucagon, comprising administering to said individual an organoid that produces glucagon, or an organoid comprising cells that produce glucagon, or glucagon produced by such organoids, e.g., a therapeutically effective amount of glucagon, e.g., the organoid described in Section 4.6.5, above. Production of glucagon in said individual may be assessed using art-known assays with a sample of serum from said individual.

In another embodiment, provided herein is a method of treating an individual in need of insulin, comprising administering to said individual an organoid that produces insulin, or an organoid comprising cells that produce insulin, or insulin produced by such organoids, e.g., a therapeutically effective amount of insulin, e.g., the organoids described in Section 4.6.5, above. Production of insulin in said individual may be assessed using art-known blood sugar tests with a sample of blood from said individual. In a specific embodiment, said individual has diabetes mellitus.

In another embodiment, provided herein is a method of treating an individual in need of amylin, comprising administering to said individual an organoid that produces amylin, or an organoid comprising cells that produce amylin, or amylin produced by such organoids, e.g., a therapeutically effective amount of amylin, e.g., the organoid described in Section 4.6.5, above. Production of amylin in said individual may be assessed, e.g., using the IAPP (Human) ELISA Kit (Abnova, Taipei City, Taiwan) with a sample of serum from said individual.

In another embodiment, provided herein is a method of treating an individual in need of grehlin, comprising administering to said individual an organoid that produces grehlin, or an organoid comprising cells that produce grehlin, or grehlin produced by such organoids, e.g., a therapeutically effective amount of grehlin, e.g., the organoid described in Section 4.6.5, above. Production of grehlin in said individual may be assessed, e.g., using the Grehlin (Human, Mouse, Rat) ELISA Kit (Abnova, Taipei City, Taiwan) with a sample of serum from said individual.

In another embodiment, provided herein is a method of treating an individual in need of pancreatic polypeptide (PP), comprising administering to said individual an organoid that produces PP, or an organoid comprising cells that produce PP, or PP produced by such organoids, e.g., a therapeutically effective amount of PP, e.g., the organoids described in Section 4.6.5, above. Production of pancreatic polypeptide in said individual may be assessed, e.g., using the Human Pancreatic Polypeptide (PP) ELISA Kit (EMD Millipore, Billerica, ME) with a sample of serum from said individual.

In certain embodiments, the decellularized placenta can be used to culture one or more of any of the cell types disclosed herein. The one or more types of cells can be, e.g., seeded onto the placental matrix, injected into the placental matrix, and/or passaged through the substantially intact placental vascular scaffolding for a time sufficient for at least a plurality of the cells to become attached to the placental matrix. Culture of the cells can proceed, e.g., in cell culture medium, e.g., under standard cell culture conditions, such as a temperature of about 37°C under air+5%CO₂.

### 5. EXAMPLES

### 5.1. Example 1: Conductivity of Decellularized Placental Vasculature

This Example demonstrates a method of efficiently and gently decellularizing placenta in such a manner as to preserve the vascular matrix of the placenta substantially intact, and the successful repopulation of the vascular matrix with non-placental cells.

### Materials and Methods

Placentas: All placentas used were pre-perfused to remove placental and umbilical cord blood. The perfusion tubing in the two umbilical cord arteries were kept and used for perfusion decellularization. Placentas were either used for perfusion decellularization immediately or frozen in a -80°C freezer in a sealed plastic package.

Perfusion Decellularization: Decellularization solutions comprising phosphate-buffered saline (PBS) and 1% Triton X-100, 0.5% SDS, and PBS, respectively, were sequentially infused into the placenta via the arteries of the umbilical cord. Residual detergent following decellularization was rinsed off using a PBS solution. Progress of decellularization was monitored by visual inspection for morphology changes of the placenta, by analysis of DNA content, and by H&E staining of the decellularized tissues.

Perfusion decellularization was set up using a peristaltic pump (VWR) with controlled flow rate between 8 to 16 mL/min, with a second, linked peristaltic pump to drain the flow-through of solution into a waste bin. Each step of perfusion utilized approximately 10 to 20 L of medium over the course of between 8 and 24 hrs. After completing the last PBS perfusion, the decellularized placental vascular scaffold was preserved in PBS with antibiotics (1% penicillin + streptomycin) at 4°C in, e.g., a stainless pan or desiccator (VWR). In a modification of the protocol, placentas were frozen at -80°C for more than 24 hrs and thawed at room temperature for 24 hours before decellularization as above.

DNA Content Analysis: The DNA content in the placental tissues was assessed by extracting and measuring DNA amount of the tissues during the processing (expressed as µg DNA/mg wet tissue weight) using a Tissue DNA isolation kit (OMEGA Bio-Tek, Cat#D3396-01). For each processing step, 4 to 6 different individual samples were used to extract DNA.

Perfusion Solutions: Stock solutions of 10% Triton X-100, 20% SDS, and 10X PBS were purchased from AMRESCO, VWR, or Sigma and diluted with distilled water to desired concentrations.

Fluid conductivity: A surface vessel fluid conductivity (SVFC) assay was established to access fluid conductivity of decellularized placental vascular scaffold. Briefly, a 0.4% Trypan Blue (100 mL to 200 mL) solution was infused into the two arteries of the umbilical cord. Distance of the dye on the location of the placental disc and the radius of the placental disc were measured. The conductivity is determined by the distance of dye travelled (D) and the radius of the placental disc (R) at the same position and calculated as the following formula (D/R) x 100%. For each placenta, 8 different data points were collected and the average was taken.

Cell conductivity: The cell conductivity of the decellularized placenta vascular scaffold was investigated by the distribution of luciferase-expressing cells after infusion of luciferase-labeled cells. The distribution of cells was imaged using a Xenogen IVIS Spectrum, and digital bioluminescent data was analyzed with Living Image 3.0 software.

### Results

### Perfusion Decellularization: Method Development

Upon perfusion decellularization, as outlined above, the decellularized vascular tree showed a translucent or transparent appearance, indicating substantially complete decellularization. This translucent or transparent appearance was reproducible across several different placentas, and was not altered by freezing the placenta prior to decellularization.

The decellularization method was simplified by using two steps of detergent-perfusion (1% Triton X-100 followed by 0.5% SDS) instead of multiple steps and multiple detergents as described above. Upon morphology inspection, decellularized human placenta appeared as a white and opaque tissue from top to the bottom of the placenta disc. DNA content analysis confirmed that the simplified two-step method can be used to efficiently and sufficiently achieve significant DNA reduction and decellularization.

### Characterization of Decellularized Placental Vascular Scaffold: DNA content analysis

DNA content of the tissues was used to examine the extent of decellularization of five experimental placentas. It was shown that first Triton X-100 perfusion significantly increased the DNA content in the tissue as compared to placental tissue not treated with Triton X-100 (P=0.02), possibly because Triton X-100 improved recovery of DNA from tissues. Subsequent 0.5% SDS perfusion reduced the average total DNA content by 69% (N=5, ranges from 81% to 50%) significantly different as comparing with the Triton X-100 treatment step (p=0.01). The second cycle of Triton X-100 and SDS perfusion appeared to increase the DNA content, likely by further releasing more DNA from the tissues. The final wash of PBS also reduced the amount of DNA in the placental tissue. DAPI and H&E staining confirmed that, after two rounds of perfusion decellularization, few intact nuclei remained in the decellularized placenta matrix. Residual DNA was most likely genomic DNA released during decellularization, which is removable using, e.g., DNAseI treatment. It is worth noting that the residual DNA is present in the decellularized matrix, and not in the vascular system, since isolated vessels from the decellularized placenta matrix have little DNA content.

### Characterization of Decellularized Placental Vascular Scaffold: Fluid Conductivity

To demonstrate the intactness of the placental vascular system after decellularization, Trypan Blue dye was infused into the vascular system after decellularization. Trypan blue dye was distributed from the center of the vein to the edge of the placenta disc, indicating that both the major and small vascular system retained conductivity post-decellularization. To quantitatively characterize the fluid conductivity, a method called "surface vessel fluid conductivity" (SVFC) was established as described under "Methods" above. SVFC of each placenta after Trypan blue dye infusion was measured at eight positions around the placenta, radially dividing the placenta into roughly equal portions. The average surface vessel fluid conductivity of three placentas was determined to be 93%.

### Characterization of Decellularized Placental Vascular Scaffold: Cell Conductivity and Distribution

To investigate the cell conductivity of the decellularized human placental vascular scaffold, luciferase-labeled cells were perfused into the decellularized placental vasculature, and the distribution of the cells within the decellularized matrix was determined by luminescence imaging and digital analysis. Four individual experiments were performed (Study 1 to Study 4).

Study 1 was a feasibility study directed to method establishment using 300 million 4T1-luc mouse breast carcinoma cells as the infused cell population. Placentas were frozen at -80°C overnight, and thawed. The placentas were decellularized using 0.1X PBS. Before cell infusion, the placenta were pre-conditioned by perfusion of 500 mL of 5% FCS-PBS. Cells resuspended in 300 mL cell culture medium were infused first, followed by an infusion of luciferin at 1.2 mg/mL. Images were taken at three different settings and at 0 hr and 2 hr after cell infusion. Quantitative image analysis was performed (circular zone and pie zone) for cell distribution. The results showed that Luciferase-labeled cells infused into placental scaffold could be imaged and visualized in this novel study method. Cells were found to be distributed in both major and small vessels throughout most of the placental vasculature.

Study 2 confirmed the results of study 1 by using detergent-decellularization derived human placental vascular scaffold. In particular, the effects of any residual detergent on Luciferase activity and cell distribution signals was evaluated using 300 million 4T1-luc mouse breast carcinoma cells as the infused cell population. Decellularization was performed by freezing and thawing, as described above, followed by decellularization with two rounds of sequential decellularization using 1% Triton X-100 and 0.5% SDS, followed by a PBS wash. Placenta matrix was also pre-conditioned with 5% FCS in PBS before cell infusion. In this study, the cells and Luciferin were premixed together and infused. Confirming the results of Study 1, the luciferase activity of cells remained at 2 hrs after infusion, indicating that detergent-based decellularization of placental scaffold has no toxicity for cells.

Study 3 was designed to demonstrate the distribution of human cells in the decellularized placental vascular scaffold. In contrast to Study 1 and Study 2, 300 million human breast carcinoma MDA-231-Luc cells, resuspended in 300 mL of growth medium, were infused into a decellularized placenta, prepared as in Study 1 and Study 2. The human cells distributed throughout the placental vasculature as efficiently as did the mouse cells.

Study 4 was designed to demonstrate decellularized human placental vascular scaffold can be used to culture cells for tissue engineering by cell repopulation. A proto-type bioreactor system was set up by culturing intact placental vasculature matrix in a sterile stainless pan (9x2 inches). Circulation through the matrix was established by insertion of input tubing into the two cord artery matrices, and insertion of output tubing into the placental vein matrix to collect flow-through medium/cells. The placenta was cultured in a 37°C incubator during perfusion of the cells. Circulation was maintained by a peristaltic pump with controlled flow rate of about 6 to 12 mL/min. 200 million human breast carcinoma MDA-231-Luc cells were resuspended in 500 mL of growth medium, and were continuously infused/reinfused into decellularized human placental vasculature using the system described as above. Circulation was maintained overnight, after which culture was discontinued and the placental vasculature was infused with Luciferin as in Studies 1-3.

After incubation overnight in this system, there was no contamination in the culture. Analysis of the flow through medium in the pan found no cells, suggesting that substantially all of the infused cells were retained in the placental vascular scaffold, likely due to the repeated circulation. The imaging analysis revealed that the cell distribution in the vascular system is similar to previous studies. There is improvement in the even distribution as shown in the pie-analysis. Strong signals also were found on the maternal face of the placenta, demonstrating that the cells were distributed throughout the thickness of the placenta, from the fetal face of the placenta (that is, the side with the umbilical cord) to the maternal face (that is, the opposite side).

### Conclusions

This set of experiments demonstrated a method to decellularize a whole human placenta, while retaining a substantially intact vascular scaffold, and that the vascular scaffold can conduct fluid efficiently with minimal loss. These results support the idea that a placental scaffold can be used as a platform for tissue and organ engineering. One potential application of the decellularized human placental vascular scaffold is to use it to engineer an extracorporeal organoid system.

### 5.2. Example 2: Cell Culture Using Decellularized Placental Vascular Scaffold

This Example demonstrates that additional human cell types can be infused into, and cultured within, decellularized placental vascular scaffolds.

### Materials and Methods

Human decellularized placenta scaffolds, prepared as described in Example 1, above, were used in this study.

Placental scaffold sterilization: Decellularized placenta scaffold was sterilized in 0.1% peracetic acid (PAA) in PBS for 3 hrs, with solution change every 1 hr, at room temperature. Agitation washes were performed 6 times in same amount of PBS, for one hour each.

Micro scaffold tissue preparation: The decellularized placenta tissue was cut into ∼2 to 3 cubic mm micro blocks with a surgical blade under sterile conditions. The blocks then were rinsed with cell culture medium.

Cells for recellularization in culture: GFP- PDAC®, HUVEC, 293/GFP cells (Cell Biolabs, Inc.), and HepaRG cells were used in the recellularization study. 293/GFP cells are a permanent cell line established from a primary embryonic human kidney transformed with human adenovirus type 5 DNA, engineered to stably express green fluorescent protein.

Quantum dot labeling of placenta-derived adherent cells (PDAC®): Quantum dots (QDs) are fluorescent semiconductor nanoparticles, recently adopted for use in *in vitro* and *in vivo* bioimaging. In this study, Q605 quantum dots (Invitrogen) were used for labeling PDAC® according to the vendor's protocol.

Cell growth assay: Growth of cells was determined using an assay based on the Promega MTS protocol using the CellTiter 96® AQueous Assay kit. In brief, 20µl of MTS solution was added into each well of a 96-well assay plate containing 100µl of cells in culture medium per well. The plate was incubated for 1 hour at 37°C in a humidified, 5% CO₂ atmosphere, followed by recordation of absorbance at 490nm using an ELISA plate reader.

GFP quantitative assay: 293/GFP cells were seeded on the decellularized placental vascular scaffold (∼2 x 2 x2 mm³) at 2 x10⁴ per 96-well. Cell attachment was measured in a quantitative GFP ELISA assay with a GFP ELISA Kit (AKR-121) (Cell Biolabs, Inc.) according to the vendor's protocol.

Live/dead cell determination: Live and dead cells were determined using the CytoSelect 96-well Anoikis Assay staining kit (CBA-081) (Cell Biolabs, Inc.).

Histology evaluation: The histology evaluation of decellularization and recellularization was performed using H&E staining and Masson Trichrome staining (Histoserv). The tissue sections were analyzed and recorded under a microscope.

Hepatocyte functional assays: Hepatocyte function was determined by measurement of albumin production (Albumin Blue Fluorescent Assay Kit; Active Motif, Carlsbad, CA); a urea assay (Quantichrom Urea Assay NC9283832 Bioassay Systems No. DIUR-500, Fisher Scientific Co.); and a P450assay (P450-Glo™ CYP3A4 Assay (Luciferin-PFBE) Cell-Based/Biochemical Assay, Promega).

### Results:

### Decellularized Human Placental Vascular Scaffold (DHPVS) Maintains Architecture and ECM Components

Decellularized placenta matrix represents a natural scaffold on which to repopulate cells and study the potential of stem cells to regenerate tissues or organoids. Decellularized placenta presents as transparent tissue with micro vascular tree extension, which has a rich cotton-like matrix.

To determine the effect of the decellularization process on placental morphology and architecture, the decellularized vascular scaffold was fixed in 4% paraformaldehyde and sectioned for histology analysis after hematoxylin and eosin stain (H & E). Decellularization removed substantially all cells, as evidenced by the lack of hematoxylin staining of cell nuclei. Placental structures readily visible under a microscope after H&E staining included large vessels surrounded by villous tissue, and the characteristic spongy matrix of the placenta, the smallest branches of the chorionic villi. In contrast to the images taken after decellularization, nondecellularized control placenta tissue showed a rich cell distribution with positive staining for hematoxylin (the nuclei of cells stained blue).

To assess whether the decellularization process had an adverse effect on extracellular matrix (ECM) components and their arrangement, the same tissue block of decellularized placenta was fixed and stained by H&E or Masson's trichrome. Visual inspection under microscopy revealed that the decellularized placenta tissue was indeed acellular and that the placenta matrix was intact. Matrix structure and collagen (ECM) (green color by Masson's staining) remained.

### Decellularized Human Placental Vascular Scaffold (DHPVS) Can Be Recellularized with Cells in Culture

To determine whether decellularized placenta scaffold could support cell growth in culture, PDAC® and human umbilical vein endothelial cells (HUVECs) were seeded over a block of DHPVS, and injected into the block, and cultured at 37°C in a humidified, 5% CO2 atmosphere for 14 days. The resulting DHPVS tissue blocks were sectioned and stained by H&E for recellularization analysis. Visual inspection under microscopy revealed that PDAC® could grow along the decellularized scaffold surface while maintaining a morphology indicative of live cells in the scaffold space, and that HUVEC had repopulated at least a portion of the vascular scaffold.

### Quantitative Evaluation of Cell Attachment and Growth on Decellularized Human Placental Vascular Scaffold (DHPVS)

To assess cell attachment on DHPVS in culture, a green fluorescent protein (GFP) quantization assay was used. Current ELISA-based assays allow detection of as little as 30pg/ml of GFP in the culture. 293/GFP cells seeded onto DHPVS showed ∼1.4 fold (p=0.004) better growth, by ELISA, than growth of the same cells in a 96-well plate after 24 h.

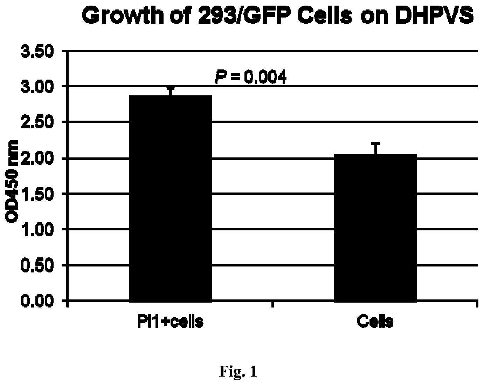

Figure 1 depicts the results of a representative experiment showing the increase in growth of 293/GFP cells seeded onto DHPVS as compared to growth of the same cells in culture.

### PDAC® Can Adhere and Proliferate on Decellularized Human Placental Vascular Scaffold (DHPVS)

To determine whether DHPVS is capable of supporting growth of cells, PDAC®-GFP cells were seeded onto DHPVS blocks and cultured. The cells proliferated during culture, and were well integrated into DHPVS for growth, as visualized by fluorescent imaging. Proliferation of PDAC®-GFP cells in DHPVS was also demonstrated by an MTS assay. PDAC®-GFP was seeded onto DHPVS blocks (∼2 x 2 x 2 mm³) at 2 x10⁴ per 96-well in growth medium. The scaffolds were moved on day 1 and day 3 to new 96-well for MTS assay to assess cell proliferation. Results indicated that DHPVS is a suitable scaffold for PDAC® adherence and proliferation.

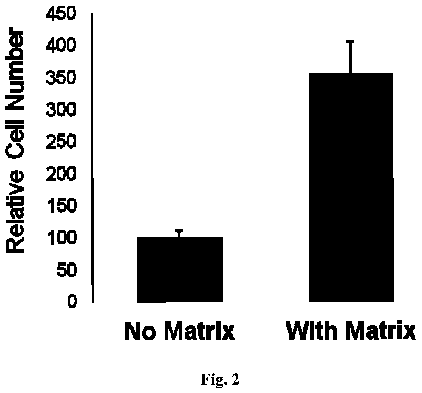

Subsequent experiments, using similar approaches to those described above, demonstrated that over a two-week culture period, PDAC®-GFP cells cultured on DHPVS increased approximately 2.5 fold as compared to the same cells grown in culture alone (Fig. 2).

### PDAC®-GFP Adhere and Grow on Decellularized Large, Small, and Micro Placental Vessels

Cell attachment and growth on decellularized vessels is an essential step for organoid regeneration. To assess PDAC® attachment and growth on isolated placental decellularized vessels, decellularized umbilical cord (∼2 mm thickness) was seeded with PDAC®-GFP (0.5 x 10⁶ in 2 ml medium) and cultured for 3 days, and visualized as described in Methods, above. The resulting fluorescent image showed that PDAC®-GFP cells preferentially adhere to and grow around decellularized vessels.

As an additional approach to assess cell attachment and growth in decellularized placental small vessels, PDAC®-GFP cells and Q605 labeled PDAC® were equally mixed to a total of 1x10⁶/mL and injected into small vessels within the DHPVM and cultured at 37°C in growth medium for 3 days. Photographs were taken for the two cell populations in the same vessel area using a fluorescent microscope. Representative fluorescent images of both GFP-expressing cells and Qdot-labeled cells show that PDAC® can attach and grow inside decellularized placental small vessels.

Cell attachment and growth of tissue specific cell were also demonstrated in decellularized placental micro-vessels. To achieve this goal, 300 µl of 293/GFP cells at a concentration of 1x10⁶/mL were infused into a DHPVM block (∼3 x 3 x 5 mm³) and cultured in a 24-well plate. After 7 days, the cells were visualized and photographed. Results demonstrated that 293/GFP cells can grow readily, and are well-distributed in DHPVS over the course of the 7 days.

### Hepatocytes can Maintain Functional Growth in a Decellularized Human Placental Vascular Scaffold

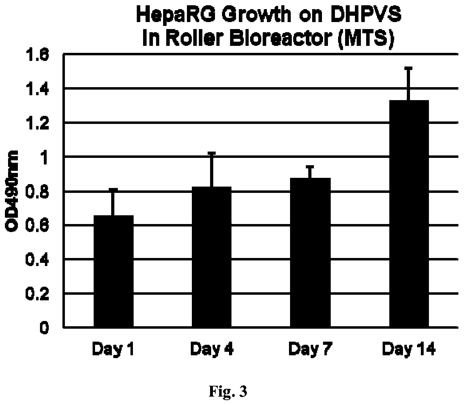

To establish hepatocyte growth on DHPVS, HepaRG cells were seeded at 2 x 10⁴/96-well over DHPVS, or injected into DHPVS, and cultured in 620 medium. The cells were visualized and photographed on Day 4 and Day 7 under phase contrast microscopy. Results indicated that HepaRG cells displayed an aggregate growth pattern and hepatocyte growth morphology in the presence of DHPVS. Results of a representative experiment assessing hepatocyte growth on DHPVS are shown in Figure 3.

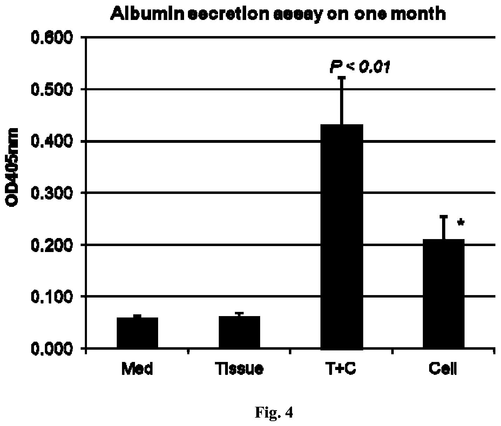

Functional analysis of hepatocytes using an albumin secretion assay was performed on culture day 3, day 6, and day 8. The culture medium samples were collected and tested by the Albumin Blue Fluorescent Assay Kit (Active Motif). Standard curves were generated using purified human albumin. Hepatocytes cultured alone were used as a control. Hepatocytes cultured on DHPVS were found to produce significantly more albumin than cells grown in the absence of DHPVS (P < 0.02), suggesting that hepatocytes maintain important functions when cultured in DHPVS. Results of a representative experiment assessing albumin production by hepatocytes grown on DHPVS are shown in Figure 4.

### 5.3. Example 3: Bioprinted Scaffolds Support Attachment And Growth Of Placental Stem Cells

This example demonstrates that synthetic material can be bioprinted to produce scaffolds of controlled fiber diameter and pore size, and that such scaffolds provide a suitable substrate for the application of extracellular matrix (ECM). This example further demonstrates that scaffolds comprising bioprinted synthetic material and ECM (hybrid scaffolds) represent a suitable substrate for the attachment and growth of cells, including placental cells, such as placental stem cells.

### Methods

To fabricate hybrid scaffolds comprising synthetic material and ECM, polycaprolactone (PCL) (Mn 45,000, Sigma) was first printed into scaffolds (54 x 54 x 0.64 mm) using a bioprinter (EnvisionTEC, Gladbeck, Germany). The printing conditions were as follows: temperature at 90°C, printing pressure 3∼5.5 bar, printing speed 2-6 mm/s, with suitable size needles. ECM was isolated from human placenta as previously described (see, e.g., Bhatia MB, Wounds 20, 29, 2008). Isolated ECM was applied to both sides of the bioprinted PCL scaffolds and allowed to dry (dehydrate) so as to generate hybrid scaffolds comprising PCL and ECM. The resultant hybrid PCL-ECM scaffolds were punched into 10 mm diameter disks, pre-wet with media overnight, and seeded with placental stem cells prepared in accordance with the methods described herein (see, e.g., Section 4.4) at 12,500 cells/cm². The cells were cultured over an 8-day time period. Calcein staining and MTS proliferation assays were performed in accordance with standard protocols at different time points (n=3) to determine cell viability and proliferation.

### Results

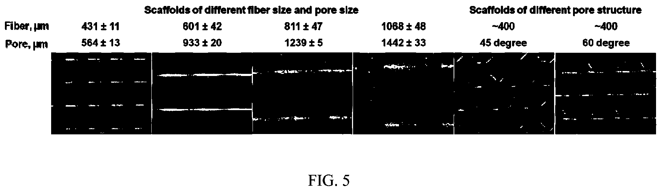

By optimizing printing conditions, PCL scaffolds of different fiber sizes, pore sizes and pore structures were generated (Fig. 5). The printed fibers formed a stable network for the generation of hybrid scaffolds comprising PCL and ECM. Further, the printing of varying fiber sizes and pore structures made it possible to make hybrid scaffolds comprising various properties.

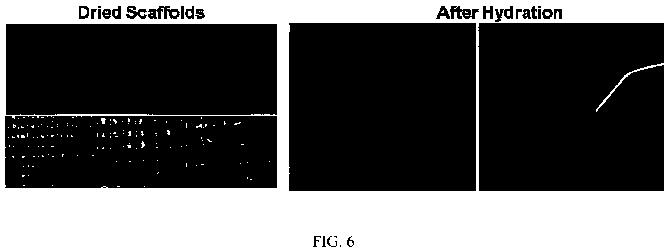

Dehydration of ECM on both sides of the bioprinted PCL scaffolds resulted in the generation of hybrid scaffolds. Good integration was seen between the PCL and ECM; no separation between the PCL and ECM was noticed when the hybrid scaffolds were manipulated, i.e. by processing or culturing of the scaffolds, which included rehydration (Fig. 6).

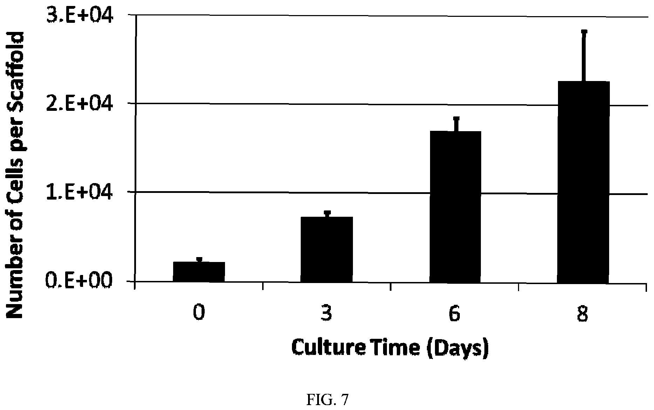

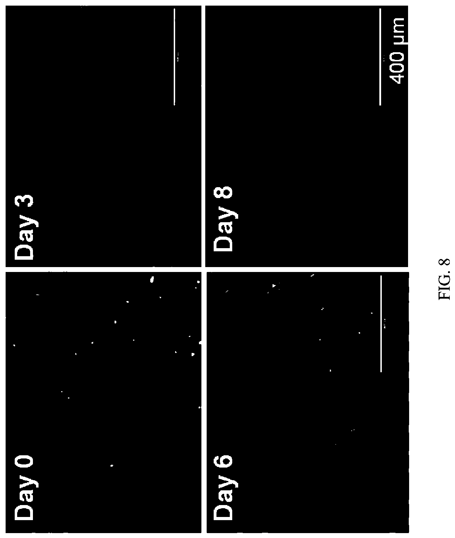

The placental stem cells spread over the surface of the hybrid scaffolds over time, and covered the majority of the surface of the hybrid scaffolds by day 6 of culture. The MTS cell proliferation assay demonstrated that cell number significantly increased over time (Fig. 7). In addition, the placental stem cells seeded on the hybrid scaffolds demonstrated good viability over the 8 day culture period, as indicated by calcein staining (Fig. 8). Together, these data indicate that PCL-ECM hybrid scaffolds support cellular attachment, survival, and growth.

### Conclusion

This example demonstrates that hybrid scaffolds comprising ECM and synthetic material (PCL) can be generated by methods that comprise bioprinting, and that cells not only attach to such scaffolds, but survive and proliferate when cultured on such scaffolds.

### 5.4. Example 4: Bioprinted Scaffolds Support Attachment And Growth Of Placental Stem Cells

This example demonstrates that synthetic material and ECM comprising cells, such as placental cells, e.g., placental stem cells, can be simultaneously bioprinted to produce hybrid scaffolds. As demonstrated by this Example, the bioprinted cells not only survive the bioprinting process, but proliferate over time in culture with the hybrid scaffolds.

### Methods

ECM was prepared as described in Example 3 and mixed with 0.5% alginate hydrogel containing 1 million/ml placental stem cells. Next, PCL and the cell-containing ECM were bioprinted, in layers, to generate a hybrid scaffold comprising PCL and ECM. In each layer of the scaffold, PCL was first printed, then the ECM/cell component was printed to fill the gaps in between the PCL lines. Two or five of such layers were printed and crosslinked with CaCl₂ solution to generate the hybrid scaffolds. The bioprinted, cell-containing scaffolds (cells/ECM/PCL) were cultured for seven days, and cell proliferation and survival were assessed at various time points via calcein staining and an MTS cell proliferation assay.

### Results

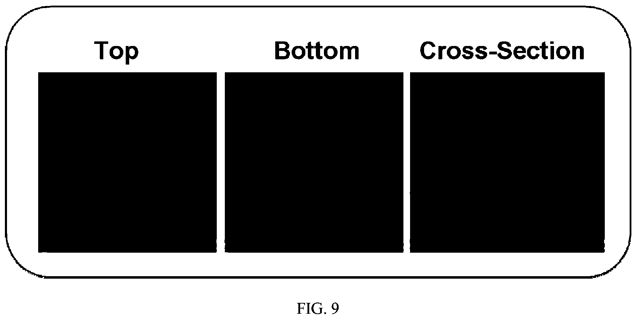

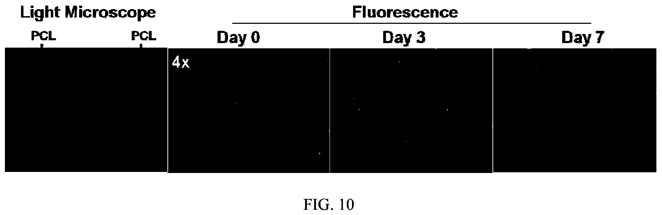

The bioprinted scaffolds maintained an intact structure throughout the duration of cell culture (Fig. 9). PCL provided a good structural support for the ECM hydrogels, which allowed for the generation of three-dimensional constructs. Following bioprinting and throughout culture, the cells were well-distributed throughout the three-dimensional constructs; cells were found throughout the depth of the scaffolds during culture (Fig. 10).

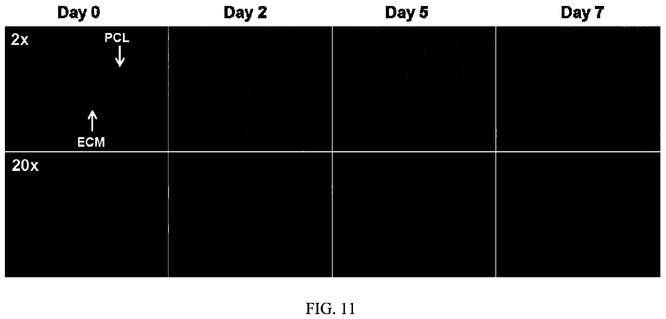

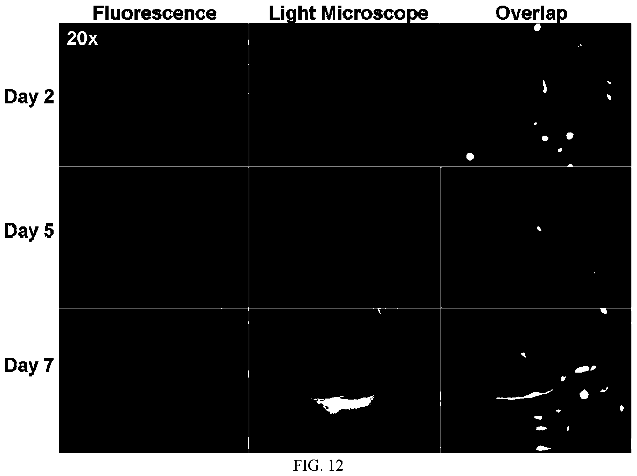

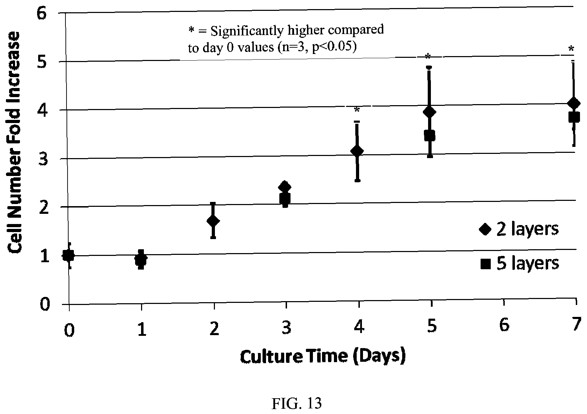

The placental stem cells survived the bioprinting process and continued to proliferate in the three-dimensional bioprinted hybrid scaffolds throughout culture, as evidenced by calcein staining (Fig. 11). As shown in Figure 12, most of the cells were found to spread throughout the ECM in the hybrid scaffolds, indicating that the ECM enhanced cell attachment and spreading in the ECM hydrogel. This was confirmed by comparing the location of cells in alginate alone with that of the cells in the scaffolds. Additionally, as shown in Figure 13, an MTS cell proliferation assay demonstrated increases in cell number for both the 2-layer and 5-layer scaffolds, indicating that these hybrid scaffolds supported cell growth.

### Conclusion

This example demonstrates that hybrid scaffolds comprising ECM and synthetic material (PCL) can be generated by methods that comprise simultaneous bioprinting of ECM and PCL. Also demonstrated by this Example is the fact that cells can be bioprinted along with the components of the hybrid scaffold (ECM and PCL), and that the cells survive the bioprinting process which indicates that cells, e.g., placental stem cells, can be bioprinted to surfaces such as decellularized placental vascular scaffolds. Further, the cells bioprinted along with the components of the hybrid scaffold proliferate when cultured on such scaffolds and intersperse throughout the scaffolds better than when cultured in cellular matrix (alginate) alone. This example further illustrates that cells can survive bioprinting.

### 5.5. Example 5: Functional Organoids Generated Using Decellularized Placental Vascular Scaffold

This example demonstrates that functional organoids can be engineered using decellularized placental vascular scaffolds (DPVS).

### Methods

A human thyroid tissue derived cell line CRL1803-TT ("TT"; available from the American Type Culture Collection ("ATCC")) and human umbilical vein endothelial cells (HUVEC) were cultured alone or co-cultured on a DPVS that was prepared as described above. As points of comparison, HUVEC and TT were co-cultured without DPVS as a substrate and cultured alone without DPVS as a substrate.

Each type of cell was seeded onto blocks of DPVS. Quantification of viable cells was measured using an MTT assay (Promega) and the function of TT cells was assessed by measuring secretion of calcitonin into the culture supernatant by the TT cells (calcitonin levels were measured by ELISA).

### Results

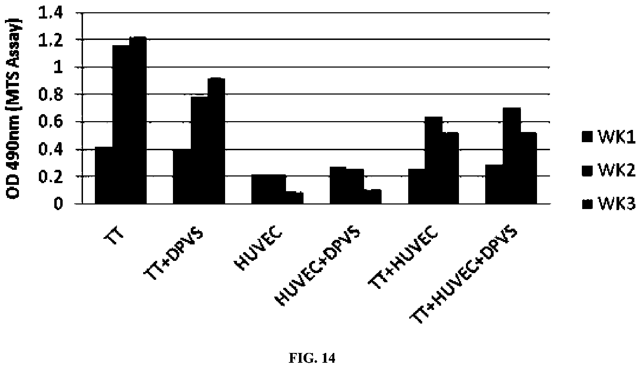

The MTT assay showed that when HUVEC and TT were co-cultured on DPVS, the total number viable cells was similar to those observed under normal culture conditions in tissue culture flasks (without DPVS), indicating that culturing on DPVS did not have a negative effect on cell viability when TT cells and HUVEC were co-cultured (Fig. 14). TT cells cultured alone on DPVS demonstrated slightly lower numbers of viable cells than TT cells cultured in tissue culture flasks, while HUVEC cultured alone on DPVS resulted in comparable numbers of viable cells than HUVEC cultured in tissue culture flasks (Fig. 14).

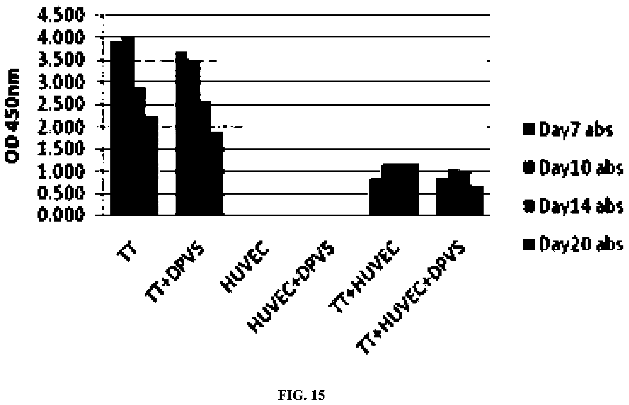

Production of calcitonin by TT cells was detected when TT cells were cultured on DPVS, although the levels were slightly reduced as compared to calcitonin production by TT cells cultured alone in culture flasks; calcitonin production by TT cells co-cultured with HUVEC was comparable whether the cells were cultured with DPVS or not, indicated that functionality of the TT cells was maintained upon culturing on DPVS (Fig. 15).

### Conclusion

Multiple types of cells of cells were cultured on DPVS while remaining viable and maintaining their function, thus indicating that DPVS is a suitable substrate for generation of organoids.

### 5.6. Example 6: Functional Organoids Generated Using Decellularized Placental Vascular Scaffold

This example further demonstrates that functional organoids can be engineered using decellularized placental vascular scaffolds (DPVS).

### Methods

The human thyroid tissue derived cell line CRL1803-TT described above ("TT") and human placenta derived adherent cells (PDAC®) were cultured alone or co-cultured on a DPVS that was prepared as described above. As points of comparison, PDAC® and TT were co-cultured without DPVS as a substrate and cultured alone without DPVS as a substrate.

Each type of cell was seeded onto blocks of DPVS. Quantification of viable cells was measured using an MTT assay (Promega). The function of TT cells was assessed by measuring secretion of calcitonin into the culture supernatant by the TT cells (calcitonin levels were measured by ELISA). The function of PDAC® was assessed by measuring the secretion of HGF (Hepatocyte Growth Factor) by the PDAC® into the culture supernatant (HGF levels were measured by ELISA).

### Results

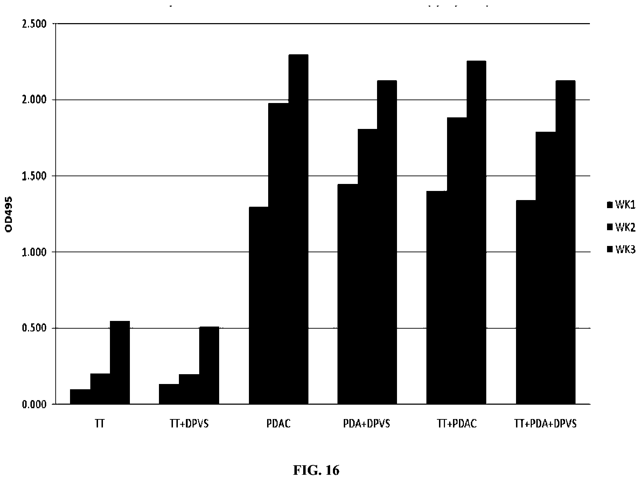

The MTT assay showed that when PDAC® and TT were co-cultured on DPVS, the total number viable cells was similar to those observed under normal culture conditions in tissue culture flasks (without DPVS), indicating that culturing on DPVS did not have a negative effect on cell viability when TT cells and PDAC® were co-cultured (Fig. 16). In this experiment, TT cells cultured alone on DPVS and PDAC® cultured alone on DPVS resulted in comparable numbers of viable cells as compared to TT cells and PDAC® cultured in tissue culture flasks alone (Fig. 16).

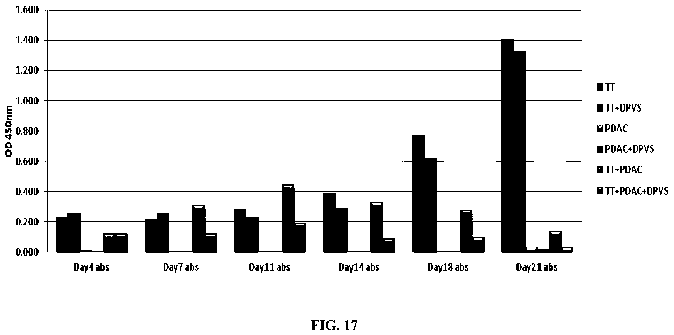

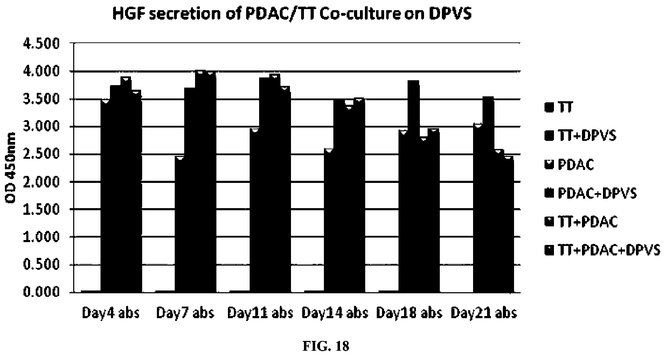

As demonstrated in Example 5, production of calcitonin by TT cells was detected when TT cells were cultured on DPVS (Fig. 17). Calcitonin production by TT cells co-cultured with PDAC® was detectable, whether the cells were cultured with DPVS or not, but levels varied over the culture period (Fig. 17). As with TT cells, PDAC® maintained their functionality when cultured on DPVS, as demonstrated by their ability to secrete HGF (Fig. 18).

### Conclusion

This Example demonstrates that multiple types of cells, including placental stem cells, can be cultured on DPVS and that viability and functionality of the cells is maintained during the culture period, further confirming that DPVS is a suitable substrate for generation of organoids.

### 5.7. Example 7: Cells Cultured on DPVS Remain Metabolically Active

This example demonstrates that cells cultured on DPVS remain viable and metabolically active.

HCT116 cells in culture medium (available from ATCC) were infused into a decellularized placenta (a DPVS) via the umbilical cord arteries and placed in an incubator. Circulation of the cells in the DPVS was maintained using a pump. The culture was maintained for 48 hours in the incubator and portions of tissues were dissected from different anatomical locations of the cultured DPVS to examine cell viability. Twenty different pieces of tissue were analyzed from each anatomical location of the cultured placenta (top, middle and bottom of the DPVS). Cell viability was measured with an MTS assay (Promega).

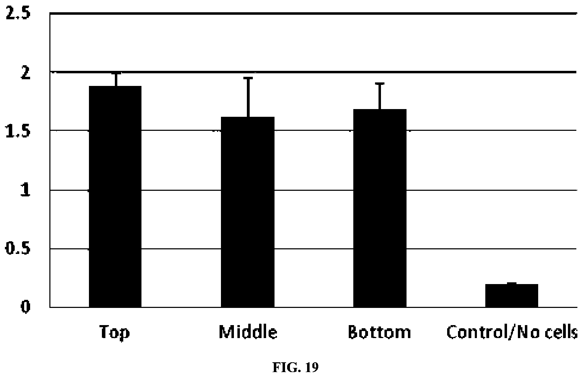

After 48 hours of culture, the cells were localized relatively evenly in all three parts of the placenta, with each 0.5cm³ tissue section estimated to contain approximately 40,000 to 60,000 cells (Fig. 19).

HCT116 cell activity in the DPVS was assessed by collecting the culture medium at different time points and analyzing the nutrient conditions of the culture medium as compared to the nutrient conditions in control medium (identical medium without HCT116 cells) over the same time course. The nutrient conditions were assessed using an automatic medium analyzer. As shown in Table 1, below, cGlu (Glucose) was consumed by the HCT116 cells cultured in the DPVS from 5 hours to 24 hours of culture, indicating that the cells were metabolically active in addition to remaining viable.

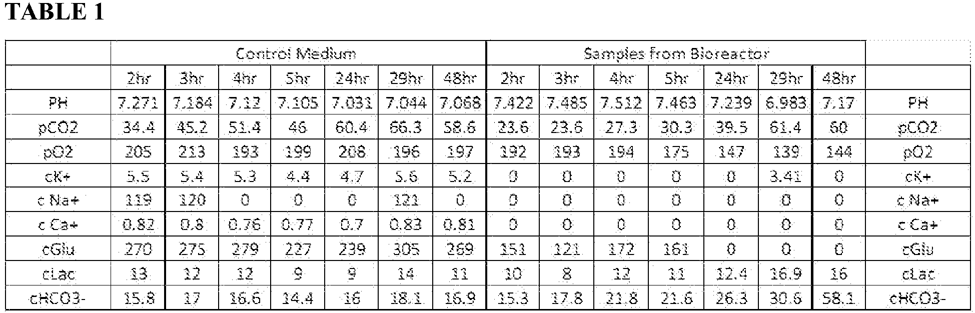

### 5.8. Example 8: Stem Cells Cultured on DPVS Differentiate

This example demonstrates that decellularized human placenta vascular scaffold (DPVS) can be used as a platform for tissue engineering based on the ability of DPVS to support the differentiation of stem cells.

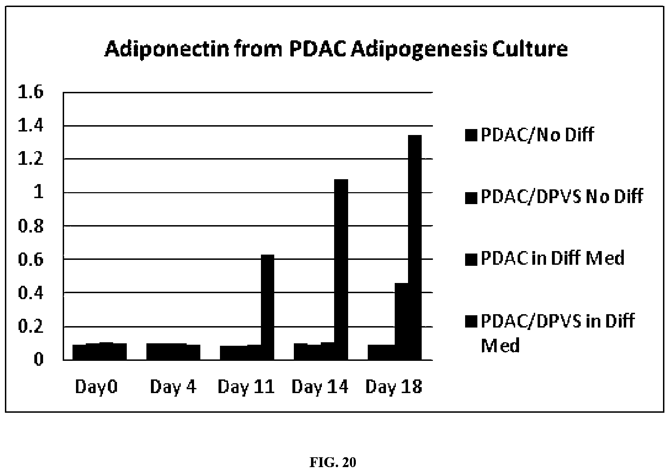

PDAC were seeded onto blocks of DPVS and cultured in 24-well plates under non-differentiation conditions or differentiation conditions using the Mesenchymal Stem Cell Adipogenesis Kit (Chemicon International) according to the manufacturer's protocols. This kit assays for differentiation of cells into adipocytes, which produce adiponectin. An ELISA assay was used to measure the production of adiponectin from PDAC® cultured with or without DPVS. Culture medium was collected from day 0 to day 18 of the culture. Without differentiation induction, PDAC® alone and PDAC® cultured on DPVS did not produce adiponectin (Fig. 20). However, when PDAC® cultured on DPVS were cultured in differentiation medium, adiponectin was detected in the culture medium beginning on day 11, whereas PDAC® cultured in medium alone did not produce adiponectin until day 18 (Fig. 20). Moreover, PDAC® cultured on DPVS produced higher levels of adiponectin than PDAC® cultured in medium alone (Fig. 20). Similar to PDAC®, human bone marrow derived mesenchymal cells were able to differentiate into adipocytes when cultured on DPVS.

### 5.9. Example 9: DPVS Mimics the In Vivo Environment

This example demonstrates that decellularized human placenta vascular scaffold (DPVS) is an ideal cell culture environment that can mimic *in vivo* culture conditions.

HepaRG cells were cultured either on DPVS or in tissue culture flasks under normal culture conditions. Culture medium was collected twice a week for one month from the separate cultures. The levels of glucose and lactate in the collected culture media were determined using a radiometer machine. The stoichiometric ratio of lactate to glucose (ΔL / ΔG), which represents the moles of lactate produced by the cells as compared to the moles of Glucose consumed by the cells, was assessed for the two culture conditions.

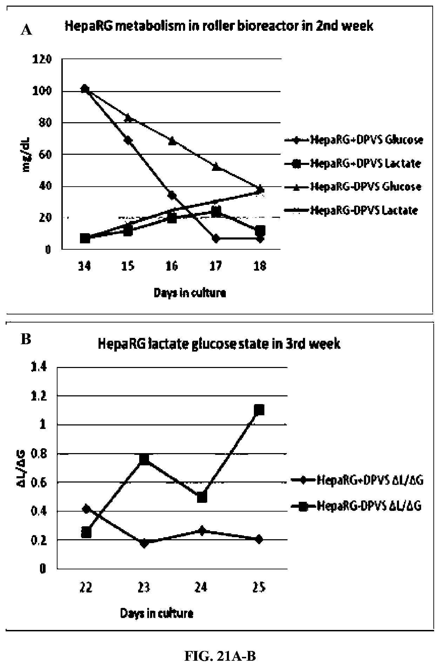

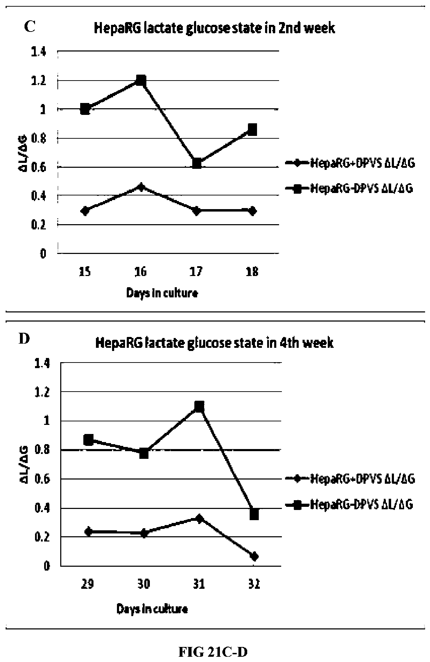

As demonstrated in Figure 21, the ΔL/ΔG value for the HepaRG/DPVS culture group was consistently smaller than the value from HepaRG control group (Fig. 21B-D). This lower ΔL/ΔG value suggests that the HepaRG cultured on DPVS cells undergo metabolism in a more efficient state, characterized by a dramatic reduction in the amount of lactate produced by the cells. This low ΔL/ΔG state is a physiological state with minimal waste product formation, that is comparable to the environment in vivo.

### 5.10. Example 10: Placental Cotyledons Can Be Used As DPVS

This example demonstrates that placental cotyledons can be used as DPVS.

Placental cotyledons were isolated from placenta and decellularized as described above. The average area of a single cotyledon was determined to represent about 10% of a whole placenta. Decellularized placenta cotyledons comprise vasculature and were shown to be able to circulate fluid. The fluid circulation rates (efflux volume/influx volume) of single cotyledons isolated from seven different placentas were evaluated and determined to be, on averge, 28.92%. Thus, placental cotyledons represent smaller physical units that can be used as DPVS in accordance with the uses described in the examples above.

To demonstrate that isolated single placental cotyledons can be used for tissue engineering, the ability of placental cotyledons to support cell growth was assessed. The vasculature of a single isolated placental cotyledon was infused with Luciferase expressing cells (MDA-MB231/Luc; Cell Biolabs Inc.) in a medium containing the luciferase substrate Luciferin (Sigma, 100ng/mL). The single cotyledon was placed on a petri dish and imaged using the Xenogen IVIS imaging system. Cells were observed to localize within the vasculature of the placental cotyledon, indicating that the vasculature remained intact and thus that placental cotyledons represent a suitable DPVS for tissue engineering.

## Claims

1. An organoid, comprising one or more types of cells, and comprising decellularized placental vascular scaffold,
wherein said organoid performs at least one function of an organ, or a tissue from an organ;
wherein said at least one function of an organ or tissue from an organ is production of a protein, growth factor, cytokine, interleukin, or small molecule characteristic of at least one cell type from said organ or tissue;
wherein said decellularized placental vascular scaffold comprises substantially intact placental vasculature matrix;
wherein said cells have been genetically engineered to produce a protein or polypeptide not naturally produced by the cell, or have been genetically engineered to produce a protein or polypeptide in an amount greater than that naturally produced by the cell; and
wherein said cellular composition comprises differentiated cells.

2. The organoid of claim 1, additionally comprising a synthetic matrix, preferably
wherein said synthetic matrix comprises or consists of a polymer or a thermoplastic, wherein preferably said thermoplastic is polycaprolactone, polylactic acid, polybutylene terephthalate, polyethylene terephthalate, polyethylene, polyester, polyvinyl acetate, or polyvinyl chloride; or
said polymer is polyvinylidine chloride, poly(o-carboxyphenoxy)-p-xylene) (poly(o-CPX)), poly(lactide-anhydride) (PLAA), n-isopropyl acrylamide, acrylamide, pent erythritol diacrylate, polymethyl acrylate, carboxymethylcellulose, or poly(lactic-co-glycolic acid) (PLGA); or
said polymer is polyacrylamide.

3. The organoid of claim 1 or 2, wherein said one or more types of cells comprise:
natural killer (NK) cells, wherein preferably
said NK cells comprise CD56⁺ CD16⁻ placental intermediate natural killer (PiNK) cells;
dendritic cells;
thymocytes;
thymocytes, lymphoid cells, epithelial reticular cells, and thymic stromal cells;
follicular cells, wherein preferably said organoid comprises cells that express thyroglobulin;
follicular cells, wherein said organoid additionally comprises thyroid epithelial cells and parafollicular cells;
stem cells or progenitor cells, wherein preferably
said stem cells or progenitor cells are induced pluripotent stem cells, mesenchymal stem cells, bone marrow-derived mesenchymal stem cells, bone marrow-derived mesenchymal stromal cells, tissue plastic-adherent placental stem cells (PDAC®), umbilical cord stem cells, amniotic fluid stem cells, amnion derived adherent cells (AMDACs), osteogenic placental adherent cells (OPACs), adipose stem cells, limbal stem cells, dental pulp stem cells, myoblasts, endothelial progenitor cells, neuronal stem cells, exfoliated teeth derived stem cells, hair follicle stem cells, dermal stem cells, parthenogenically derived stem cells, reprogrammed stem cells, amnion derived adherent cells, or side population stem cells;
hematopoietic stem cells or hematopoietic progenitor cells;
tissue culture plastic-adherent CD34⁻, CD10⁺, CD105⁺, and CD200⁺ placental stem cells, wherein preferably
said placental stem cells are additionally one or more of CD45⁻, CD80⁻, CD86⁻, or CD90⁺; or
differentiated cells, wherein preferably
said differentiated cells comprise endothelial cells, epithelial cells, dermal cells, endodermal cells, mesodermal cells, fibroblasts, osteocytes, chondrocytes, natural killer cells, dendritic cells, hepatic cells, pancreatic cells, or stromal cells;
said differentiated cells comprise salivary gland mucous cells, salivary gland serous cells, von Ebner's gland cells, mammary gland cells, lacrimal gland cells, ceruminous gland cells, eccrine sweat gland dark cells, eccrine sweat gland clear cells, apocrine sweat gland cells, gland of Moll cells, sebaceous gland cells. bowman's gland cells, Brunner's gland cells, seminal vesicle cells, prostate gland cells, bulbourethral gland cells, Bartholin's gland cells, gland of Littre cells, uterus endometrium cells, isolated goblet cells, stomach lining mucous cells, gastric gland zymogenic cells, gastric gland oxyntic cells, pancreatic acinar cells, paneth cells, type II pneumocytes, clara cells, somatotropes, lactotropes, thyrotropes, gonadotropes, corticotropes, intermediate pituitary cells, magnocellular neurosecretory cells, gut cells, respiratory tract cells, thyroid epithelial cells, parafollicular cells, parathyroid gland cells, parathyroid chief cell, oxyphil cell, adrenal gland cells, chromaffin cells, Leydig cells, theca interna cells, corpus luteum cells, granulosa lutein cells, theca lutein cells, juxtaglomerular cell, macula densa cells, peripolar cells, mesangial cell,
blood vessel and lymphatic vascular endothelial fenestrated cells, blood vessel and lymphatic vascular endothelial continuous cells, blood vessel and lymphatic vascular endothelial splenic cells, synovial cells, serosal cell (lining peritoneal, pleural, and pericardial cavities), squamous cells, columnar cells, dark cells, vestibular membrane cell (lining endolymphatic space of ear), stria vascularis basal cells, stria vascularis marginal cell (lining endolymphatic space of ear), cells of Claudius, cells of Boettcher, choroid plexus cells, pia-arachnoid squamous cells, pigmented ciliary epithelium cells, nonpigmented ciliary epithelium cells, corneal endothelial cells, peg cells,
respiratory tract ciliated cells, oviduct ciliated cell, uterine endometrial ciliated cells, rete testis ciliated cells, ductulus efferens ciliated cells, ciliated ependymal cells,
epidermal keratinocytes, epidermal basal cells, keratinocyte of fingernails and toenails, nail bed basal cells, medullary hair shaft cells, cortical hair shaft cells, cuticular hair shaft cells, cuticular hair root sheath cells, hair root sheath cells of Huxley's layer, hair root sheath cells of Henle's layer, external hair root sheath cells, hair matrix cells,
surface epithelial cells of stratified squamous epithelium, basal cell of epithelia, urinary epithelium cells,
auditory inner hair cells of organ of Corti, auditory outer hair cells of organ of Corti, basal cells of olfactory epithelium, cold-sensitive primary sensory neurons, heat-sensitive primary sensory neurons, Merkel cells of epidermis, olfactory receptor neurons, pain-sensitive primary sensory neurons, photoreceptor rod cells, photoreceptor blue-sensitive cone cells, photoreceptor green-sensitive cone cells, photoreceptor red-sensitive cone cells, proprioceptive primary sensory neurons, touch-sensitive primary sensory neurons, type I carotid body cells, type II carotid body cell (blood pH sensor), type I hair cell of vestibular apparatus of ear (acceleration and gravity), type II hair cells of vestibular apparatus of ear, type I taste bud cells,
cholinergic neural cells, adrenergic neural cells, peptidergic neural cells, inner pillar cells of organ of Corti, outer pillar cells of organ of Corti, inner phalangeal cells of organ of Corti, outer phalangeal cells of organ of Corti, border cells of organ of Corti, Hensen cells of organ of Corti, vestibular apparatus supporting cells, taste bud supporting cells, olfactory epithelium supporting cells, Schwann cells, satellite cells, enteric glial cells, astrocytes, neurons, oligodendrocytes, spindle neurons, anterior lens epithelial cells, crystallin-containing lens fiber cells, hepatocytes, adipocytes, white fat cells, brown fat cells, liver lipocytes, kidney glomerulus parietal cells, kidney glomerulus podocytes, kidney proximal tubule brush border cells, loop of Henle thin segment cells, kidney distal tubule cells, kidney collecting duct cells, type I pneumocytes, pancreatic duct cells, nonstriated duct cells, duct cells, intestinal brush border cells, exocrine gland striated duct cells, gall bladder epithelial cells, ductulus efferens nonciliated cells, epididymal principal cells, epididymal basal cells, ameloblast epithelial cells, planum semilunatum epithelial cells, organ of Corti interdental epithelial cells, loose connective tissue fibroblasts, corneal keratocytes, tendon fibroblasts, bone marrow reticular tissue fibroblasts, nonepithelial fibroblasts, pericytes, nucleus pulposus cells, cementoblast/cementocytes, odontoblasts, odontocytes, hyaline cartilage chondrocytes, fibrocartilage chondrocytes, elastic cartilage chondrocytes, osteoblasts, osteocytes, osteoclasts, osteoprogenitor cells, hyalocytes, stellate cells (ear), hepatic stellate cells (Ito cells), pancreatic stelle cells,
red skeletal muscle cells, white skeletal muscle cells, intermediate skeletal muscle cells, nuclear bag cells of muscle spindle, nuclear chain cells of muscle spindle, satellite cells, ordinary heart muscle cells, nodal heart muscle cells, Purkinje fiber cells, smooth muscle cells, myoepithelial cells of iris, myoepithelial cell of exocrine glands,
reticulocytes, megakaryocytes, monocytes, connective tissue macrophages. epidermal Langerhans cells, dendritic cells, microglial cells, neutrophils, eosinophils, basophils, mast cell, helper T cells, suppressor T cells, cytotoxic T cell, natural Killer T cells, B cells, natural killer cells,
melanocytes, retinal pigmented epithelial cells,
oogonia/oocytes, spermatids, spermatocytes, spermatogonium cells, spermatozoa, ovarian follicle cells, Sertoli cells, thymus epithelial cell, and/or interstitial kidney cells.

4. The organoid of any of claims 1 to 3, wherein the organoid comprises about 10¹² cells, about 10¹¹ cells, about 10¹⁰ cells, or about 10⁹ cells.

5. The organoid of any of claims 1 to 4, wherein said protein or polypeptide is
(i) a cytokine or a peptide comprising an active part thereof, wherein preferably said cytokine is adrenomedullin (AM), angiopoietin (Ang), bone morphogenetic protein (BMP), brain-derived neurotrophic factor (BDNF), epidermal growth factor (EGF), erythropoietin (Epo), fibroblast growth factor (FGF), glial cell line-derived neurotrophic factor (GNDF), granulocyte colony stimulating factor (G-CSF), granulocyte-macrophage colony stimulating factor (GM-CSF), growth differentiation factor (GDF-9), hepatocyte growth factor (HGF), hepatoma derived growth factor (HDGF), insulin-like growth factor (IGF), migration-stimulating factor, myostatin (GDF-8), myelomonocytic growth factor (MGF), nerve growth factor (NGF), placental growth factor (PlGF), platelet-derived growth factor (PDGF), thrombopoietin (Tpo), transforming growth factor alpha (TGF-α), TGF-β, tumor necrosis factor alpha (TNF-α), vascular endothelial growth factor (VEGF), or a Wnt protein;
(ii) a soluble receptor for AM, Ang, BMP, BDNF, EGF, Epo, FGF, GNDF, G-CSF, GM-CSF, GDF-9, HGF, HDGF, IGF, migration-stimulating factor, GDF-8, MGF, NGF, PlGF, PDGF, Tpo, TGF-α, TGF-β, TNF-α, VEGF, or a Wnt protein;
(iii) an interleukin, wherein preferably
said interleukin is interleukin-1 alpha (IL-1α), IL-1β, IL-1F1, IL-1F2, IL-1F3, IL-1F4, IL-1F5, IL-1F6, IL-1F7, IL-1F8, IL-1F9, IL-2, IL-3, IL-4, IL-5, IL-6, IL-7, IL-8, IL-9, IL-10, IL-11, IL-12 35 kDa alpha subunit, IL-12 40 kDa beta subunit, both IL-12 alpha and beta subunits, IL-13, IL-14, IL-15, IL-16, IL-17A, IL-17B, IL-17C, IL-17D, IL-17E, IL-17F isoform 1, IL-17F isoform 2, IL-18, IL-19, IL-20, IL-21, IL-22, IL-23 p19 subunit, IL-23 p40 subunit, IL-23 p19 subunit and IL-23 p40 subunit together, IL-24, IL-25, IL-26, IL-27B, IL-27-p28, IL-27B and IL-27-p28 together, IL-28A, IL-28B, IL-29, IL-30, IL-31, IL-32, IL-33, IL-34, IL-35, IL-36α, IL-36β, IL-36y;
(iv) a soluble receptor for IL-1α, IL-1β, IL-1F1, IL-1F2, IL-1F3, IL-1F4, IL-1F5, IL-1F6, IL-1F7, IL-1F8, IL-1F9, IL-2, IL-3, IL-4, IL-5, IL-6, IL-7, IL-8, IL-9, IL-10, IL-11, IL-12 35 kDa alpha subunit, IL-12 40 kDa beta subunit, IL-13, IL-14, IL-15, IL-16, IL-17A, IL-17B, IL-17C, IL-17D, IL-17E, IL-17F isoform 1, IL-17F isoform 2, IL-18, IL-19, IL-20, IL-21, IL-22, IL-23 p19 subunit, IL-23 p40 subunit, IL-24, IL-25, IL-26, IL-27B, IL-27-p28, IL-28A, IL-28B, IL-29, IL-30, IL-31, IL-32, IL-33, IL-34, IL-35, IL-36α, IL-36β, IL-36γ;
(v) an interferon (IFN), wherein preferably
said interferon is IFN-α, IFN-β, IFN-γ, IFN-λ1, IFN-λ2, IFN-λ3, IFN-K, IFN-ε, IFN-κ, IFN-τ, IFN-δ, IFN-ζ, IFN-ω, or IFN-v;
(vi) a soluble receptor for IFN-α, IFN-β, IFN-γ, IFN-λ1, IFN-λ2, IFN-λ3, IFN-K, IFN-ε, IFN-κ, IFN-τ, IFN-δ, IFN-ζ, IFN-ω, or IFN-v;
(vii) insulin or proinsulin;
(viii) a receptor for insulin;
(ix) insulin or a receptor for insulin, wherein said cells have additionally been genetically engineered to produce one or more of prohormone convertase 1, prohormone convertase 2, or carboxypeptidase E.
(x) leptin (LEP);
(xi) erythropoietin;
(xii) thrombopoietin;
(xiii) tyrosine 3-monooxygenase;
(xiv) tyrosine 3-monooxygenase, wherein said cells are further engineered to express aromatic L-amino acid decarboxylase;
(xv) a hormone or prohormone, wherein preferably
said hormone is antimullerian hormone (AMH), adiponectin (Acrp30), adrenocorticotropic hormone (ACTH), angiotensin (AGT), angiotensinogen (AGT), antidiuretic hormone (ADH), vasopressin, atrial-natriuretic peptide (ANP), calcitonin (CT), cholecystokinin (CCK), corticotrophin-releasing hormone (CRH), erythropoietin (Epo), follicle-stimulating hormone (FSH), testosterone, estrogen, gastrin (GRP), ghrelin, glucagon (GCG), gonadotropin-releasing hormone (GnRH), growth hormone (GH), growth hormone releasing hormone (GHRH), human chorionic gonadotropin (hCG), human placental lactogen (HPL), inhibin, leutinizing hormone (LH), melanocyte stimulating hormone (MSH), orexin, oxytocin (OXT), parathyroid hormone (PTH), prolactin (PRL), relaxin (RLN), secretin (SCT), somatostatin (SRIF), thrombopoietin (Tpo), thyroid-stimulating hormone (Tsh), and/or thyrotropin-releasing hormone (TRH).
(xvi) cytochrome P450 side chain cleavage enzyme (P450SCC); or
(xvii) a protein missing or malfunctioning in an individual who has a genetic disorder or disease, wherein preferably
said genetic disorder or disease is familial hypercholesterolemia and said protein is low density lipoprotein receptor (LDLR);
said genetic disorder or disease is polycystic kidney disease, and said protein is polycystin-1 (PKD1), PKD-2 or PKD3; or
said genetic disorder or disease is phenylketonuria and said protein is phenylalanine hydroxylase.

6. The organoid of claim 5, wherein said organoid comprises 1 x 10⁸ cells and produces at least 1.0 to 10 µM of said protein or polypeptide in *in vitro* culture in growth medium over 24 hours.

7. The organoid of any of claims 1 to 6, wherein said organoid comprises an immune suppressive compound or an anti-inflammatory compound, wherein preferably
said compound is a non-steroidal anti-inflammatory drug (NSAID), acetaminophen, naproxen, ibuprofen, acetylsalicylic acid, a steroid, an anti-T cell receptor antibody, an anti-IL-2 receptor antibody, basiliximab, daclizumab (ZENAPAX®), anti T cell receptor antibodies (e.g., Muromonab-CD3), azathioprine, a corticosteroid, cyclosporine, tacrolimus, mycophenolate mofetil, sirolimus, calcineurin inhibitors, and the like. In a specific embodiment, the immumosuppressive agent is a neutralizing antibody to macrophage inflammatory protein (MIP)-1α or MIP-1β

8. The organoid of any of claims 1 to 7, wherein said organoid performs at least one function of a liver, kidney, pancreas, thyroid or lung.

9. The organoid of any of claims 1 to 8, wherein said organoid comprises
(i) pituitary gland acidophil cells, pituitary basophil cells, or both pituitary gland acidophil cells and basophil cells,
wherein preferably said organoid comprises
pituitary somatotropes,
pituitary mammotrophs,
pituitary corticotrophs,
pituitary thyrotrophs,
pituitary gonadotrophs,
two or more of pituitary somatotrophs, pituitary mammotrophs, pituitary corticotrophs, pituitary thyrotrophs, and/or pituitary gonadotrophs, or
cells that produce or have been genetically engineered to produce one or more of GH, STH, PRL, ACTH, MSH, TSH, FSH, ADH and/or LH;
(ii) hypothalamic neurons, pituicytes, or both hypothalamic neurons and pituicytes,
wherein preferably said organoid comprises cells that produce or have been genetically engineered to produce one or both of ADH and/or oxytocin;
(iii) endothelial vessel-forming cells,
(iv) thyroid epithelial cells, thyroid parafollicular cells, thyroglobulin-producing cells, or two or more of thyroid epithelial cells, thyroid parafollicular cells, and thyroglobulin-producing cells,
wherein preferably said organoid comprises cells that produce or have been genetically engineered to produce one or more of T3, T4 and/or calcitonin;
(v) parathyroid chief cells, parathyroid oxyphil cells, or both parathyroid chef cells and parathyroid oxyphil cells,
wherein preferably said organoid comprises cells that produce or have been genetically engineered to produce said PTH;
(vi) adrenal gland zona glomerulosa cells, adrenal gland fasciculate cells, adrenal gland zona reticulata cells, or adrenal gland chromaffin cells,
wherein preferably said organoid comprises cells that produce or have been genetically engineered to produce one or more of aldosterone, 18 hydroxy 11 deoxycorticosterone, cortisol, fludrocortisones, a non-cortisol glucocorticoid, epinephrine, adrenosterone, and/or dehydroepiandrosterone;
(vii) hepatocytes,
wherein preferably said organoid comprises cells that produce one or more of coagulation factor I (fibrinogen); coagulation factor II (prothrombin); coagulation factor V (factor five); coagulation factor VII (proconvertin); coagulation factor IX (Christmas factor); coagulation factor X (Stuart-Prower factor; prothrombinase); coagulation factor XI (plasma thromboplastin antecedent); protein C (autoprothrombin IIA; blood coagulation factor XIV), protein S, antithrombin, IGF-1 or thrombopoietin;
(viii) hepatic vessel endothelial cells;
(ix) pancreatic alpha cells, pancreatic beta cells, pancreatic delta cells, pancreatic PP cells, pancreatic epsilon cells, or two or more of pancreatic alpha cells, pancreatic beta cells, pancreatic delta cells, pancreatic PP cells, and/or pancreatic epsilon cells,
wherein preferably said organoid comprises cells that produce a detectable amount of one or more of insulin, glucagon, amylin, somatostatin, pancreatic polypeptide, and/or grehlin.

10. An organoid as defined in claim 9(i) for use in a method of treating an individual in need of:
(a) human growth hormone (hGH);
(b) somatotrophic hormone (STH);
(c) prolactin (PRL), wherein preferably said individual has one or more of metabolic syndrome, arteriogenic erectile dysfunction, premature ejaculation, oligozoospermia, asthenospermia, hypofunction of seminal vesicles, or hypoandrogenism;
(d) adrenocorticotropic hormone (ACTH), wherein preferably said individual has Addison's disease.
(e) melanocyte-stimulating hormone (MSH), wherein preferably said individual has Alzheimer's' disease;
(f) thyroid-stimulating hormone (TSH), wherein preferably said individual has or manifests cretinism;
(g) follicle-stimulating hormone (FSH), wherein preferably said individual has or manifests infertility or azoospermia; or
(h) leutenizing hormone (LH), wherein preferably said individual has or manifests low testosterone, low sperm count or infertility.

11. An organoid as defined in claim 9(ii) for use in a method of treating an individual in need of
(a) antidiuretic hormone (ADH), wherein preferably said individual has hypothalamic diabetes insipidus; or
(b) oxytocin.

12. An organoid as defined in claim 9(iv) for use in a method of treating an individual in need of
(a) thyroxine (T4), wherein preferably said individual has or manifests mental retardation, dwarfism, weakness, lethargy, cold intolerance, or moon face;
(b) triiodothyronine (T3), wherein preferably said individual has heart disease, wherein more preferably said individual has a serum concentration of T3 that is less than 3.1 pmol/L; or
(c) calcitonin, wherein preferably said individual has osteoporosis or chronic autoimmune hypothyroidism.

13. An organoid as defined in claim 9(v) for use in a method of treating an individual in need of parathyroid hormone (PTH).

14. An organoid as defined in claim 9(vi) for use in a method of treating an individual in need of:
(a) aldosterone, wherein said individual has idiopathic hypoaldosteronism, hypereninemic hypoaldosteronism, or hyporeninemic hypoaldosteronism, or chronic renal insufficiency;
(b) 18 hydroxy 11 deoxycorticosterone;
(c) fludrocortisone;
(d) cortisol, wherein preferably said individual has acute adrenal deficiency, Addison's disease, or hypoglycemia;
(e) a non-cortisol glucocorticoid;
(f) epinephrine;
(g) adrenosterone; or
(h) dehydroepiandrosterone.

15. An organoid as defined in claim 9(vii) for use in a method of treating an individual in need of a compound, wherein said compound is coagulation factor I (fibrinogen); coagulation factor II (prothrombin); coagulation factor V (factor five); coagulation factor VII (proconvertin); coagulation factor IX (Christmas factor); coagulation factor X (Stuart-Prower factor; prothrombinase); coagulation factor XI (plasma thromboplastin antecedent); protein C (autoprothrombin IIA; blood coagulation factor XIV); protein S; antithrombin; IGF-1; and/or thrombopoietin.

16. An organoid as defined in claim 9(ix) for use in a method of treating an individual in need of:
(a) glucagon;
(b) insulin, wherein preferably said individual has diabetes mellitus;
(c) amylin;
(d) grehlin; or
(e) pancreatic polypeptide.

## Patentansprüche

1. Organoid, das einen oder mehrere Zelltypen und ein dezellularisiertes Plazentagefäßgerüst umfasst,
wobei das Organoid zumindest eine Funktion eines Organs oder eines Gewebes von einem Organ erfüllt;
wobei die zumindest eine Funktion eines Organs oder eines Gewebes von einem Organ die Produktion eines Proteins, Wachstumsfaktors, Zytokins, Interleukins oder eines kleinen Moleküls ist, das/der für zumindest einen Zelltyp des Organs oder Gewebes typisch ist;
wobei das dezellularisierte Plazentagefäßgerüst eine im Wesentlichen intakte Plazentagefäßstrukturmatrix umfasst;
wobei die Zellen gentechnisch verändert wurden, um ein Protein oder ein Polypeptid zu erzeugen, das durch die Zelle natürlicherweise nicht erzeugt wird, oder gentechnisch verändert wurden, um ein Protein oder ein Polypeptid in einer größeren Menge zu erzeugen als natürlich durch die Zelle erzeugt wird; und
wobei die Zellzusammensetzung differenzierte Zellen umfasst.

2. Organoid nach Anspruch 1, das zusätzlich dazu eine synthetische Matrix umfasst,
wobei die synthetische Matrix vorzugsweise ein Polymer oder einen Thermoplast umfasst oder aus diesem besteht,
wobei der Thermoplast vorzugsweise Polycaprolacton, Polymilchsäure, Polybutylenterephthalat, Polyethylenterephthalat, Polyethylen, Polyester, Polyvinylacetat oder Polyvinylchlorid ist; oder
das Polymer Polyvinylidenchlorid, Poly(o-carboxyphenoxy)-p-xylol) (Poly(o-CPX)), Poly(lactidanhydrid) (PLAA), n-Isopropylacrylamid, Acrylamid, Penterythritdiacrylat, Polymethylacrylat, Carboxymethylcellulose oder Poly(milch-co-glykolsäure) (PLGA) ist oder
das Polymer Polyacrylamid ist.

3. Organoid nach Anspruch 1 oder 2, wobei der eine oder die mehreren Zelltypen Folgendes umfassen:
natürliche Killer- (NK-) Zellen, wobei die NK-Zellen vorzugsweise natürliche CD56⁺-CD16⁻-Plazentazwischenkiller- (PiNK-) Zellen umfassen;
dendritische Zellen;
Thymozyten;
Thymozyten, lymphoide Zellen, Epithel-Reticulumzellen und thymische Stromazellen;
Follikelzellen, wobei das Organoid vorzugsweise Zellen umfasst, die Thyreoglobulin exprimieren;
Follikelzellen, wobei das Organoid zusätzlich dazu thyreoidale Epithelzellen und Parafollikelzellen umfasst;
Stammzellen oder Vorläuferzellen, wobei
die Stammzellen oder Vorläuferzellen vorzugsweise induzierte pluripontente Stammzellen, mesenchymale Stammzellen, vom Knochenmark stammende mesenchymale Stammzellen, an Gewebekunststoff adhärente Plazentastammzellen (PDAC®), Nabelschnurstammzellen, Fruchtwasserstammzellen, aus dem Fruchtwasser stammende adhärente Zellen (AMDACs), osteogene adhärente Plazentazellen (OPACs), adipöse Stammzellen, limbale Stammzellen, Zahnpulpastammzellen, Myoblasten, Endothelvorläuferzellen, Nervenstammzellen, exfolierte von den Zähnen stammende Stammzellen, Haarfollikelstammzellen, Hautstammzellen, parthenogenetisch abgeleitete Stammzellen, reprogrammierte Stammzellen, vom Amnion stammende adhärente Zellen oder Nebenpopulationsstammzellen sind;
hämatopoetische Stammzellen oder hämatopoetische Vorläuferzellen;
an Gewebekulturkunststoff adhärente CD34⁻-, CD10⁺-, CD105⁺- und CD200⁺-Plazentastammzellen, wobei
die Plazentastammzellen vorzugsweise zusätzlich dazu eines oder mehreres von CD45⁻, CD80⁻, CD86⁻ oder CD90⁺ sind; oder
differenzierte Zellen, wobei vorzugsweise
die differenzierten Zellen folgende umfassen: Endothelzellen, Epithelzellen, Hautzellen, endodermale Zellen, mesodermale Zellen, Fibroblasten, Osteozyten, Chondrozyten, natürliche Killerzellen, dendritische Zellen, hepatische Zellen, pankreatische Zellen oder Stromazellen;
die differenzierten Zellen folgende umfassen: Speicheldrüsenschleimhautzellen, seröse Speicheldrüsenzellen, von-Ebner-Drüsenzellen, Milchdrüsenzellen, Tränendrüsenzellen, Ceruminaldrüsenzellen, dunkle ekkrine Schweißdrüsenzellen, helle ekkrine Schweißdrüsenzellen, apokrine Schweißdrüsenzellen, Moll-Drüsenzellen, Talgdrüsenzellen, Bowman-Drüsenzellen, Brunner-Drüsenzellen, Samenbläschenzellen, Prostatadrüsenzellen, Bulbourethaldrüsenzellen, Bartholinische Drüsenzellen, Littre-Drüsenzellen, Gebärmutterendometriumzellen, isolierte Goblet-Zellen, Magenschleimhautzellen, enzymbildende Magendrüsenzellen, säurebildende Magendrüsenzellen, Pankreasazinuszellen, Paneth-Zellen, Typ-II-Pneumozyten, Clarazellen, somatotrope Zellen, lactotrope Zellen, thyreotrope Zellen, gonadotrope Zellen, corticotrope Zellen, Hypophysenzwischenzellen, magnozellulläre neurosekretorische Zellen, Darmzellen, Atemwegszellen, Schilddrüsenepithelzellen, Parafollikelzellen, Nebenschilddrüsenzellen, Nebenschilddrüsenhauptzellen, oxyphile Zellen, Nebennierenzellen, chormaffine Zellen, Leydig-Zellen, Theca-interna-Zellen, Corpusluteum-Zellen, Granulosa-lutein-Zellen, Theca-lutein-Zellen, juxtaglomeruläre Zellen, Macula-densa-Zellen, peripolare Zellen, Mesangiumzellen, fenestrierte Blutgefäß- und Lymphgefäßzellen, kontinuierliche Blutgefäß- und Lymphgefäßendothelzellen, Blutgefäß- und Lymphgefäßmilzendothelzellen, synoviale Zellen, serosale Zellen (Auskleidung von Bauch-, Pleura- und Perikardhöhle), Plattenepithelzellen, hochprismatische Zellen, dunkle Zellen, Vestibularmembranzellen (Auskleidung des endolymphatischen Ohrraums), Stria-vascularis-Basalzellen, Stria-vascularis-Marginalzellen (Auskleidung des endolymphatischen Ohrraums), Claudius-Zellen, Böttcher-Zellen, Choroid-plexus-Zellen, Pia-Arachnoidea-Plattenepithelzellen, pigmentierte ziliäre Epithelzellen, nicht pigmentierte ziliäre Epithelzellen, Hornhautepithelzellen, Peg-Zellen, nicht pigmentierte Atemwegszilienzellen, Eileiterzilienzellen, Gebärmutterendometriumzilienzelle, Rete-testis-Zilienzellen, Ductulus-efferens-Zilienzellen, ependymale Zilienzellen,
epidermale Keratinozyten, epidermale Basalzellen, Keratinozyten von Fingernägeln und Zehennägeln, Nagelbettbasalzellen, medulläre Haarschaftzellen, kortikale Haarschaftzellen, kutikulare Haarschaftzellen, kutikulare Haarwurzelschaftzellen, Haarwurzelschaftzellen der Huxley-Schicht, Haarwurzelschaftzellen der Henle-Schicht, Außenhaarwurzelschaftzellen, Haarmatrixzellen, Oberflächenepithelzellen von geschichtetem Plattenepithel, Basalzellen von Epithelien, Blasenepithelzellen,
innere Gehörganghaarzellen des Cortischen Organs, äußere Gehörganghaarzellen des Cortischen Organs, Basalzellen des Riechepithels, kälteempfindliche primäre sensorische Neuronen, wärmeempfindliche primäre sensorische Neuronen, Merkel-Zellen der Epidermis, Riechrezeptorneuronen, schmerzempfindliche primäre sensorische Neuronen, Photorezeptorstäbchenzellen, blauempfindliche Photorezeptorzäpfchenzellen, grünempfindliche Photorezeptorzäpfchenzellen, rotempfindliche Photorezeptorzäpfchenzellen, propriozeptive primäre sensorische Neuronen, berührungsempfindliche primäre sensorische Neuronen, Typ-I-Zellen der Karotisdrüse, Typ-II-Zellen der Karotisdrüse (Blut-pH-Sensor), Typ-I-Haarzelle des Vestibularapparats des Ohres (Beschleunigung und Schwerkraft), Typ-II-Haarzellen des Vestibularapparats des Ohres, Typ-I-Geschmacksknospenzellen, cholinerge Nervenzellen, adrenerge Nervenzellen, peptiderge Nervenzellen, innere Säulenzellen des Cortischen Organs, äußere Säulenzellen des Cortischen Organs, innere phalangeale Zellen des Cortischen Organs, äußere phalangeale Zellen des Cortischen Organs, Randzelle des Cortischen Organs, Hensen-Zellen des Cortischen Organs, Vestibulumstützzellen, Geschmacksknospenstützzellen, Riechepithelstützzellen, Schwann-Zellen, Satelliten-Gliazellen, enterische Gliazellen, Astrozyten, Neuronen, Oligodendrozyten, Spindelneuronen, vordere Linsenepithelzellen, kristallhältige Linsenfaserzellen,
Hepatozyten, Adipozyten, weiße Fettzellen, braune Fettzellen, Leberlipozyten, Belegzellen der Nieren-Glomeruli, Podozyten der Nieren-Glomeruli, Bürstensaumzelle der proximalen Tubuli der Nieren, Dünnes-Segment-Zellen der Henleschen Schleife, Nierenzellen der distalen Tubuli, Nierensammelrohrzellen, Typ-I-Pneumozyten, Pankreasgangzellen, ungestreifte Kanalzellen, Kanalzellen, Bürstensaumzellen des Darms, quergestreifte Kanalzellen der exokrinen Drüsen, Gallenblasenepithelzellen, unbegeißelte Zellen des Ductulus efferens, Nebenhodenhauptzellen, Nebenhodenbasalzellen, Ameloblastepithelzellen, Epithelzellen des Planum semilunatum, interdentale Epithelzelle des Cortischen Organs, Fibroblasten des weichen Bindegewebes, Fibroblasten der Hornhaut, Sehnenfibroblasten, Fibroblasten des Knochenmark-Retikulumgewebes, nicht epitheliale Fibroblasten, Perizyten, Nucleus-pulposus-Zellen, Cementoblasten/Cementozyten, Odontoblasten, Odontozyten, Chondrozyten der Hyalinknorpel, Chondrozyten der Faserknorpel, Chondrozyten der elastischen Knorpel, Osteoblasten, Osteozyten, Osteoclasten, Osteoprogenitorzellen, Hyalozyten, Sternzellen (Ohr), Lebersternzellen (Ito-Zellen), Pankreassternzellen,
rote Skelettmuskelzellen, weiße Skelettmuskelzellen, Zwischenskelettmuskelzelle, Kernsackzellen der Muskelspindel, Kernkettenzellen der Muskelspindel, Satellitenzellen, normale Herzmuskelzellen, Knotenherzmuskelzellen, Purkinje-Faser Zellen, Glattmuskelzellen, Myoepithelzellen der Iris, Myoepithelzelle der exokrinen Drüsen,
Erythrozyten, Megakaryozyten, Monozyten, Bindegewebs-Makrophagen, epidermale Langerhans-Zellen, dendritische Zellen, Mikrogliazellen, Neutrophile, Eosinophile, Basophile, Mastzellen, T-Helferzellen, Suppressor-T-Zellen, zytotoxische T-Zellen, natürliche-Killer-T-Zellen, B-Zellen, natürliche Killerzellen,
Melanozyten, pigmentierte Retinaepithelzellen,
Oogonia/Eizellen, Spermatide, Spermatozyten, Spermatogoniezellen, Spermatozoen, Ovarialfollikelzellen, Sertolizellen, Thymus-Epithelzellen und/oder interstitielle Nierenzellen.

4. Organoid nach einem der Ansprüche 1 bis 3, wobei das Organoid etwa 10¹² Zellen, etwa 10¹¹ Zellen, etwa 10¹⁰ Zellen oder etwa 10⁹ Zellen umfasst.

5. Organoid nach einem der Ansprüche 1 bis 4, wobei das Protein oder Polypeptid Folgendes ist:
(i) ein Zytokin oder Peptid, das einen aktiven Teil davon umfasst, wobei das Zytokin vorzugsweise Folgendes ist: Adrenomedullin (AM), Angiopoietin (Ang), knochenmorphogenetisches Protein (BMP), neurotropher Faktor aus dem Gehirn (BDNF), epidermaler Wachstumsfaktor (EGF), Erythropoietin (Epo), Fibroblasten-Wachstumsfaktor (FGF), von Gliazellinien abgeleiteter neurotropher Faktor (GNDF), Granulozytenkolonie-stimulierender Faktor (G- CSF), Granulozyten-Makrophagen-Kolonie-stimulierender Faktor (GM-CSF), Wachstumsdifferenzierungsfaktor (GDF-9), Hepatozytenwachstumsfaktor (HGF), von Hepatom abgeleiteter Wachstumsfaktor (HDGF), insulinähnlicher Wachstumsfaktor (IGF), migrationsstimulierender Faktor, Myostatin (GDF-8), myelomonozytischer Wachstumsfaktor (MGF), Nervenwachstumsfaktor (NGF), Plazenta-Wachstumsfaktor (PIGF), von Blutplättchen abgeleiteter Wachstumsfaktor (PDGF), Thrombopoietin (Tpo), transformierender Wachstumsfaktor α (TGF-α), TGF-β, Tumornekrosefaktor α (TNF-α), Gefäßendothel-Wachstumsfaktor (VEGF) oder ein Wnt-Protein;
(ii) ein löslicher Rezeptor für AM, Ang, BMP, BDNF, EGF, Epo, FGF, GNDF, G-CSF, GM-CSF, GDF-9, HGF, HDGF, IGF, migrationsstimulierenden Faktor, GDF-8, MGF, NGF, PIGF, PDGF, Tpo, TGF-α, TGF-β, TNF-α, VEGF oder ein Wnt-Protein;
(iii) ein Interleukin, wobei vorzugsweise
das Interleukin Folgendes ist: Interleukin-1 α (IL-1a), IL-1β, IL-1F1, IL-1F2, IL-1F3, IL-1F4, IL-1F5, IL-1F6, IL-1F7, IL-1F8, IL-1F9, IL-2, IL-3, IL- 4, IL-5, IL-6, IL-7, IL-8, IL-9, IL-10, IL-11, 35-kDa-α-Untereinheit von IL-12, 40-kDa-β-Untereinheit von IL-12, sowohl α- als auch β-Untereinheit von IL-12, IL-13, IL-14, IL-15, IL-16, IL-17A, IL-17B, IL-17C, IL-17D, IL-17E, IL-17F-Isoform 1, IL-17F-Isoform 2, IL-18, IL-19, IL-20, IL-21, IL-22, IL-23-p19-Untereinheit, IL-23-p40-Untereinheit, IL-23-p19-Untereinheit und IL-23-p40-Untereinheit zusammen, IL-24, IL-25, IL-26, IL-27B, IL-27-p28, IL-27B und IL-27-p28 zusammen, IL-28A, IL-28B, IL-29, IL-30, IL-31, IL-32, IL-33, IL-34, IL-35, IL-36α, IL-36β, IL-36γ;
(iv) ein löslicher Rezeptor für IL-1α, IL-1β, IL-1F1, IL-1F2, IL-1F3, IL-1F4, IL-1F5, IL-1F6, IL-1F7, IL-1F8, IL-1F9, IL-2, IL-3, IL-4, IL-5, IL-6, IL-7, IL-8, IL-9, IL-10, IL-11, 35-kDa-α-Untereinheit von IL-12, 40-kDa-β-Untereinheit von IL-12, IL-13, IL-14, IL-15, IL-16, IL-17A, IL-17B, IL-17C, IL-17D, IL-17E, IL-17F-Isoform 1, IL-17F-Isoform 2, IL-18, IL-19, IL-20, IL-21, IL-22, IL-23-p19-Untereinheit, IL-23-p40-Untereinheit, IL-24, IL-25, IL-26, IL-27B, IL-27-p28, IL-28A, IL-28B, IL-29, IL-30, IL-31, IL-32, IL-33, IL-34, IL-35, IL-36α, IL-36β, IL-36γ;
(v) ein Interferon (IFN), wobei vorzugsweise
das Interferon Folgendes ist: IFN-α, IFN-β, IFN-γ, IFN-λ1, IFN-λ2, IFN-λ3, IFN-K, IFN-ε, IFN-κ, IFN-τ, IFN-δ, IFN-ζ, IFN-ω oder IFN-v;
(vi) ein löslicher Rezeptor für IFN-α, IFN-β, IFN-γ, IFN-λ1, IFN-λ2, IFN-λ3, IFN-κ, IFN-ε, IFN-κ, IFN-τ, IFN-δ, IFN-ζ, IFN-ω oder IFN-v;
(vii) Insulin oder Proinsulin;
(viii) ein Insulinrezeptor;
(ix) Insulin oder ein Insulinrezeptor, wobei die Zellen zusätzlich dazu gentechnisch verändert wurden, um eines oder mehrere von Prohormonconvertase 1, Prohormonconvertase 2 oder Carboxypeptidase E zu erzeugen;
(x) Leptin (LEP);
(xi) Erythropoietin;
(xii) Thrombopoietin;
(xiii) Tyrosin-3-monooxygenase;
(xiv) Tyrosin-3-monooxygenase, wobei die Zellen außerdem gentechnisch verändert wurden, um aromatische L-Aminosäuredecarboxylase zu exprimieren;
(xv) ein Hormon oder Prohormon, wobei vorzugsweise
das Hormon Folgendes ist: Anti-Müller-Hormon (AMH), Adiponectin (Acrp30), adrenocorticotropes Hormon (ACTH), Angiotensin (AGT), Angiotensinogen (AGT), antidiuretisches Hormon (ADH), Vasopressin, atrial-natriuretisches Peptid (ANP), Calcitonin (CT), Cholecystokinin (CCK), Corticotrophin-freisetzendes Hormon (CRH), Erythropoietin (Epo), Follikelstimulationshormon (FSH), Testosteron, Östrogen, Gastrin (GRP), Ghrelin, Glucagon (GCG), Gonadotropin-freisetzendes Hormon (GnRH), Wachstumshormon (GH), Wachstumshormon-freisetzendes Hormon (GHRH), humanes Choriongonadotropin (hCG), humanes Plazenta-Laktogen (HPL), Inhibin, luteinisierendes Hormon (LH), Melanozyten-stimulierendes Hormon (MSH), Orexin, Oxytocin (OXT), Nebenschilddrüsenhormon (PTH), Prolactin (PRL), Relaxin (RLN), Sekretin (SCT), Somatostatin (SRIF), Thrombopoietin (Tpo), Schilddrüsen-stimulierendes Hormon (Tsh) und/oder Thyreotropin-freisetzendes Hormon (TRH);
(xvi) Cytochrom-P450-Seitenkettenspaltungsenzym (P450SCC) oder
(xvii) ein Protein, das bei einem Individuum mit einer genetischen Störung oder Erkrankung fehlt oder nicht funktioniert, wobei vorzugsweise die genetische Störung oder Erkrankung familiäre Hypercholesterinämie ist und das Protein ein Lipoproteinrezeptor niedriger Dichte (LDLR) ist;
die genetische Störung oder Krankheit eine polyzystische Nierenerkrankung ist und das Protein Polycystin-1 (PKD-1), PKD-2 oder PKD-3 ist; oder
die genetische Störung oder Krankheit Phenylketonurie ist und das Protein Phenylalaninhydroxylase ist.

6. Organoid nach Anspruch 5, wobei das Organoid 1 x 10⁸ Zellen umfasst und in einer In-vitro-Kultur in Wachstumsmedium innerhalb von 24 h zumindest 1,0 bis 10 µM des Proteins oder Polypeptids erzeugt.

7. Organoid nach einem der Ansprüche 1 bis 6, wobei das Organoid eine immunsuppressive Verbindung oder eine entzündungshemmende Verbindung umfasst, wobei vorzugsweise
die Verbindung ein nicht-steroidales entzündungshemmendes Arzneimittel (NSAID), Acetaminophen, Naproxen, Ibuprofen, Acetylsalicylsäure, ein Steroid, ein Anti-T-Zell-Rezeptor-Antikörper, ein Anti-IL-2-Rezeptor-Antikörper, Basiliximab, Daclizumab (ZENAPAX®), Anti-T-Zell-Rezeptor-Antikörper (z.B. Muromonab-CD3), Azathioprin, ein Corticosteroid, Cyclosporin, Tacrolimus, Mycophenolatmofetil, Sirolimus, Calcineurin-Hemmer und dergleichen ist, wobei das immunsuppressive Mittel in einer spezifischen Ausführungsform ein neutralisierender Antikörper gegen das entzündliche Makrophagenprotein- (MIP-) 1α oder MIP-1β ist.

8. Organoid nach einem der Ansprüche 1 bis 7, wobei das Organoid zumindest eine Funktion einer Leber, Niere, Bauchspeicheldrüse, Schilddrüse oder Lunge ausführt.

9. Organoid nach einem der Ansprüche 1 bis 8, wobei das Organoid Folgendes umfasst:
(i) acidophile Zellen der Hypophyse, Basophile der Hypophyse oder sowohl acidophile Zellen als auch Basophile der Hypophyse;
wobei das Organoid vorzugsweise Folgendes umfasst:
Hypophysen-Somatotrophe,
Hypophysen-Mammotrophe,
Hypophysen-Corticotrophe,
Hypophysen-Thyreotrophe,
Hypophysen-Gonadotrophe,
zwei oder mehr aus Hypophysen-Somatotrophen, Hypophysen-Mammotrophen, Hypophysen-Corticotrophen, Hypophysen-Thyreotrophen und/oder Hypophysen-Gonadotrophen oder
Zellen, die ein oder mehrere von GH, STH, PRL, ACTH, MSH, TSH, FSH, ADH und/oder LH produzieren oder gentechnisch verändert wurden, um das zu tun;
(ii) hypothalamische Neuronen, Pituizyten oder sowohl hypothalamische Neuronen als auch Pituizyten,
wobei das Organoid vorzugsweise Zellen umfasst, die eines oder beides von ADH und/oder Oxytocin erzeugen oder gentechnisch verändert wurden, um das zu tun;
(iii) Endothelgefäß-bildende Zellen,
(iv) Schilddrüsenepithelzellen, Schilddrüsenparafollikelzellen, Thyreoglobulin produzierende Zellen oder zwei oder mehr von Schilddrüsenepithelzellen, Schilddrüsenparafollikelzellen und Thyreoglobulin produzierenden Zellen,
wobei das Organoid vorzugsweise Zellen umfasst, die eines oder mehrere von T3, T4 und/oder Calcitonin erzeugen oder gentechnisch verändert wurden, um das zu tun;
(v) Nebenschilddrüsenhauptzellen, oxyphile Nebenschilddrüsenzellen oder sowohl Nebenschilddrüsenhauptzellen als auch oxyphile Nebenschilddrüsenzellen,
wobei das Organoid vorzugsweise Zellen umfasst, die PTH erzeugen oder gentechnisch verändert wurden, um das zu tun;
(vi) Zellen der Zona glomerulosa der Nebenniere, faszikulierte Nebennierenzellen, Zellen der Zona reticulata der Nebenniere, chromaffine Nebennierenzellen, wobei das Organoid vorzugsweise Zellen umfasst, die eines oder mehrere von Aldosteron, 18-Hydroxy-11-desoxycorticosteron, Cortisol, Fludrocortisone, ein Nicht-Cortison-Glucocorticoid, Epinephrin, Adrenosteron und/oder Dehydroepiandrosteron erzeugen oder gentechnisch verändert wurden, um das zu tun;
(vii) Hepatozyten,
wobei das Organoid vorzugsweise Zellen umfasst, die eines oder mehrere von Gerinnungsfaktor I (Fibrinogen), Gerinnungsfaktor II (Prothrombin); Gerinnungsfaktor V (Faktor V), Gerinnungsfaktor VII (Proconvertin); Gerinnungsfaktor IX (Christmas-Faktor), Gerinnungsfaktor X (Stuart-Prower-Faktor; Prothrombinase); Gerinnungsfaktor XI (Plasmathromboplastinvorläufer); Protein C (Autoprothrombin IIA; Blutgerinnungsfaktor XIV); Protein S, Antithrombin, IGF-1 oder Thrombopoietin erzeugen oder gentechnisch verändert wurden, um das zu tun;
(viii) Lebergefäßendothelzellen;
(xi) Pankreas-α-Zehen, Pankreas-β-Zellen, Pankreas-δ-Zellen, Pankreas-PP-Zellen, Pankreas-ε-Zellen oder zwei oder mehr von Pankreas-α-Zehen, Pankreas-β-Zellen, Pankreas-δ-Zellen, Pankreas-PP-Zellen und/oder Pankreas-ε-Zellen;
wobei das Organoid vorzugsweise Zellen umfasst, die eine detektierbare Menge von einem oder mehreren von Insulin, Glukagon, Amylin, Somatostatin, Pankreaspolypeptid und/oder Grehlin erzeugen.

10. Organoid nach Anspruch 9(i) zur Verwendung in einem Verfahren zur Behandlung eines Individuums, das folgendes benötigt:
(a) menschliches Wachstumshormon (hGH);
(b) somatotrophes Hormon (STH);
(c) Prolactin (PRL), wobei das Individuum vorzugsweise eines oder mehrere des folgenden aufweist: metabolisches Syndrom, arteriogene erektile Dysfunktion, vorzeitige Ejakulation, Oligozoospermie, Asthenospermie, Unterfunktion der Samenblasen oder Hypoandrogenismus;
(d) adrenocorticotropes Hormon (ACTH), wobei das Individuum vorzugsweise die Addison-Krankheit aufweist;
(e) Melanozyten-stimulierendes Hormon (MSH), wobei das Individuum vorzugsweise die Alzheimer-Krankheit aufweist;
(f) ein Schilddrüsen-stimulierendes Hormon (TSH), wobei das Individuum vorzugsweise Kretinismus aufweist oder manifestiert;
(g) follikelstimulierendes Hormon (FSH), wobei das Individuum vorzugsweise Unfruchtbarkeit oder Azoospermie aufweist oder manifestiert; oder
(h) luteinisierendes Hormon (LH), wobei das Individuum vorzugsweise einen niedrigen Testosteronspiegel, eine niedrige Spermienzahl oder Unfruchtbarkeit aufweist oder manifestiert.

11. Organoid nach Anspruch 9(ii) zur Verwendung in einem Verfahren zur Behandlung eines Individuums, das folgendes benötigt:
(a) antidiuretisches Hormon (ADH), wobei das Individuum vorzugsweise hypothalamischen Diabetes insipidus aufweist; oder
(b) Oxytocin.

12. Organoid nach Anspruch 9(iv) zur Verwendung in einem Verfahren zur Behandlung eines Individuums, das folgendes benötigt:
(a) Thyroxin (T4), wobei das Individuum vorzugsweise mentale Retardierung, Zwergwuchs, Schwäche, Lethargie, Kälteintoleranz oder Vollmondgesicht aufweist oder manifestiert;
(b) Triiodthyronin (T3), wobei das Individuum vorzugsweise eine Herzerkrankung aufweist, wobei das Individuum noch bevorzugter eine Serumkonzentration von T3 aufweist, die kleiner als 3,1 pmol/l ist;
(c) Calcitonin, wobei das Individuum vorzugsweise Osteoporose oder chronische Autoimmunhypothyreose aufweist.

13. Organoid nach Anspruch 9(v) zur Verwendung in einem Verfahren zur Behandlung eines Individuums, das Nebenschilddrüsenhormon (PTH) benötigt.

14. Organoid nach Anspruch 9(vi) zur Verwendung in einem Verfahren zur Behandlung eines Individuums, das folgendes benötigt:
(a) Aldosteron, wobei das Individuum idiopathischen Hypoaldosteronismus, hypereninämischen Hypoaldosteronismus oder hyporeninämischen Hypoaldosteronismus oder chronische Niereninsuffizienz aufweist;
(b) 18-Hydroxy-11-desoxycorticosteron;
(c) Fludrocortison;
(d) Cortisol, wobei das Individuum vorzugsweise akute Nebennierendefizienz, Addison-Krankheit oder Hypoglykämie aufweist;
(e) Nicht-Cortisol-Glucocorticoid;
(f) Epinephrin;
(g) Adrenosteron oder
(h) Dehydroepiandrosteron.

15. Organoid nach Anspruch 9(vii) zur Verwendung in einem Verfahren zur Behandlung eines Individuums, das einer Verbindung bedarf, wobei die Verbindung Gerinnungsfaktor I (Fibrinogen), Gerinnungsfaktor II (Prothrombin); Gerinnungsfaktor V (Faktor V), Gerinnungsfaktor VII (Proconvertin); Gerinnungsfaktor IX (Christmas-Faktor), Gerinnungsfaktor X (Stuart-Prower-Faktor; Prothrombinase); Gerinnungsfaktor XI (Plasmathromboplastinvorläufer); Protein C (Autoprothrombin IIA; Blutgerinnungsfaktor XIV); Protein S, Antithrombin, IGF-1 und/oder Thrombopoietin ist.

16. Organoid nach Anspruch 9(ix) zur Verwendung in einem Verfahren zur Behandlung eines Individuums, das Folgendes benötigt:
(a) Glucagon;
(b) Insulin, wobei das Individuum vorzugsweise Diabetes mellitus aufweist;
(c) Amylin;
(d) Ghrelin oder
(e) Pankreaspolypeptid.

## Revendications

1. Organoïde, comprenant un ou plusieurs types de cellules, et comprenant un échafaudage vasculaire placentaire décellularisé,
où ledit organoïde réalise au moins une fonction d'un organe, ou d'un tissu issu d'un organe ;
où ladite au moins une fonction d'un organe ou d'un tissu issu d'un organe est la production d'une protéine, d'un facteur de croissance, d'une cytokine, d'une interleukine, ou d'une petite molécule caractéristique d'au moins un type cellulaire dudit organe ou tissu ;
où ledit échafaudage vasculaire placentaire décellularisé comprend une matrice de système vasculaire placentaire essentiellement intacte ;
où lesdites cellules ont été génétiquement modifiées pour produire une protéine ou un polypeptide non produit(e) par la cellule à l'état naturel, ou ont été génétiquement modifiées pour produire une protéine ou un polypeptide en une quantité supérieure à celle produite par la cellule à l'état naturel ; et
où ladite composition cellulaire comprend des cellules différenciées.

2. Organoïde selon la revendication 1, comprenant en outre une matrice synthétique, de préférence dans lequel ladite matrice synthétique comprend ou consiste en un polymère ou un thermoplastique, dans lequel de préférence ledit thermoplastique est la polycaprolactone, l'acide polylactique, le polytéréphtalate de butylène, le polytéréphtalate d'éthylène, le polyéthylène, le polyester, l'acétate de polyvinyle, ou le chlorure de polyvinyle ; ou ledit polymère est le chlorure de polyvinylidine, le poly(o-carboxyphénoxy)-p-xylène) (poly(o-CPX)), un polylactide-anhydride (PLAA), le n-isopropylacrylamide, l'acrylamide, le diacrylate de pentaérythritol, le polyacrylate de méthyle, la carboxyméthylcellulose, ou l'acide poly(lactique-co-glycolique) (PLGA) ; ou
ledit polymère est le polyacrylamide.

3. Organoïde selon la revendication 1 ou 2, dans lequel lesdits un ou plusieurs types de cellules comprennent :
des cellules tueuses naturelles (NK), où de préférence
lesdites cellules NK comprennent des cellules tueuses naturelles intermédiaires placentaires (PiNK) CD56⁺ CD16⁻ ;
des cellules dendritiques ;
des thymocytes ;
des thymocytes, des cellules lymphoïdes, des cellules réticulaires épithéliales, et des cellules stromales thymiques ;
des cellules folliculaires, où de préférence ledit organoïde comprend des cellules qui expriment la thyroglobuline ;
des cellules folliculaires, où ledit organoïde comprend en outre des cellules épithéliales thyroïdiennes et des cellules parafolliculaires ;
des cellules souches ou des cellules progénitrices, où de préférence
lesdites cellules souches ou cellules progénitrices sont des cellules souches pluripotentes induites, des cellules souches mésenchymateuses, des cellules souches mésenchymateuses dérivées de moelle osseuse, des cellules stromales mésenchymateuses dérivées de moelle osseuse, des cellules souches placentaires adhérentes à un plastique de culture tissulaire (PDAC®), des cellules souches du cordon ombilical, des cellules souches du liquide amniotique, des cellules adhérentes dérivés d'amnios (AMDAC), des cellules adhérentes placentaires ostéogéniques (OPAC), des cellules souches du tissu adipeux, des cellules souches limbiques, des cellules souches de la pulpe dentaire, des myoblastes, des cellules progénitrices endothéliales, des cellules souches neuronales, des cellules souches dérivées de dents exfoliées, des cellules souches des follicules pileux, des cellules souches dermiques, des cellules souches dérivées par parthénogenèse, des cellules souches reprogrammées, des cellules adhérentes dérivées d'amnios, ou des cellules souches SP (« side population ») ;
des cellules souches hématopoïétiques ou des cellules progénitrices hématopoïétiques ;
des cellules souches placentaires CD34⁻, CD10⁺, CD105⁺ et CD200⁺ adhérentes à un plastique de culture tissulaire, où de préférence
lesdites cellules souches placentaires sont en outre une ou plusieurs de CD45⁻, CD80⁻, CD86⁻, ou CD90⁺ ; ou
des cellules différenciées, où de préférence lesdites cellules différenciées comprennent des cellules endothéliales, des cellules épithéliales, des cellules dermiques, des cellules endodermiques, des cellules mésodermiques, des fibroblastes, des ostéocytes, des chondrocytes, des cellules tueuses naturelles, des cellules dendritiques, des cellules hépatiques, des cellules pancréatiques, ou des cellules stromales ;
lesdites cellules différenciées comprennent des cellules muqueuses des glandes salivaires, des cellules séreuses des glandes salivaires, des cellules des glandes de von Ebner, des cellules des glandes mammaires, des cellules des glandes lacrymales, des cellules des glandes cérumineuses, des cellules sombres des glandes sudoripares eccrines, des cellules claires des glandes sudoripares eccrines, des cellules des glandes sudoripares apocrines, des cellules des glandes de Moll, des cellules des glandes sébacées, des cellules des glandes de Bowman, des cellules des glandes de Brunner, des cellules des vésicules séminales, des cellules de la glande prostatique, des cellules des glandes bulbo-urétrales, des cellules de la glande de Bartholin, des cellules de la glande de Littré, des cellules de l'endomètre utérin, des cellules caliciformes isolées, des cellules de la muqueuse tapissant l'estomac, des cellules zymogènes des glandes gastriques, des cellules oxyntiques des glandes gastriques, des cellules acinaires pancréatiques, des cellules de Paneth, des pneumocytes de type II, des cellules de Clara, des somatrotopes, des lactotropes, des thyrotropes, des gonadotropes, des corticotropes, des cellules de l'hypophyse intermédiaire,
des cellules neurosécrétoires magnocellulaires, des cellules intestinales, des cellules du tractus respiratoire, des cellules épithéliales de la thyroïde, des cellules parafolliculaires, des cellules des glandes parathyroïdes, des cellules principales de la parathyroïde,
des cellules oxyphiles, des cellules des glandes surrénales, des cellules chromaffines, des cellules de Leydig, des cellules de la thèque interne, des cellules du corps jaune, des cellules lutéales de la granulosa, des cellules lutéales de la thèque, des cellules juxtaglomérulaires, des cellules de la macula densa, des cellules péripolaires, des cellules mésangiales,
des cellules endothéliales fenêtrées des vaisseaux sanguins et du système vasculaire lymphatique, des cellules endothéliales continues des vaisseaux sanguins et du système vasculaire lymphatique, des cellules endothéliales spléniques des vaisseaux sanguins et du système vasculaire lymphatique, des cellules synoviales, des cellules séreuses (tapissant les cavités péritonéale, pleurale, et
péricardique), des cellules squameuses, des cellules columnaires, des cellules sombres, des cellules de la membrane vestibulaire (tapissant l'espace endolymphatique de l'oreille), des cellules basales de la strie vasculaire,
des cellules marginales de la strie vasculaire (tapissant l'espace endolymphatique de l'oreille), des cellules de Claudius, des cellules de Boettcher, des cellules des plexus choroïdes, des cellules squameuses de la pie-mère et
l'arachnoïde, des cellules de l'épithélium ciliaire pigmentaire, des cellules de l'épithélium ciliaire non pigmentaire, des cellules endothéliales cornéennes, des cellules intercalaires,
des cellules ciliées du tractus respiratoire, des cellules ciliées de l'oviducte, des cellules ciliées de l'endomètre utérin, des cellules ciliées du rete testis, des cellules ciliées du canal efférent, des cellules ciliées de l'épendyme,
des kératinocytes épidermiques, des cellules basales épidermiques, des kératinocytes des ongles des mains et des ongles des pieds, des cellules basales du lit unguéal, des cellules médullaires de la tige pilaire, des cellules corticales de la tige pilaire, des cellules cuticulaires de la tige pilaire, des cellules cuticulaires de la gaine de la racine pilaire, des cellules de la couche de Huxley de la gaine de la racine pilaire, des cellules de la couche de Henle de la gaine de la racine pilaire, des cellules externes de la gaine de la racine pilaire, des cellules de la matrice pilaire,
des cellules épithéliales de surface de l'épithélium squameux stratifié, des cellules basales de l'épithélium, des cellules de l'épithélium urinaire,
des cellules ciliées auditives internes de l'organe de Corti, des cellules ciliées auditives externes de l'organe de Corti, des cellules basales de l'épithélium olfactif, des neurones sensoriels primaires sensibles au froid, des neurones sensoriels primaires sensibles à la chaleur, des cellules de Merkel de l'épiderme, des neurones récepteurs olfactifs, des neurones sensoriels primaires sensibles à la douleur, des cellules photoréceptrices en bâtonnet, des cellules photoréceptrices des cônes sensibles au bleu, des cellules photoréceptrices des cônes sensibles au vert, des cellules photoréceptrices des cônes sensibles au rouge, des neurones sensoriels primaires proprioceptifs, des neurones sensoriels primaires du toucher, des cellules du corpuscule carotidien de type I, des cellules du corpuscule carotidien de type II (capteur du pH sanguin), des cellules ciliées de type I de l'appareil vestibulaire de l'oreille (accélération et gravité), des cellules ciliées de type II de l'appareil vestibulaire de l'oreille, des cellules des papilles gustatives de type I, des cellules neurales cholinergiques, des cellules neurales adrénergiques, des cellules neurales peptidergiques, des cellules des piliers internes de l'organe de Corti, des cellules des piliers externes de l'organe de Corti, des cellules des phalanges internes de l'organe de Corti, des cellules des phalanges externes de l'organe de Corti, des cellules de la bordure de l'organe de Corti, des cellules de Hensen de l'organe de Corti, des cellules de soutien de l'appareil vestibulaire,
des cellules de soutien des papilles gustatives, des cellules de soutien de l'épithélium olfactif, des cellules de Schwann, des cellules satellites, des cellules gliales entériques, des astrocytes, des neurones, des oligodendrocytes, des neurones en fuseau, des cellules épithéliales de la lentille antérieure, des cellules fibreuses de la lentille contenant le cristallin, des hépatocytes, des adipocytes, des adipocytes blancs, des adipocytes bruns, des lipocytes hépatiques, des cellules pariétales des glomérules rénaux, des podocytes des glomérules rénaux, des cellules de la bordure en brosse des tubules rénaux proximaux, des cellules du segment grêle de l'anse de Henlé, des cellules des tubules rénaux distaux,
des cellules des tubes collecteurs rénaux, des pneumocytes de type I, des cellules canalaires pancréatiques, des cellules des canaux non striés, des cellules canalaires,
des cellules de la bordure en brosse intestinale, des cellules des canaux striés des glandes exocrines, des cellules épithéliales de la vésicule biliaire, des cellules non ciliées des canaux efférents, des cellules principales de l'épididyme, des cellules basales de l'épididyme, des cellules épithéliales des améloblastes, des cellules épithéliales du planum semilunatum, des cellules épithéliales interdentaires de l'organe de Corti, des fibroblastes du tissu conjonctif lâche, des kératocytes cornéens, des fibroblastes du tendon, des fibroblastes du tissu réticulaire de la moelle osseuse, des fibroblastes non-épithéliaux, des péricytes, des cellules du noyau pulpeux, des cémentoblastes/cémentocytes, des odontoblastes, des odontocytes, des chondrocytes du cartilage hyalin, des chondrocytes du fibrocartilage, des chondrocytes du cartilage élastique, des ostéoblastes, des ostéocytes, des ostéoclastes, des cellules ostéoprogénitrices, des hyalocytes, des cellules stellaires (oreille), des cellules stellaires hépatiques (cellules de Ito), des cellules stellaires pancréatiques, des cellules rouges des muscles squelettiques, des cellules blanches des muscles squelettiques, des cellules intermédiaires des muscles squelettiques, des cellules à sac nucléaire du fuseau musculaire, des cellules à chaîne nucléaire du fuseau musculaire, des cellules satellites, des cellules musculaires cardiaques ordinaires, des cellules musculaires cardiaques nodales, des cellules des fibres de Purkinje,
des cellules musculaires lisses, des cellules myoépithéliales de l'iris, des cellules myoépithéliales des glandes exocrines,
des réticulocytes, des mégacaryocytes, des monocytes, des macrophages du tissu conjonctif, des cellules de Langerhans épidermiques, des cellules dendritiques, des cellules microgliales, des neutrophiles, des éosinophiles, des basophiles, des mastocytes, des cellules T auxiliaires, des cellules T suppressives, des cellules T cytotoxiques, des cellules T tueuses naturelles, des cellules B, des cellules tueuses naturelles,
des mélanocytes, des cellules de l'épithélium pigmentaire rétinien,
des ovogonies/ovocytes, des spermatides, des spermatocytes, des cellules de la spermatogonie, des spermatozoïdes, des cellules des follicules ovariens, des cellules de Sertoli, des cellules épithéliales thymiques, et/ou des cellules rénales interstitielles.

4. Organoïde selon l'une quelconque des revendications 1 à 3, où l'organoïde comprend environ 10¹² cellules, environ 10¹¹ cellules, environ 10¹⁰ cellules, ou environ 10⁹ cellules.

5. Organoïde selon l'une quelconque des revendications 1 à 4, dans lequel ladite protéine ou ledit polypeptide est
(i) une cytokine ou un peptide comprenant une partie active de celle-ci/celui-ci, où de préférence ladite cytokine est l'adrénomédulline (AM), l'angiopoïétine (Ang), une protéine morphogénétique osseuse (BMP), le facteur neurotrophique dérivé du cerveau (BDNF), le facteur de croissance épidermique (EGF), l'érythropoïétine (Epo), le facteur de croissances des fibroblastes (FGF), le facteur neurotrophique dérivé des cellules gliales (GNDF), le facteur de stimulation des colonies de granulocytes (G-CSF), le facteur de stimulation des colonies de granulocytes-macrophages (GM-CSF), le facteur de différenciation de croissance (GDF-9), le facteur de croissance des hépatocytes (HGF), le facteur de croissance dérivé de l'hépatome (HDGF), le facteur de croissance analogue à l'insuline (IGF), un facteur stimulant la migration, la myostatine (GDF-8), le facteur de croissance myélomonocytaire (MGF), le facteur de croissance des nerfs (NGF), le facteur de croissance placentaire (PIGF), le facteur de croissance dérivé des plaquettes (PDGF), la thrombopoïétine (Tpo), le facteur de croissance transformant alpha (TGF-α), le TGF-β, le facteur de nécrose tumorale alpha (TNF-α), le facteur de croissance de l'endothélium vasculaire (VEGF), ou une protéine Wnt ;
(ii) un récepteur soluble de l'AM, l'Ang, une BMP, le BDNF, l'EGF, l'Epo, le FGF, le GNDF, le G-CSF, le GM-CSF, le GDF-9, le HGF, le HDGF, l'IGF, un facteur stimulant la migration, le GDF-8, le MGF, le NGF, le PIGF, le PDGF, la Tpo, le TGF-α, le TGF-β, le TNF-α, le VEGF, ou une protéine Wnt ;
(iii) une interleukine, où de préférence
ladite interleukine est l'interleukine-1 alpha (IL-la), l'IL-1β, l'IL1F1, l'IL1F2, l'IL1F3, l'IL1F4, l'IL1F5, l'IL1F6, l'IL1F7, l'IL1F8, l'IL1F9, l'IL-2, l'IL-3, l'IL-4, l'IL-5, l'IL-6, l'IL-7, l'IL-8, l'IL-9, l'IL-10, l'IL-11, la sous-unité alpha de 35 kDa de l'IL-12, la sous-unité bêta de 40 kDa de l'IL-12, les deux sous-unités alpha et bêta de l'IL-12, l'IL-13, l'IL-14, l'IL-5, l'IL-16, l'IL-17A, l'IL-17B, l'IL-17C, l'IL-17D, l'IL-17E, l'isoforme 1 de l'IL-17F, l'isoforme 2 de l'IL-17F, l'IL-18, l'IL-19, l'IL-20, l'IL-21, l'IL-22, la sous-unité p19 de l'IL-23, la sous-unité p40 de l'IL-23, la sous-unité p19 de l'IL-23 et la sous-unité p40 de l'IL-23 ensemble, l'IL-24, l'IL-25, l'IL-26, l'IL-27B, l'IL-27-p28, l'IL-27B et l'IL-27-p28 ensemble, l'IL-28A, l'IL-28B, l'IL-29, l'IL-30, l'IL-31, l'IL-32, l'IL-33, l'IL-34, l'IL-35, l'IL-36α, l'IL-36β, l'IE-36γ ;
(iv) un récepteur soluble de l'IL-la, l'IL-1β, l'IL1F1, l'IL1F2, l'IL1F3, l'IL1F4, l'IL1F5, l'IL1F6, l'IL1F7, l'IL1F8, l'IL1F9, l'IL-2, l'IL-3, l'IL-4, l'IL-5, l'IL-6, l'IL-7, l'IL-8, l'IL-9, l'IL-10, l'IL-11, la sous-unité alpha de 35 kDa de l'IL-12, la sous-unité bêta de 40 kDa de l'IL-12, l'IL-13, l'IL-14, l'IL-5, l'IL-16, l'IL-17A, l'IL-17B, l'IL-17C, l'IL-17D, l'IL-17E, l'isoforme 1 de l'IL-17F, l'isoforme 2 de l'IL-17F, l'IL-18, l'IL-19, l'IL-20, l'IL-21, l'IL-22, la sous-unité p19 de l'IL-23, la sous-unité p40 de l'IL-23, l'IL-24, l'IL-25, l'IL-26, l'IL-27B, l'IL-27-p28, l'IL-28A, l'IL-28B, l'IL-29, l'IL-30, l'IL-31, l'IL-32, l'IL-33, l'IL-34, l'IL-35, l'IL-36α, l'IL-36β, l'IL-36γ ;
(v) un interféron (IFN), où de préférence
ledit interféron est l'IFN-α, l'IFN-β, l'IFN-γ, l'IFN-λ1, l'IFN-λ2, l'IFN-λ3, l'IFN-K, l'IFN-ε, l'IFN-κ, l'IFN-τ, l'IFN-δ, l'IFN-ζ, l'IFN-ω, ou l'IFN-v ;
(vi) un récepteur soluble de l'IFN-α, l'IFN-β, l'IFN-γ, l'IFN-λ1, l'IFN-λ2, l'IFN-λ3, l'IFN-κ, l'IFN-ε, l'IFN-κ, l'IFN-τ, l'IFN-δ, l'IFN-ζ, l'IFN-ω, ou l'IFN-v ;
(vii) l'insuline ou la proinsuline ;
(viii) un récepteur de l'insuline ;
(ix) l'insuline ou un récepteur de l'insuline, où lesdites cellules ont en outre été génétiquement modifiées pour produire une ou plusieurs parmi la prohormone convertase 1, la prohormone convertase 2, ou la carboxypeptidase E ;
(x) la leptine (LEP) ;
(xi) l'érythropoïétine ;
(xii) la thrombopoïétine ;
(xiii) la tyrosine 3-monooxygénase ;
(xiv) la tyrosine 3-monooxygénase, où lesdites cellules sont en outre modifiées pour exprimer l'acide-L aminé aromatique décarboxylase ;
(xv) une hormone ou une prohormone, où de préférence ladite hormone est l'hormone antimüllérienne (AMH), l'adiponectine (Acrp30), l'adrénocorticotrophine (ACTH), l'angiotensine (AGT), l'angiotensinogène (AGT), l'hormone antidiurétique (ADH), la vasopressine, le peptide natriurétique auriculaire (ANP), la calcitonine (CT), la cholécystokinine (CCK), l'hormone de libération de la corticotrophine (CRH), l'érythropoïétine (Epo), l'hormone folliculostimulante (FSH), la testostérone, l'œstrogène, la gastrine (GRP), la ghréline, le glucagon (GCG), l'hormone de libération des gonadotrophines (GnRH), l'hormone de croissance (GH), l'hormone de libération de l'hormone de croissance (GHRH), la gonadotrophine chorionique humaine (hCG), le lactogène placentaire humain (HPL), l'inhibine, l'hormone lutéinisante (LH), l'hormone de stimulation des mélanocytes (MSH), l'orexine, l'ocytocine (OXT), l'hormone parathyroïdienne (PTH), la prolactine (PRL), la relaxine (RLN), la sécrétine (SCT), la somatostatine (SRIF), la thrombopoïétine (Tpo), l'hormone thyréostimulante (Tsh), et/ou l'hormone thyréotrope (TRH) ;
(xvi) l'enzyme de clivage de la chaîne latérale du cytochrome P450 (P450SCC) ; ou
(xvii) une protéine absente ou montrant un dérèglement chez un individu qui souffre d'un trouble ou d'une maladie génétique, où de préférence
ledit trouble ou ladite maladie génétique est l'hypercholestérolémie familiale et ladite protéine est le récepteur des lipoprotéines de basse densité (LDLR) ;
ledit trouble ou ladite maladie génétique est la polykystose rénale, et ladite protéine est la polycystine-1 (PKD1), PKD2 ou PKD3 ; ou
ledit trouble ou ladite maladie génétique est la phénylcétonurie et ladite protéine est la phénylalanine hydroxylase.

6. Organoïde selon la revendication 5, où ledit organoïde comprend 1 x 10⁸ cellules et produit au moins 1,0 à 10 µM de ladite protéine ou dudit polypeptide dans une culture *in vitro* dans un milieu de croissance pendant 24 heures.

7. Organoïde selon l'une quelconque des revendications 1 à 6, où ledit organoïde comprend un composé immunosuppresseur ou un composé anti-inflammatoire, où de préférence
ledit composé est un anti-inflammatoire non stéroïdien (AINS), l'acétaminophène, le naproxène, l'ibuprofène, l'acide acétylsalicylique, un stéroïde, un anticorps anti-récepteur des cellules T, un anticorps anti-récepteur de l'IL-2, le basiliximab, le daclizumab (ZENAPAX®), des anticorps anti-récepteur des cellules T (par exemple, Muromonab-CD3), l'azathioprine, un corticostéroïde, la cyclosporine, le tacrolimus, le mycophénolate mofétil, le sirolimus, des inhibiteurs de la calcineurine, et analogues. Dans un mode de réalisation spécifique, l'agent immunosuppresseur est un anticorps neutralisant dirigé contre la protéine inflammatoire des macrophages (MIP)-1α ou MIP-1β.

8. Organoïde selon l'une quelconque des revendications 1 à 7, où ledit organoïde réalise au moins une fonction du foie, du rein, du pancréas, de la thyroïde ou du poumon.

9. Organoïde selon l'une quelconque des revendications 1 à 8, où ledit organoïde comprend
(i) des cellules acidophiles hypophysaires, des cellules basophiles hypophysaires, ou à la fois des cellules acidophiles et des cellules basophiles hypophysaires,
où de préférence ledit organoïde comprend
des cellules somatotropes hypophysaires,
des cellules mammotropes hypophysaires,
des cellules corticotropes hypophysaires,
des cellules thyréotropes hypophysaires,
des cellules gonadotropes hypophysaires,
deux ou plusieurs parmi des cellules somatotropes hypophysaires, des cellules mammotropes hypophysaires, des cellules corticotropes hypophysaires, des cellules thyréotropes hypophysaires et/ou des cellules gonadotropes hypophysaires, ou
des cellules qui produisent ou ont été génétiquement modifiées pour produire une ou plusieurs parmi la GH, STH, PRL, ACTH, MSH, TSH, FSH, ADH et/ou LH ;
(ii) des neurones hypothalamiques, des pituicytes, ou à la fois des neurones hypothalamiques et des pituicytes,
où de préférence ledit organoïde comprend des cellules qui produisent ou ont été génétiquement modifiées pour produire l'une de l'ADH et/ou l'ocytocine, ou les deux ;
(iii) des cellules formant des vaisseaux endothéliaux,
(iv) des cellules épithéliales thyroïdiennes, des cellules parafolliculaires thyroïdiennes, des cellules produisant la thyroglobuline, ou deux ou plusieurs parmi des cellules épithéliales thyroïdiennes, des cellules parafolliculaires thyroïdiennes, et des cellules produisant la thyroglobuline,
où de préférence ledit organoïde comprend des cellules qui produisent ou ont été génétiquement modifiées pour produire une ou plusieurs parmi la T3, la T4 et/ou la calcitonine ;
(v) des cellules principales de la parathyroïde, des cellules oxyphiles de la parathyroïde, ou à la fois des cellules principales de la parathyroïde et des cellules oxyphiles de la parathyroïde,
où de préférence ledit organoïde comprend des cellules qui produisent ou ont été génétiquement modifiées pour produire ladite PTH ;
(vi) des cellules de la zone glomérulée de la glande surrénale, des cellules de la zone fasciculée de la glande surrénale, des cellules de la zone réticulée de la glande surrénale, ou des cellules chromaffines de la glande surrénale,
où de préférence ledit organoïde comprend des cellules qui produisent ou ont été génétiquement modifiées pour produire un(e) ou plusieurs parmi l'aldostérone, la 18-hydroxy-11-désoxycorticostérone, le cortisol, des fludrocortisones, un glucocorticoïde non cortisol, l'épinéphrine, l'adrénostérone, et/ou la déhydroépiandrostérone ;
(vii) des hépatocytes,
où de préférence ledit organoïde comprend des cellules qui produisent un ou plusieurs parmi le facteur de la coagulation I (fibrinogène) ; le facteur de la coagulation II (prothrombine) ; le facteur de la coagulation V (facteur cinq) ; le facteur de la coagulation VII (proconvertine) ; le facteur de la coagulation IX (facteur Christmas) ; le facteur de la coagulation X (facteur Stuart-Prower ; prothrombinase) ; le facteur de la coagulation XI (antécédent de la thromboplastine plasmatique) ; la protéine C (autoprothrombine IIA ; facteur de la coagulation sanguine XIV), la protéine S, l'antithrombine, l'IGF-1 ou la thrombopoïétine ;
(viii) des cellules endothéliales des vaisseaux hépatiques ;
(ix) des cellules alpha pancréatiques, des cellules bêta pancréatiques, des cellules delta pancréatiques, des cellules PP pancréatiques, des cellules epsilon pancréatiques, ou deux ou plusieurs parmi des cellules alpha pancréatiques, des cellules bêta pancréatiques, des cellules delta pancréatiques, des cellules PP pancréatiques et/ou des cellules epsilon pancréatiques,
où de préférence ledit organoïde comprend des cellules qui produisent une quantité détectable d'un ou plusieurs parmi l'insuline, le glucagon, l'amyline, la somatostatine, le polypeptide pancréatique, et/ou la ghréline.

10. Organoïde tel que défini dans la revendication 9(i) destiné à être utilisé dans un procédé de traitement d'un individu ayant besoin :
(a) d'hormone de croissance humaine (hGH) ;
(b) d'hormone somatotrope (STH) ;
(c) de prolactine (PRL), où de préférence ledit individu souffre d'un ou de plusieurs parmi le syndrome métabolique, la dysfonction érectile artériogène, l'éjaculation précoce, l'oligozoospermie, l'asthénospermie, l'hypofonction des vésicules séminales, ou l'hypoandrogénisme ;
(d) d'adrénocorticotrophine (ACTH), où de préférence ledit individu souffre de la maladie d'Addison ;
(e) d'hormone de stimulation des mélanocytes (MSH), où de préférence ledit individu souffre de la maladie d'Alzheimer ;
(f) d'hormone thyréostimulante (TSH), où de préférence ledit individu souffre de ou manifeste un crétinisme ;
(g) d'hormone folliculostimulante (FSH), où de préférence ledit individu souffre de ou manifeste une infertilité ou une azoospermie ; ou
(h) d'hormone lutéinisante (LH), où de préférence ledit individu souffre de ou manifeste un faible taux de testostérone, une faible numération des spermatozoïdes ou une infertilité.

11. Organoïde tel que défini dans la revendication 9(ii) destiné à être utilisé dans un procédé de traitement d'un individu ayant besoin :
(a) d'hormone antidiurétique (ADH), où de préférence ledit individu souffre de diabète insipide hypothalamique ; ou
(b) d'ocytocine.

12. Organoïde tel que défini dans la revendication 9(iv) destiné à être utilisé dans un procédé de traitement d'un individu ayant besoin
(a) de thyroxine (T4), où de préférence ledit individu souffre de ou manifeste un retard mental, un nanisme, une faiblesse, une léthargie, une intolérance au froid, ou un visage lunaire ;
(b) de triiodothyronine (T3), où de préférence ledit individu souffre d'une maladie cardiaque, où de manière davantage préférée ledit individu présente une concentration sérique de T3 qui est inférieure à 3,1 pmol/l ; ou
(c) de calcitonine, où de préférence ledit individu souffre d'ostéoporose ou d'hypothyroïdie auto-immune chronique.

13. Organoïde tel que défini dans la revendication 9(v) destiné à être utilisé dans un procédé de traitement d'un individu ayant besoin d'hormone parathyroïdienne (PTH).

14. Organoïde tel que défini dans la revendication 9(vi) destiné à être utilisé dans un procédé de traitement d'un individu ayant besoin :
(a) d'aldostérone, où ledit individu souffre d'hypoaldostéronisme idiopathique, d'hypoaldostéronisme hyperréninémique, ou d'hypoaldostéronisme hyporéninémique, ou d'insuffisance rénale chronique ;
(b) de 18-hydroxy-11-désoxycorticostérone ;
(c) de fludrocortisone ;
(d) de cortisol, où de préférence ledit individu souffre d'insuffisance surrénalienne aiguë, de la maladie d'Addison, ou d'hypoglycémie ;
(e) d'un glucocorticoïde non cortisol ;
(f) d'épinéphrine ;
(g) d'adrénostérone ; ou
(h) de déhydroépiandrostérone.

15. Organoïde tel que défini dans la revendication 9(vii) destiné à être utilisé dans un procédé de traitement d'un individu ayant besoin d'un composé, où ledit composé est le facteur de la coagulation I (fibrinogène) ; le facteur de la coagulation II (prothrombine) ; le facteur de la coagulation V (facteur cinq) ; le facteur de la coagulation VII (proconvertine) ; le facteur de la coagulation IX (facteur Christmas) ; le facteur de la coagulation X (facteur Stuart-Prower ; prothrombinase) ; le facteur de la coagulation XI (antécédent de la thromboplastine plasmatique) ; la protéine C (autoprothrombine IIA ; facteur de la coagulation sanguine XIV) ; la protéine S ; l'antithrombine ; l'IGF-1 ; et/ou la thrombopoïétine.

16. Organoïde tel que défini dans la revendication 9(ix) destiné à être utilisé dans un procédé de traitement d'un individu ayant besoin :
(a) de glucagon ;
(b) d'insuline, où de préférence ledit individu souffre de diabète sucré ;
(c) d'amyline ;
(d) de ghréline ; ou
(e) de polypeptide pancréatique.
